# EUROPEAN PATENT APPLICATION

(11) **EP 4 331 616 A1**
(43) Date of publication of application: **06.03.2024**
(21) Application number: 22806846.6
(22) Date of filing: 12.05.2022
(51) Int. Cl.: A61K 47/68, C07D 471/04, A61P 35/00

(54) **ANTIBODY DRUG CONJUGATE, PREPARATION METHOD THEREFOR AND APPLICATION THEREOF**

(30) Priority: 13.05.2021 CN 202110525504
(71) Applicant: Tsinghua University, Haidian District Beijing 100084 (CN); Beijing Synthetic Vaccine Biosciences Co., Ltd, Beijing 100080 (CN)
(72) Inventor: LIAO, Xuebin, Beijing 100084 (CN); WANG, Zhisong, Beijing 100084 (CN); HE, Lei, Beijing 100084 (CN); ZHANG, Yan, Beijing 100084 (CN)
(74) Representative: HGF
(86) International application number: PCT/CN2022/092556
(87) International publication number: WO 2022/237884

(57) **Abstract**

Disclosed are an antibody drug conjugate, a preparation method therefor and an application thereof, which are in particular, a conjugate of an anti-PD-L1 antibody and a TLR7 and/or TLR8 agonist, a pharmaceutical composition thereof, a preparation method therefor and an application thereof. In the present invention, a modified anti-PD-L1 antibody having mutated cysteine is obtained by means of gene editing, basically retains the structure of the original antibody, and may be used for the construction of antibody drug conjugates. By means of anti-tumor experiments, it has been discovered that the obtained antibody drug conjugate has good activity, such as strong anti-tumor activity, which may significantly improve the survival rate of tumor-bearing animals, and significantly reduce toxicity. Moreover, the antibody drug conjugate is less burdensome on the bodies of test animals, which greatly reduces the minimum effective dose of small molecular drugs when used alone, expands the therapeutic window thereof, is expected to be used in the development of therapeutic drugs for various diseases (such as tumors, viral diseases such as hepatitis B, etc.), and has good application prospects and value.

## Description

### TECHNICAL FIELD

The present invention relates to the technical field of biochemical medicines, and in particular, to an antibody-drug conjugate (ADC), and especially a conjugate of an antibody and an immunomodulator for immunotherapy, such as a conjugate of an anti-PD-L1 antibody and a TLR7 and/or TLR8 agonist, and a pharmaceutical composition thereof, a preparation method therefor and use thereof.

### BACKGROUND

Toll-like receptors are a family of receptors that have been relatively conserved in evolution, including at least 13 members, 10 of which are found in humans (TLR 1-10), wherein TLR1, TLR2, TLR4, TLR5, TLR6, and TLR10 are expressed on cell surfaces, and rapidly recognize products of bacterial metabolism; TLR3, TLR7, TLR8, and TLR9 are expressed intracellularly, and are mainly used for monitoring and recognizing viral nucleic acids, wherein TLR3 recognizes double-stranded RNA, TLR7 and TLR8 recognize single-stranded RNA, and TLR9 recognizes unmethylated CG coenzyme I and modulates the response to bacterial DNA and certain viruses.

TLRs are capable of specifically recognizing pathogen-associated molecular patterns (PAMPs), play an important role in both innate immunity and adaptive immunity, and thus are bridges connecting innate immunity and adaptive immunity. Among them, TLR7 may recruit specific adapter proteins after recognizing single-stranded RNA or artificiallysynthesized small-molecule purine compounds that bind to viruses, then activates a series of signaling cascade reactions, initiates high-level systemic adaptive immune responses, kills cells infecting viruses, and thus thoroughly eliminates viruses. TLR7 agonists have been used clinically for treating chronic viral infection diseases, such as hepatitis B, hepatitis C, and the like. In addition, TLR7 agonists as adjuvants for influenza vaccines can induce more rapid and effective immune protection. In terms of anti-tumor effects, TLR7 agonists can not only directly stimulate pDCs to secrete IFN-α, but also enhance the co-stimulation and the antigen presentation capability of pDCs. The activated pDCs promote the proliferation of CD4+ T cells, further activate CD8+ T cells, and kills tumor cells. Therefore, the role of TLR7 agonists as immune adjuvants in the body's recognition and killing of tumors is gradually receiving attention.

### SUMMARY

The present inventors, based on their previous research and development achievements, further developed an antibody-drug conjugate (ADC) (particularly a conjugate of an antibody and an immunomodulator for immunotherapy, such as a conjugate of an anti-PD-L1 antibody and a TLR7 and/or TLR8 agonist), which has better therapeutic efficacy, significantly reduces toxic and side effects, and expands the therapeutic window of immunomodulators (such as a TLR7 and/or TLR8 agonists).

Specifically, the antibody-drug conjugate described above has the following structure: 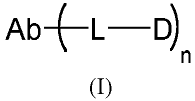 wherein,
Ab is an antibody or an antigen-binding fragment thereof;
D is a small molecule drug;
L is a linking unit between Ab and D; and
n is an integer of 1 to 100.

Specifically, the antibody described above is a monoclonal antibody.

Specifically, the form of the antibody described above may be, for example, a chimeric antibody, a humanized antibody, a fully human antibody, or the like.

Specifically, the antigen-binding fragment described above comprises a Fab fragment, an F(ab')₂ fragment, an Fd fragment, an Fv fragment, a dAb fragment, a single chain Fv (scfv), a disulfide-linked Fv (sdfv), a fragment containing a CDR, or an isolated CDR, etc.

Specifically, the antibody described above is reactive to an antigen or an epitope thereof associated with a tumor, an infectious microorganism, an autoimmune disease, or the like; specifically, the antibody described above targets antigens such as: Claudin 18.2, GPC3, HER-2/neu, carbonic anhydrase IX, B7, CCCL19, CCCL21, CSAp, BrE3, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD44, CD45, CD46, CD52, CD54, CD55, CD59, CD64, CD67, CD70, CD74, CD79a, CD80, CD83, CD95, CD126, CD133, CD138, CD147, CD154, CEACAM-5, CEACAM-6, alpha-fetoprotein (AFP), VEGF, ED-B fibronectin, EGP-1, EGP-2, EGF receptor (ErbB1), ErbB2, ErbB3, factor-H, FHL-1, Flt-3, folate receptor, Ga 733, GROB, HMGB-1, hypoxia inducible factor (HIF), HM1.24, insulin-like growth factor (ILGF), IFN-γ, IFN-α, IFN-β, IL-2R, IL-4R, IL-6R, IL-13R, IL-15R, IL-17R, IL-18R, IL-2, IL-6, IL-8, IL-12, IL-15, IL-17, IL-18, IL-25, IP-10, IGF-1R, Ia, HM1.24, ganglioside, HCG, HLA-DR, CD66a-d, MAGE, mCRP, MCP-1, MIP-1A, MIP-1B, macrophage migration inhibitory factor (MIF), MUC1, MUC2, MUC3, MUC4, MUC5, PD-1, PD-L1, placental growth factor (PIGF), PSA, PSMA, PSMA dimer, PAM4 antigen, NCA-95, NCA-90, A3, A33, Ep-CAM, KS-1, Le(y), mesothelin, S100, tenascin, TAC, Tn antigen, Thomas-Friedenreich antigen, tumor necrosis antigen, tumor angiogenesis antigen, TNF-α, TRAIL receptor (R1 and R2), VEGFR, RANTES, T101, cancer stem cell antigen, complement factors C3, C3a, C3b, C5a and C5, oncogene products, and the like.

In one embodiment of the present invention, the antibody described above is an anti-PD-L 1 antibody.

Specifically, the antibody described above comprises a reactive group (such as sulfydryl, amino, carboxyl, amido, halogen, ester group, acyl halogen, acid anhydride, epoxy group, maleimide group, aminooxy group, azido, alkynyl, 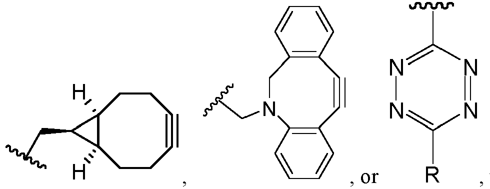 wherein R may be selected from: H, substituted or unsubstituted alkyl, substituted or unsubstituted aryl, substituted or unsubstituted aralkyl, substituted or unsubstituted cycloalkyl, substituted or unsubstituted cycloalkylalkyl, substituted or unsubstituted heterocyclyl, and substituted or unsubstituted heterocyclylalkyl), and the antibody may carry the reactive groups per se or may be obtained by mutating (such as site-directed mutagenesis) one or more amino acid residues of the known antibody into amino acids (including natural amino acids, non-natural amino acids and the like) containing the same or different required reactive groups. In one embodiment of the present invention, the antibody described above is inserted/substituted with a cysteine residue at a specific position of the amino acid sequence of the antibody by mutation (e.g., by Thiomab technology).

In one embodiment of the present invention, the antibody described above is a modified anti-PD-L1 antibody, which is obtained by mutating one or more amino acid residues of a known anti-PD-L1 antibody (e.g., atezolizumab, durvalumab, avelumab, cemiplimab, KN035, CS1001, BGB-A333, KL-A167, SHR-1316, and STI-A1014, etc.) into cysteine by genetic engineering means.

Specifically, the antibody described above comprises heavy chains and light chains;
wherein the heavy chain has an amino acid sequence set forth in SEQ ID NO: 4, or an amino acid sequence having at least 80%, 85%, 90%, 91%, 92%, 93%, 94%, 95%, 96%, 97%, 98%, or 99% identity to SEQ ID NO: 4; and/or,
the light chain has an amino acid sequence set forth in SEQ ID NO: 9, or an amino acid sequence having at least 80%, 85%, 90%, 91%, 92%, 93%, 94%, 95%, 96%, 97%, 98%, or 99% identity to SEQ ID NO: 9.

More specifically, the antibody described above has a light chain sequence set forth in SEQ ID NO: 9.

In one embodiment of the present invention, the antibody described above has a heavy chain sequence set forth in SEQ ID NO: 4 and a light chain sequence set forth in SEQ ID NO: 9.

Specifically, the n (i.e., drug/antibody ratio (DAR)) may be 1, 2, 3, 4, 5, 6, 7, 8, 9, 10, 11, 12, 13, 14, 15, 20, 25, 30, 35, 40, 45, 50, 55, 60, 65, 70, 75, 80, 85, 90, 95, or 100, e.g., 1-50, 1-40, 1-20, 1-10, 1-6, or 1-4; in some embodiments of the present invention, n = 2 or 4.

In one embodiment of the present invention, in the antibody-drug conjugate described above, a linking site between the antibody and L is a free sulfydryl group on a cysteine residue in the amino acid sequence of the antibody.

In one embodiment of the present invention, in the antibody-drug conjugate described above, the antibody has a heavy chain sequence set forth in SEQ ID NO: 4 and a light chain sequence set forth in SEQ ID NO: 9, and a linking site on the antibody for bonding with L is the free sulfydryl group on the cysteine residue at position 226 of the amino acid sequence set forth in SEQ ID NO: 9; preferably, n = 2.

In another embodiment of the present invention, in the antibody-drug conjugate described above, the antibody has a heavy chain sequence set forth in SEQ ID NO: 4 and a light chain sequence set forth in SEQ ID NO: 3, and n is 1-10, particularly 1-6.

Specifically, the small molecule drug may be an immunomodulator such as a Toll-like receptor (TLR) agonist, specifically, a TLR7 and/or TLR8 agonist, such as a pyridopyrimidine derivative and a salt thereof described in patent application publication No. WO2019/095455A1.

In one embodiment of the present invention, the moiety D in general formula I has the following structure: 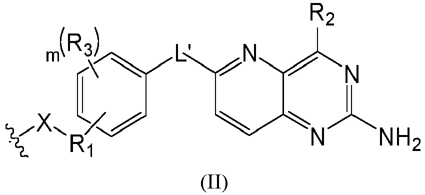 wherein,
L' is a linking group and is selected from: a single bond, C₁-C₆ alkylene, C₁-C₆ alkenylene, and C₃-C₆ cycloalkylene, wherein the groups may be optionally substituted with C₁-C₄ alkyl;
R₁ is selected from: a single bond, C₁-C₆ alkylene, C₁-C₆ alkenylene, and C₁-C₆ alkyleneoxy, wherein the groups may be optionally substituted with C₁-C₄ alkyl;
X is selected from: 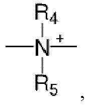 -NR₄-, -O-, -S-, a single bond, -OCO-, -COO-, -NR₄-(C₁-C₆ alkylene)-NR₅-, and heterocyclylene (particularly nitrogen-containing heterocyclylene), wherein R₄ and R₅ are independently selected from: H, C₁-C₆ alkyl, C₁-C₆ alkoxy, amino acid residue, oligopeptide residue, haloalkyl, carboxyl-substituted alkyl, ester-substituted alkyl, and 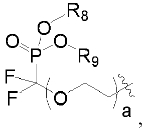 wherein a is an integer of 1 to 10 (e.g., 1, 2, 3, 4, 5, 6, 7, 8, 9, or 10), R₈ and R₉ are independently selected from: H, and C₁-C₆ alkyl, or R₈ and R₉, together with atoms to which they are attached, form 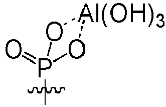 ; or, R₄ and R₅, together with the nitrogen atom to which they are attached, form heterocyclyl;
R₂ is selected from: -NR₆R₇, -OR₆, and -SR₆, wherein R₆ and R₇ are independently selected from: H, C₁-C₆ alkyl, C₁-C₆ alkoxy, and C₁-C₆ alkoxyalkyl, or, R₆ and R₇, together with the nitrogen atom to which they are attached, form heterocyclyl, or, R₆, together with the oxygen atom to which it is attached, forms heterocyclyl, or, R₆, together with the sulfur atom to which it is attached, forms heterocyclyl;
R₃ is one or more independent substituents on the phenyl ring, and is selected from: H, C₁-C₆ alkyl, C₁-C₆ alkoxy, and C₁-C₆ alkoxyalkyl; and
m is an integer of 0 to 4 (e.g., 0, 1, 2, 3, or 4).

Specifically, formula II has the following structure:

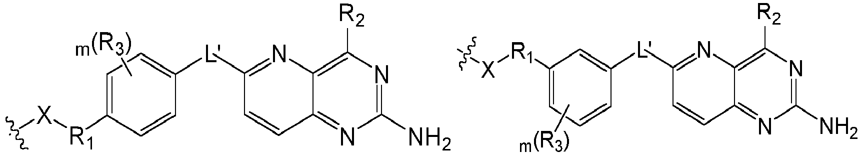

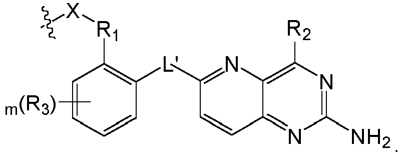

In one embodiment of the present invention, L' is C₁-C₆ alkylene, such as C₁-C₃ alkylene.

In one embodiment of the present invention, L' has the following structure: 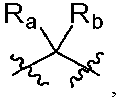 wherein Rₐ and R_{b} are independently selected from: H, and C₁-C₃ alkyl (e.g., methyl, ethyl, *n*-propyl, isopropyl), or, Rₐ and R_{b}, together with the carbon atom to which they are attached, form C₃-C₆ cycloalkylene (e.g., cyclopropylene, cyclobutylene, cyclopentylene, or cyclohexylene).

Specifically, L' may be selected from: -CH₂-,

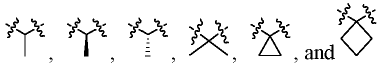

In one embodiment of the present invention, R₁ is C₁-C₆ alkylene, e.g., C₁-C₃ alkylene, such as -CH₂-.

In another embodiment of the present invention, R₁ is a single bond.

In one embodiment of the present invention, R₁ is C₁-C₆ alkyleneoxy, e.g., C₁-C₃ alkyleneoxy, such as -CH₂O-, - CH₂CH₂O-, or -CH₂CH₂CH₂O-.

Specifically, R₃ is one or more independent substituents on the phenyl ring and is selected from: H, methyl, and methoxy. In embodiments of the present invention, m is 0 or 1.

Specifically, a is an integer of 1 to 5, e.g., 1, 2, 3, 4, or 5.

Specifically, R₈ and R₉ are independently selected from: H, methyl, ethyl, *n*-propyl, and isopropyl.

Specifically, R₄ and R₅ are independently selected from: H, methyl, ethyl, *n*-propyl, isopropyl, *n*-butyl, *tert*-butyl, amino acid residue, oligopeptide residue, -CF₃, -CH₂CF₃, 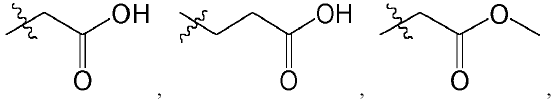 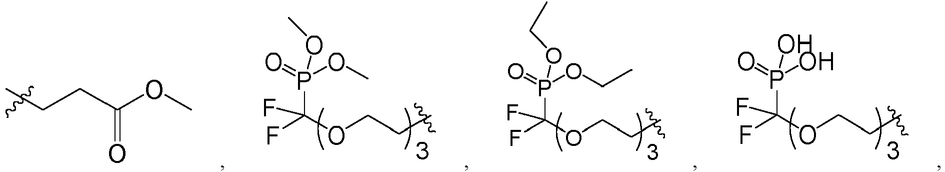 and 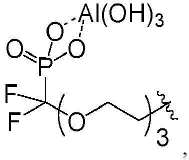 or, R₄ and R₅, together with the nitrogen atom to which they are attached, form substituted or unsubstituted heterocyclyl.

Specifically, the substituted or unsubstituted heterocyclyl described above may be selected from: 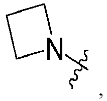 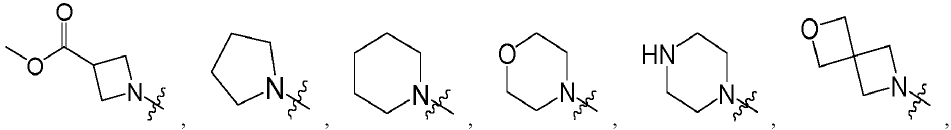 and

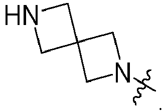

Specifically, the amino acid residue described above and the amino acid residues in the oligopeptide are independently selected from one or more of: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine residues. In one embodiment of the present invention, the amino acid residue described above is leucine residue. In one embodiment of the present invention, the oligopeptide residue is -asparagine-alanine-alanine-leucine-.

In some embodiments of the present invention, X is 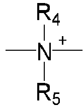 , and R₄ and R₅ are independently selected from Ci-Ce alkyl; for example, R₄ and R₅ are both methyl.

In some embodiments of the present invention, X is -NR₄-, and R₄ is selected from: H, and C₁-C₆ alkyl; for example, R₄ is H or methyl.

In some embodiments of the present invention, X is -NR₄-(C₁-C₆ alkylene)-NR₅-, and R₄ and R₅ are independently selected from C₁-C₆ alkyl; for example, R₄ and R₅ are both methyl.

In some embodiments of the present invention, X is heterocyclylene, in particular nitrogen-containing heterocyclylene, e.g., 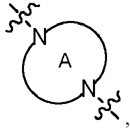 ring A is a 4- to 10-membered nitrogen-containing heterocyclic ring, and ring A may be monocyclic, bicyclic, or tricyclic (including fused, spiro, bridged); specifically, X may be 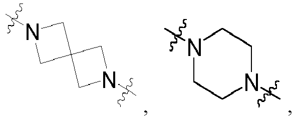 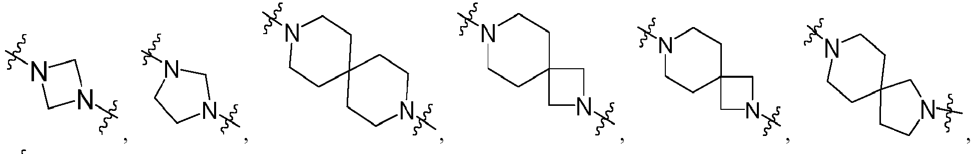 or

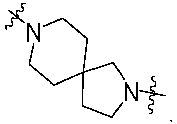

In one embodiment of the present invention, R₂ is selected from: -NHR₆, -OR₆, and -SR₆, wherein R₆ is C₁-C₆ alkyl or C₁-C₆ alkoxyalkyl, such as C₁-C₄ alkyl, e.g., butyl, in particular *n*-butyl, such as C₁-C₄ alkoxyalkyl, e.g., methoxyethyl.

In some embodiments of the present invention, R₂ is selected from:

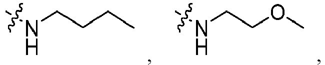

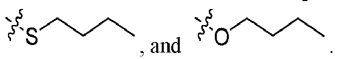

In another embodiment of the present invention, R₂ is selected from: -NR₆R₇, wherein R₆ and R₇, together with the nitrogen atom to which they are attached, form heterocyclyl, such as

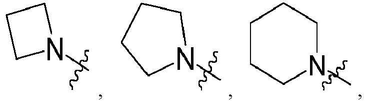

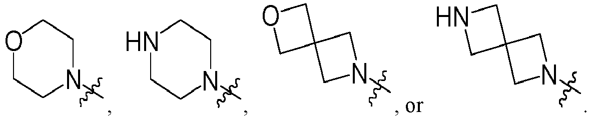

Specifically, the moiety D in general formula I may be selected from the following structures:

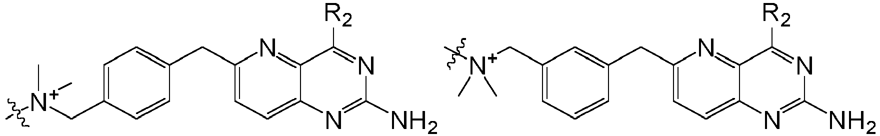

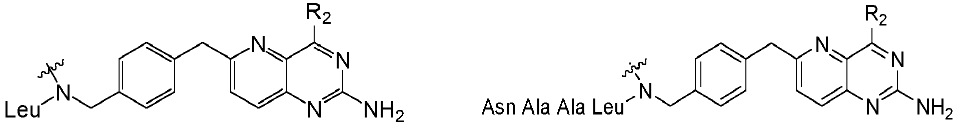

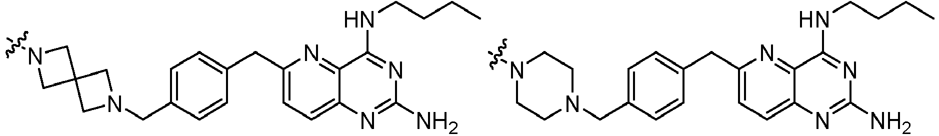

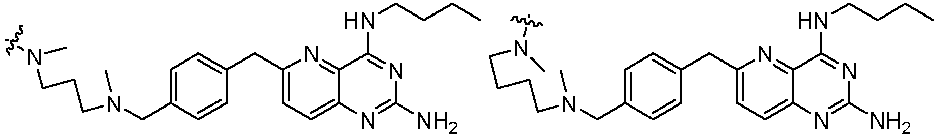

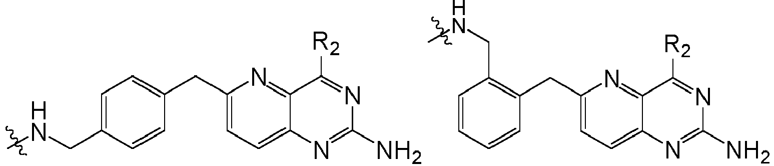

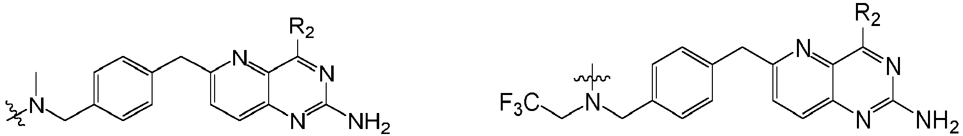

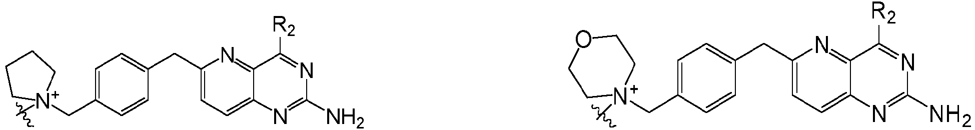

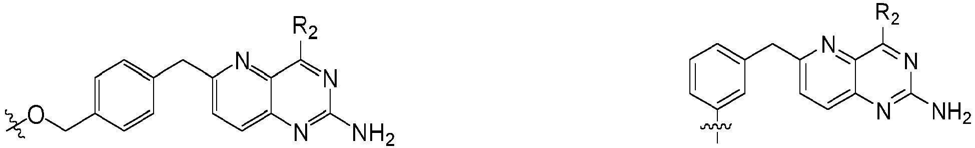

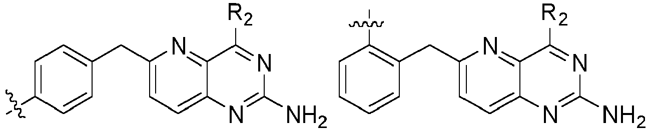

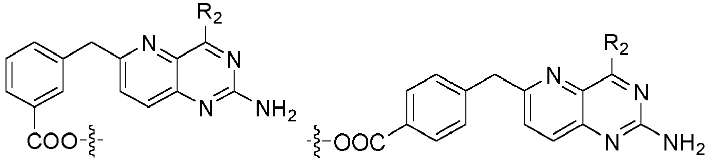

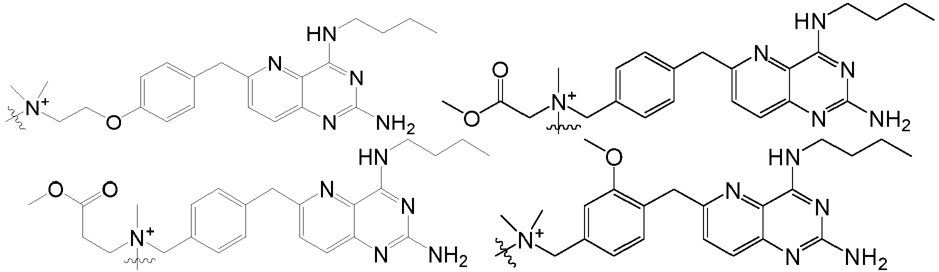

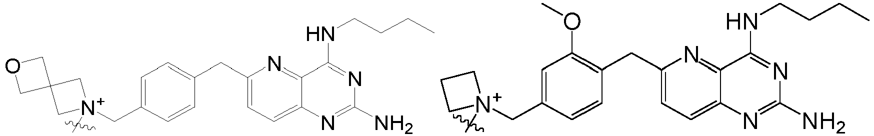

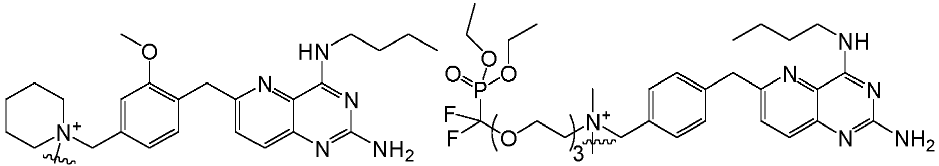

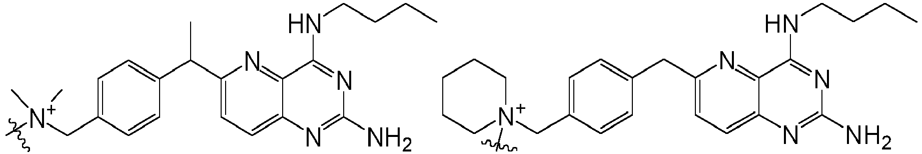

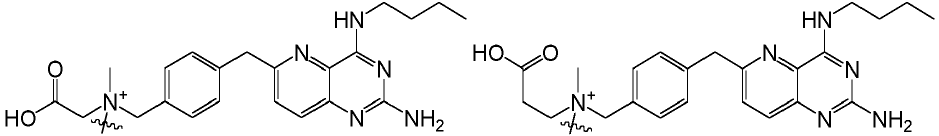

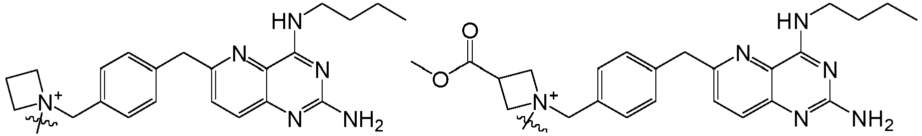

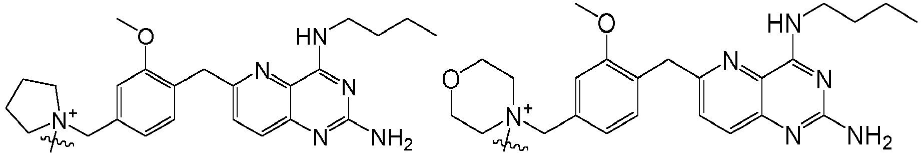

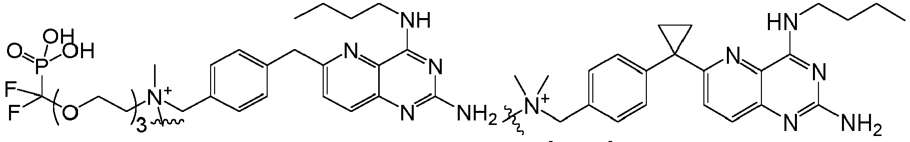

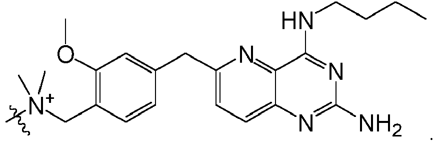

In one embodiment of the present invention, the moiety D in general formula I has the following structure:

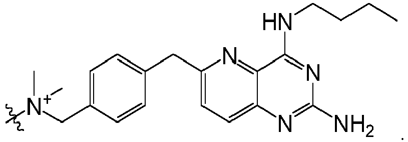

In one embodiment of the present invention, the conjugate described above has the following structure: 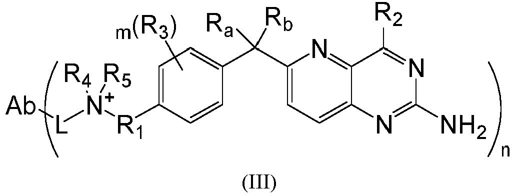 wherein Ab, n, m, R₁, R₂, R₃, R₄, R₅, Rₐ and R_{b} have the definitions described above in the present invention.

Specifically, the linking unit in the conjugate described above may be in the form of a chemically unstable linking unit (such as those containing hydrazone, or disulfide groups), a linking unit for enzymatic catalysis (such as those containing a peptide residue, an esterase-labile carbonate residue), or the like.

Specifically, the linking unit L, linking Ab with D, comprises a functional group Y, such as a single bond, 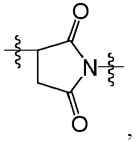 -S-, -CO-, -NH-, -CONH-, or 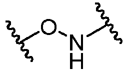 (aminooxy) for bonding with an active group of Ab (such as sulfydryl, amino, carboxyl, etc., particularly sulfydryl); in some embodiments of the present invention, Y is -S- or 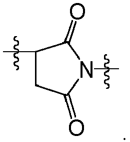 Furthermore, L may further comprises a group Q, Q being a divalent saturated or unsaturated, linear or branched C1-50 hydrocarbon chain, with 0-6 methylene units independently substituted with: -Cy-, -O-, -NR₁₀-, -S-, -OC(O)-, -C(O)O-, -C(O)-, -S(O)-, -S(O)₂-, -NR₁₀S(O)₂-, -S(O)₂-NR₁₀-, -NR₁₀-C(O)-, -C(O)NR₁₀-, -OC(O)NR₁₀-, -NR₁₀-C(O)O-, 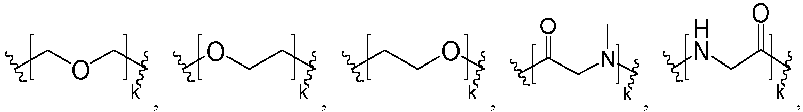 amino acid residue, or oligopeptide residue, wherein k is selected from integers of 1 to 10 (such as 1, 2, 3, 4, 5, 6, 7, 8, 9, and 10), each -Cy- is independently an optionally-substituted divalent ring selected from: arylene, cycloalkylene, and heterocyclylene; and R₁₀ is selected from: H, -OH, Ci-Ce alkyl, C₃-C₆ cycloalkyl, and heterocycloalkyl.

Specifically, each -Cy- is independently an optionally substituted divalent ring selected from: phenylene, bicyclic arylene, monocyclic cycloalkylene, bicyclic cycloalkylene, monocyclic heteroarylene, bicyclic heteroarylene, monocyclic heterocycloalkylene, and bicyclic heterocycloalkylene; in particular, phenylene, monocyclic cycloalkylene, monocyclic heteroarylene, and monocyclic heterocycloalkylene.

More specifically, each -Cy- may be independently selected from: 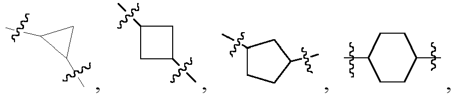 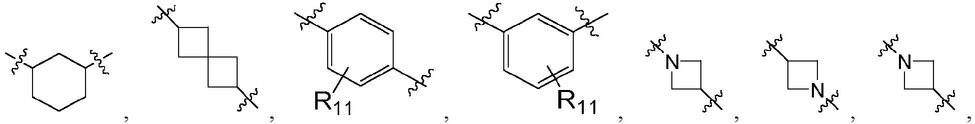 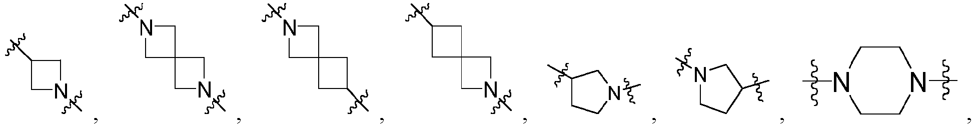 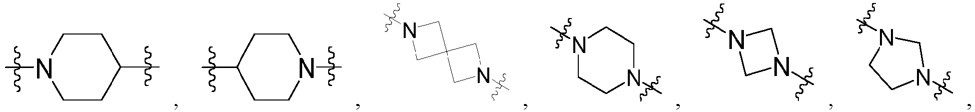 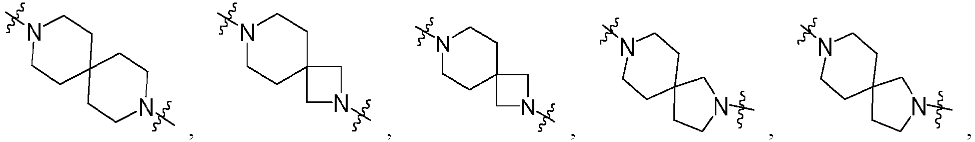 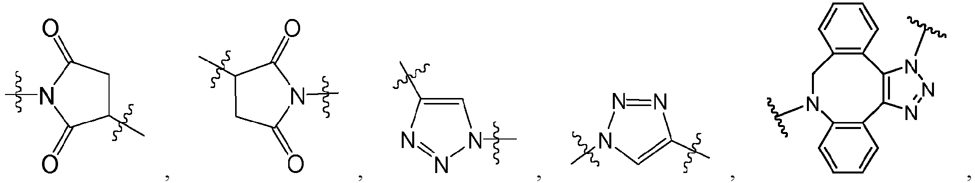 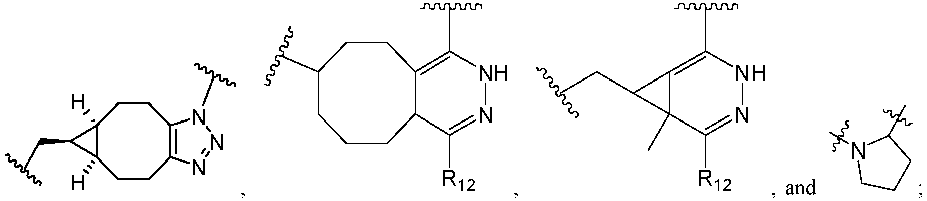 wherein R₁₁ is one or more independent substituents on the ring and is selected from: H, halogen, -CN, -NO₂, -CF₃, -OCF₃, -NH₂, -OH, C₁-C₆ alkyl, -O(C₁-C₆ alkyl), -NH(C₁-C₆ alkyl), -N(C₁-C₆ alkyl)(C₁-C₆ alkyl), and a residue of a monosaccharide or a derivative thereof, and R₁₂ is selected from: H, substituted or unsubstituted alkyl, substituted or unsubstituted aryl, substituted or unsubstituted aralkyl, substituted or unsubstituted cycloalkyl, substituted or unsubstituted cycloalkylalkyl, substituted or unsubstituted heterocyclyl, and substituted or unsubstituted heterocyclylalkyl.

Specifically, the monosaccharide or the derivative thereof may be selected from: arabinose, xylose, ribose, glucose, mannose, galactose, fructose, glucuronic acid, and galacturonic acid. In some embodiments of the present invention, the residue of the monosaccharide or the derivative thereof is a galacturonic acid residue, such as

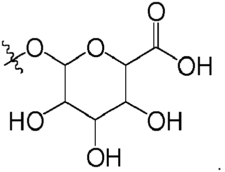

In some embodiments of the present invention, each -Cy- is independently selected from:

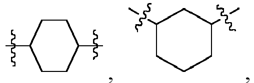

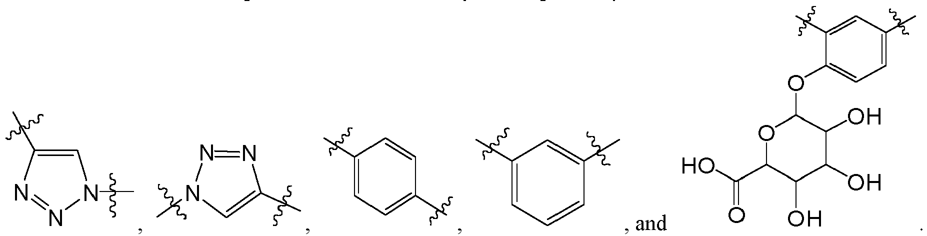

Specifically, R₁₀ may be selected from: H, and C₁-C₆ alkyl; in some embodiments of the present invention, R₁₀ is H or methyl.

Specifically, in the definition of Q described above, the amino acid residue and the amino acid residues in the oligopeptide residues are independently selected from one or more of: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, citrulline, and ornithine residues, in particular selected from: one or more of valine, citrulline, alanine, asparagine, aspartic acid, glutamic acid, proline, and glycine residues; in particular, the amino acid residue and the amino acid residues in the oligopeptide residue are residues of L-type amino acids.

Specifically, in the definition of Q described above, the oligopeptide residue consists of 2-10 amino acid residues, in particular 2-6 amino acid residues, such as 2, 3, 4 or 5 amino acid residues.

Specifically, in the definition of Q described above, the oligopeptide residue is selected from: a valine-citrulline residue, an alanine-alanine-asparagine residue, an aspartic acid-valine residue, a glutamic acid-valine residue, a glycine-proline residue, and the like.

In some embodiments of the present invention, the oligopeptide residue is

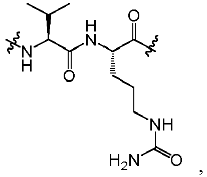

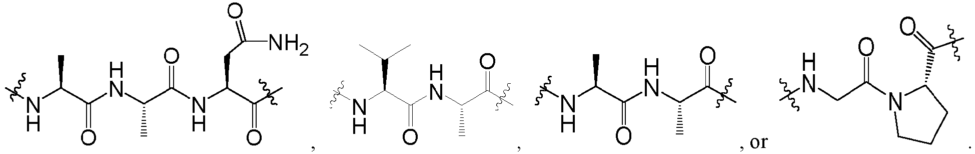

In one embodiment of the present invention, the moiety L in general formula I has the following structure: 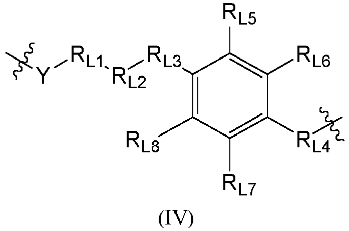 wherein,
Y is a functional group for bonding with an active group of Ab (e.g., sulfydryl, amino, carboxyl, etc., in particular sulfydryl);
R_{L1}, R_{L3}, and R_{L4} are independently selected from one or a combination of two or more of: 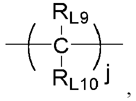 -(CH₂)ⱼO-, - (CH₂)ⱼN(R_{L9})-, -(CH₂)ⱼCO-, -(CH₂)ⱼOCO-, -(CH₂)ⱼOCON(R_{L9})-, -(CH₂)ⱼN(R_{L9})CON(R_{L9})-, -(CH₂)ⱼN(R_{L9})CO-, - O(CH₂)ⱼCOO-, -(CH₂)ⱼCOO-, -(CH₂)ⱼCON(R_{L9})-, -(CH₂)ⱼS-S-, -(CH₂)ⱼN(R_{L9})-NH=, 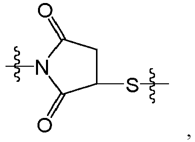 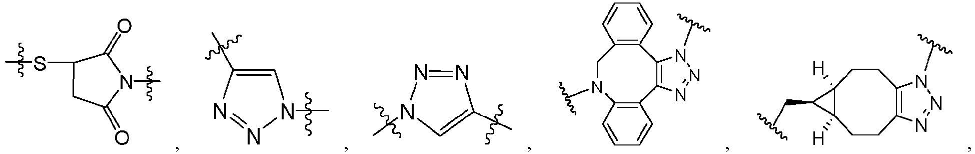 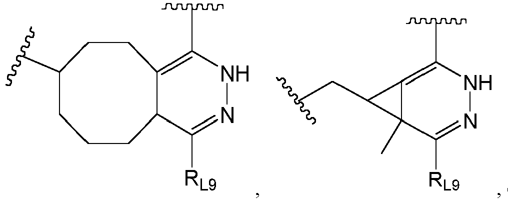 cycloalkylene, and arylene, and j is an integer of 0 to 10;
R_{L2} is selected from: a single bond, -(CH₂)ᵢOCO-, -(CH₂)ᵢOCOO-, (CH₂)ᵢNH-COO-, amino acid residues, and oligopeptide residues, wherein i is an integer of 0 to 10;
R_{L5}, R_{L6}, R_{L7}, and R_{L8} are independently selected from: H, substituted or unsubstituted alkyl, halogen, nitro, cyano, - OR_{L9}, -NR_{L9}R_{L10}, -S(O)ₜR_{L9}, -C(O)OR_{L9}, -C(O)R_{L9}, and -C(O)NR_{L9}R_{L10}, wherein t is 0, 1, or 2; and
each of R_{L9} and R_{L10} is independently selected from: H, substituted or unsubstituted alkyl, substituted or unsubstituted aryl, substituted or unsubstituted aralkyl, substituted or unsubstituted cycloalkyl, substituted or unsubstituted cycloalkylalkyl, substituted or unsubstituted heterocyclyl, and substituted or unsubstituted heterocyclylalkyl.

Specifically, Y is selected from: a single bond, 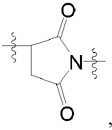 -S-, -CO-, -NH-, -CONH-, and 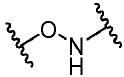 (aminooxy).

In some embodiments of the present invention, Y is -S- or

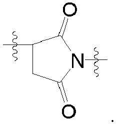

Specifically, in the definition of R_{L2} described above, the amino acid residue and the amino acid residues in the oligopeptide residue are independently selected from one or more of: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, citrulline, and ornithine residues. In particular, the amino acid residue and the amino acid residues in the oligopeptide residue are residues of L-type amino acids.

Specifically, in the definition of R_{L2} described above, the oligopeptide residue consists of 2-10 amino acid residues, in particular 2-6 amino acid residues, such as 2, 3, 4 or 5 amino acid residues.

Specifically, in the definition of R_{L2} described above, the oligopeptide residue may be selected from: a valine-citrulline residue, an alanine-alanine-asparagine residue, an aspartic acid-valine residue, a glutamic acid-valine residue, a glycine-proline residue, and the like.

In one embodiment of the present invention, R_{L2} is -(CH₂)ᵢOCO-, wherein i is an integer of 0 to 6, and in particular, i is 1.

In one embodiment of the present invention, R_{L2} is -(CH₂)ᵢ, wherein i is an integer of 1 to 10, and in particular, i is 2.

In another embodiment of the present invention, R_{L2} is an oligopeptide residue and is selected from: a valine-citrulline residue, an alanine-alanine-asparagine residue, an aspartic acid-valine residue, a glutamic acid-valine residue, and the like, in particular,

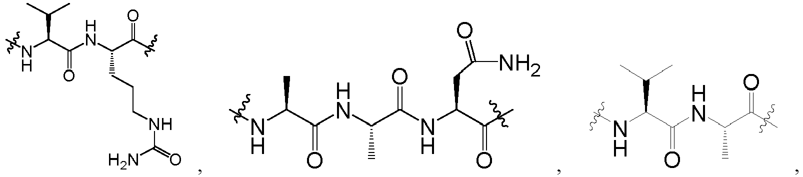

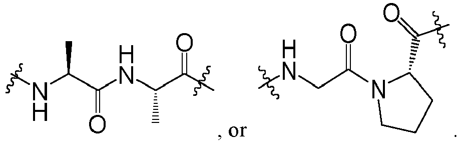

Specifically, R_{L1}, R_{L3}, and R_{L4} are independently selected from one or a combination of two or more of: -(CH₂)ⱼ-, - (CH₂)ⱼO-, -(CH₂)ⱼNH-, -(CH₂)ⱼCO-, -(CH₂)ⱼOCOO-, -(CH₂)ⱼOCONH-, -(CH₂)ⱼNHCONH-, -(CH₂)ⱼNHCO-, - O(CH₂)ⱼCOO-, -(CH₂)ⱼCOO-, and -(CH₂)ⱼCONH-, wherein j is an integer of 0 to 10.

Specifically, each j may be independently 0, 1, 2, 3, 4, 5, 6, 7, 8, 9, or 10.

In one embodiment of the present invention, R_{L1} is

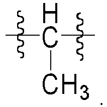

In another embodiment of the present invention, R_{L1} is -(CH₂)ⱼCO-, wherein j is an integer of 0 to 6, and in particular, j is 5.

In one embodiment of the present invention, R_{L3} is -(CH₂)ⱼNH-, wherein j is an integer of 0 to 6, and in particular, j is 0. In one embodiment of the present invention, R_{L4} is -(CH₂)ⱼ-, wherein j is an integer of 0 to 5, and in particular, j is 1. Specifically, R_{L5}, R_{L6}, R_{L7}, and R_{L8} are independently selected from: H, C1-6 alkyl, C1-6 haloalkyl, halogen, nitro, cyano, and -OR_{L9}; R_{L8} is selected from: H, and C1-6 alkyl; more specifically, R_{L5}, R_{L6}, R_{L7}, and R_{L8} are independently selected from: H, and halogen.

In one embodiment of the present invention, R_{L5} is H.

In one embodiment of the present invention, R_{L6} is H.

In one embodiment of the present invention, R_{L7} is H.

In one embodiment of the present invention, R_{L8} is H.

In one embodiment of the present invention, the moiety L has the following structure: 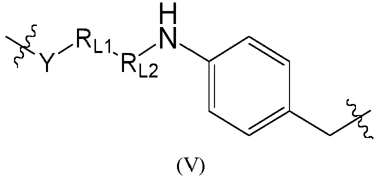 wherein Y, R_{L1}, and R_{L2} have the corresponding definitions described above in the present invention. In one embodiment of the present invention, the moiety L may have the following structure:

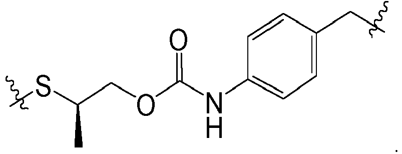

In another embodiment of the present invention, the moiety L may further have the following structure: 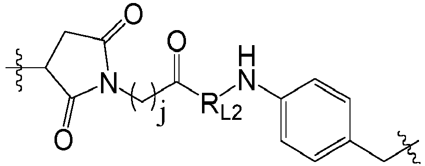 wherein R_{L2} is an oligopeptide residue and has the definition described above in the present invention.

Specifically, the moiety L described above has the following structure:

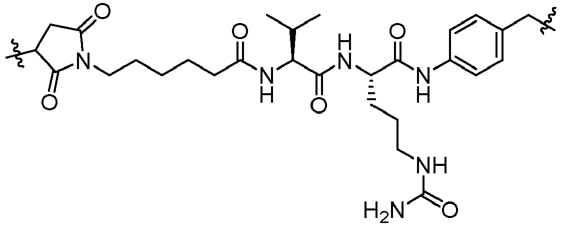

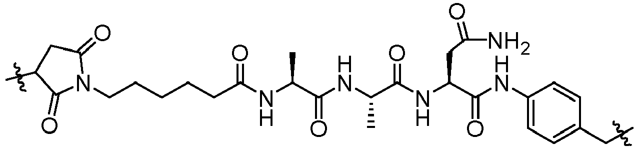

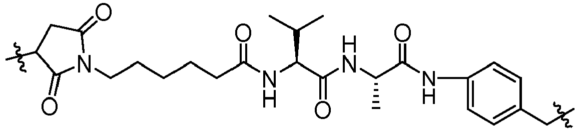

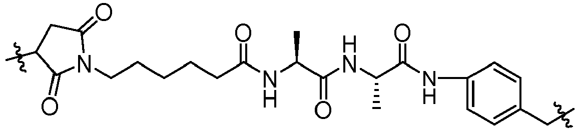

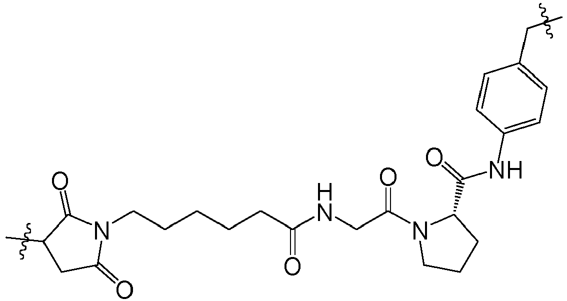

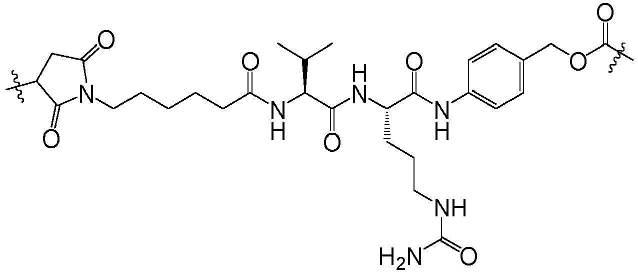

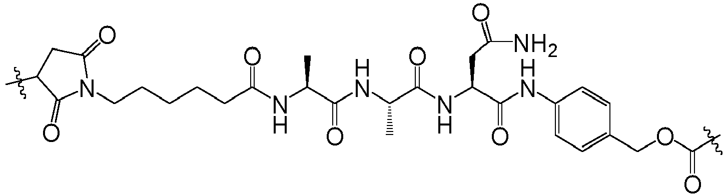

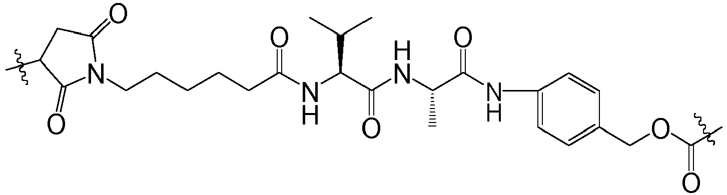

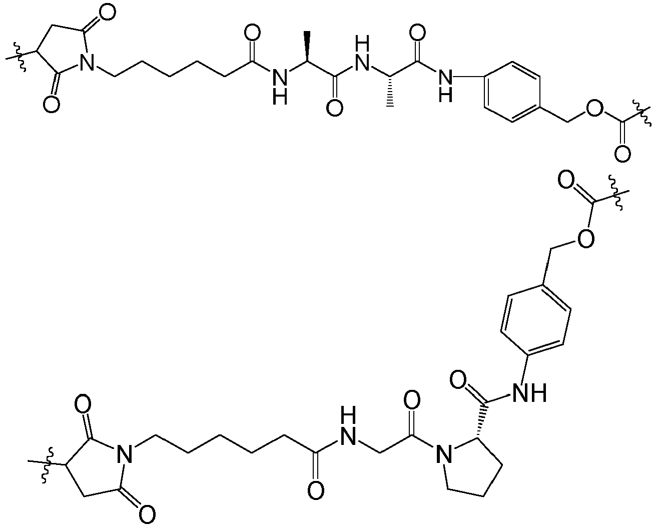

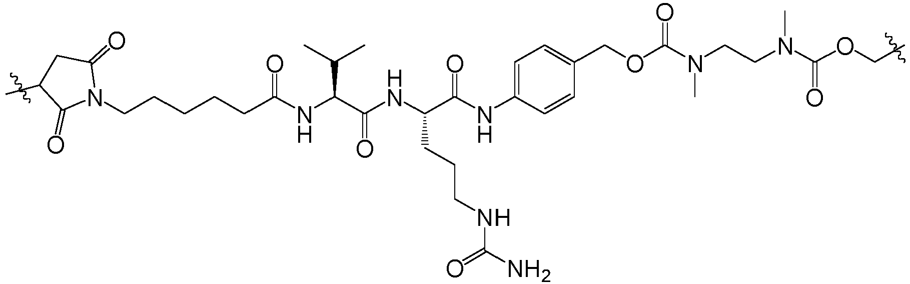

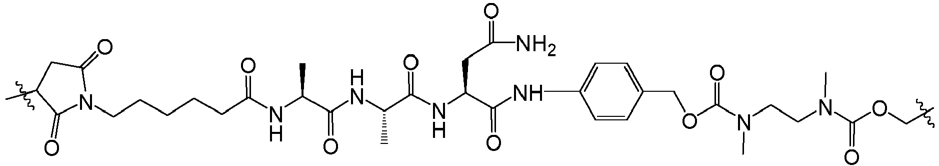

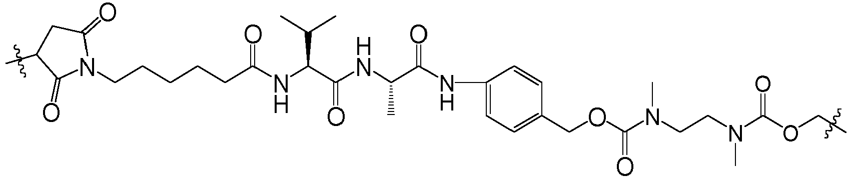

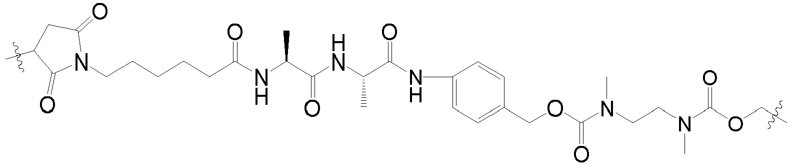

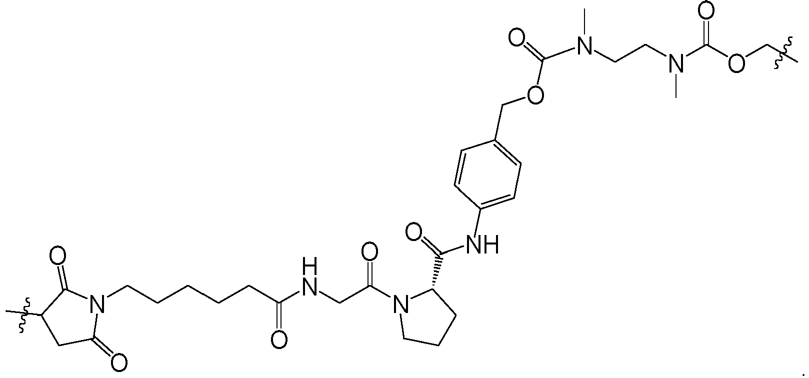

In some embodiments of the present invention, the moiety L has the following structure: 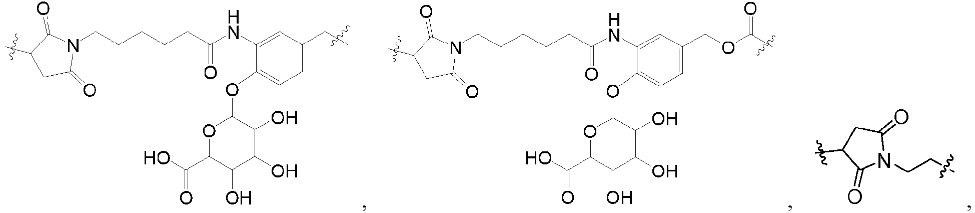 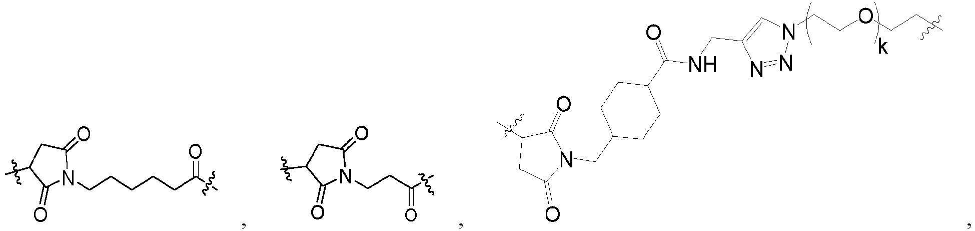 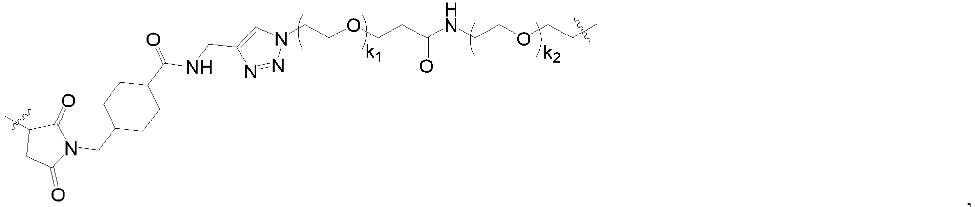 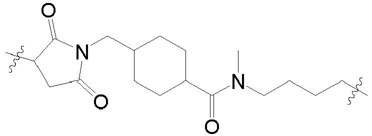 and 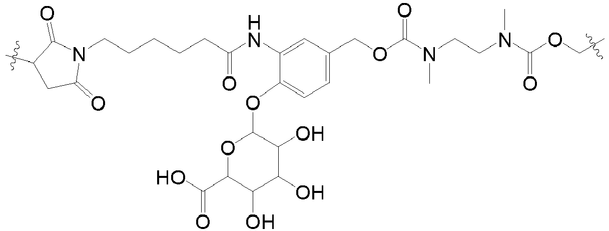 wherein k₁ and k₂ are independently selected from integers of 1 to 10.

In one embodiment of the present invention, the conjugate described above has the following structure: 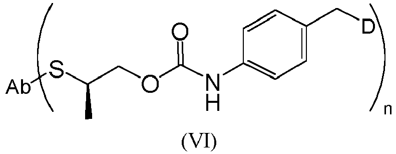 wherein Ab, n, and D have the corresponding definitions described above in the present invention.

In some embodiments of the present invention, the conjugate described above may have the following structure: 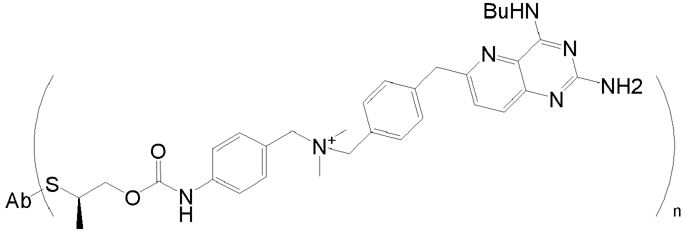 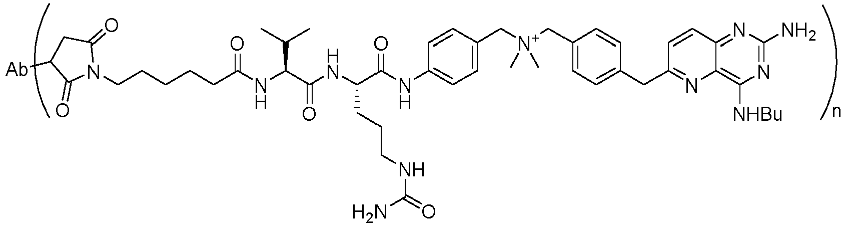 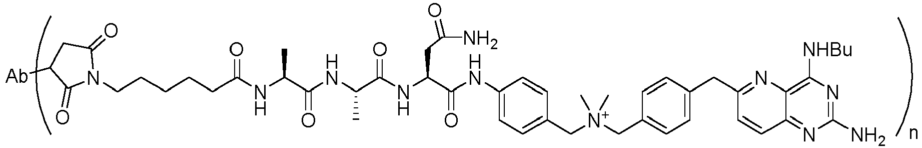 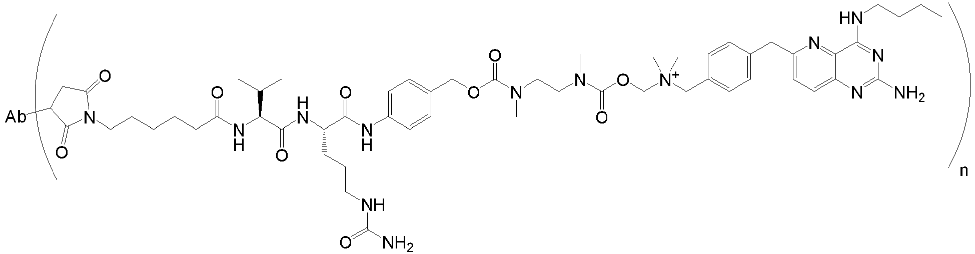 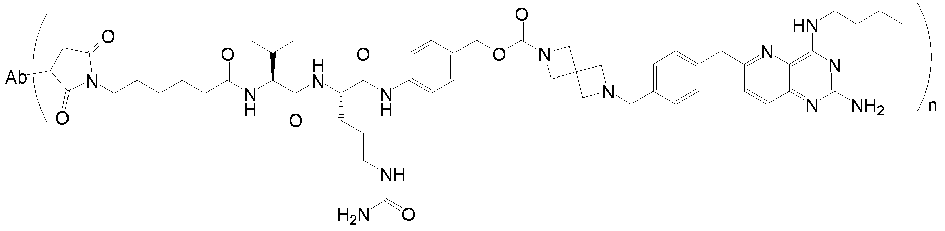 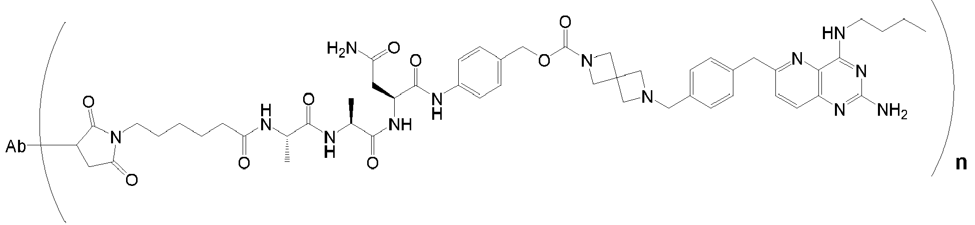 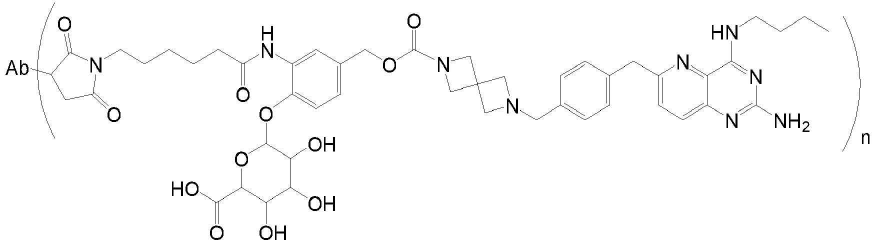 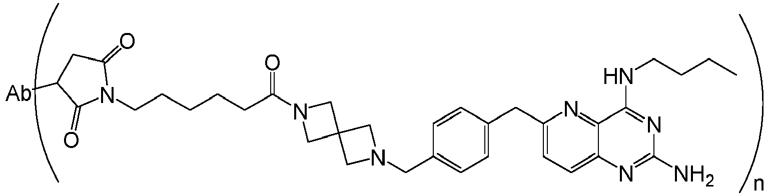 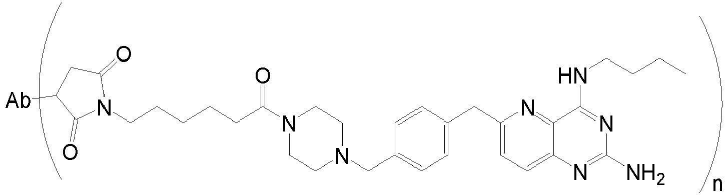 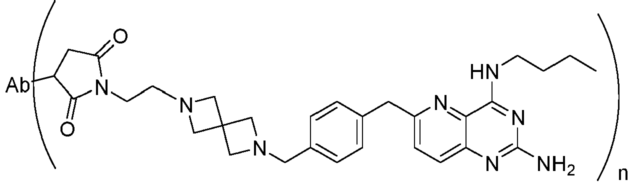 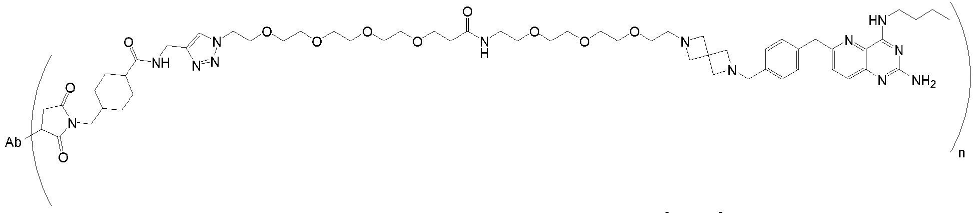 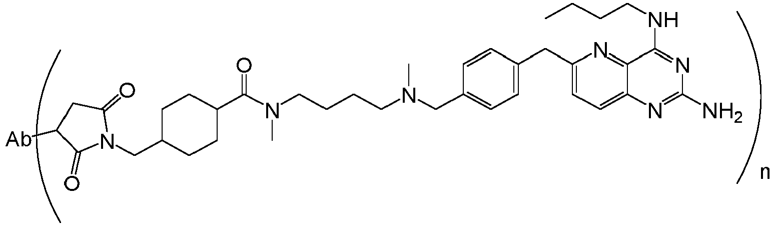 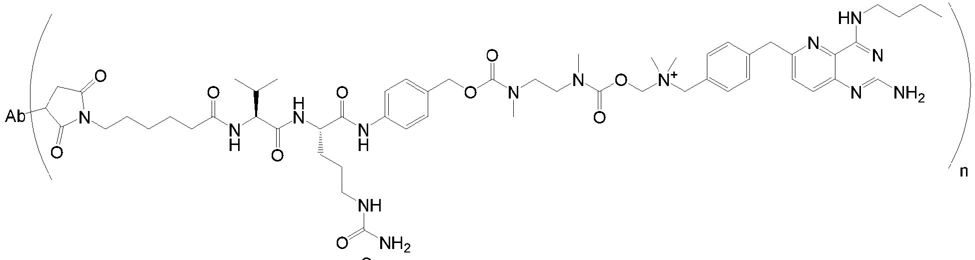 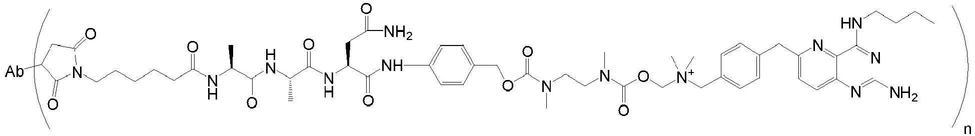 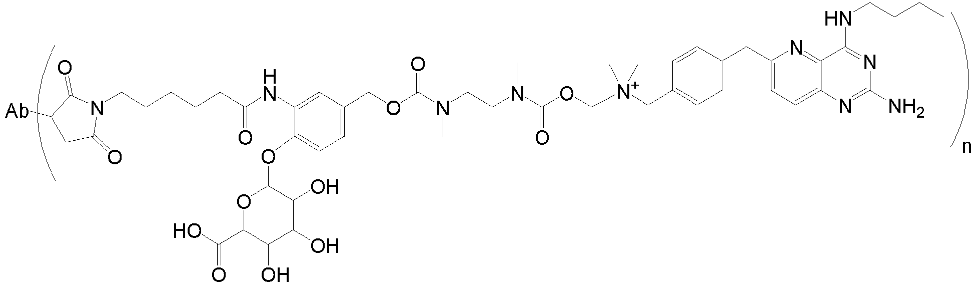 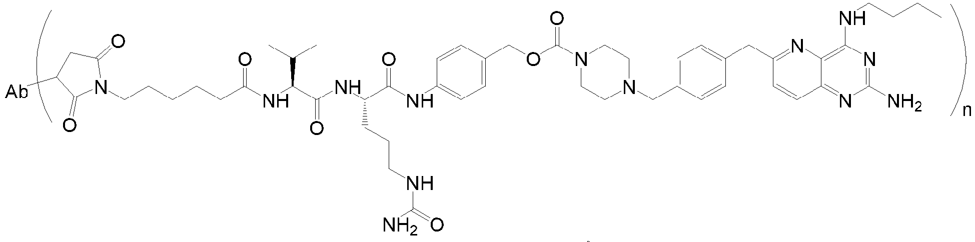 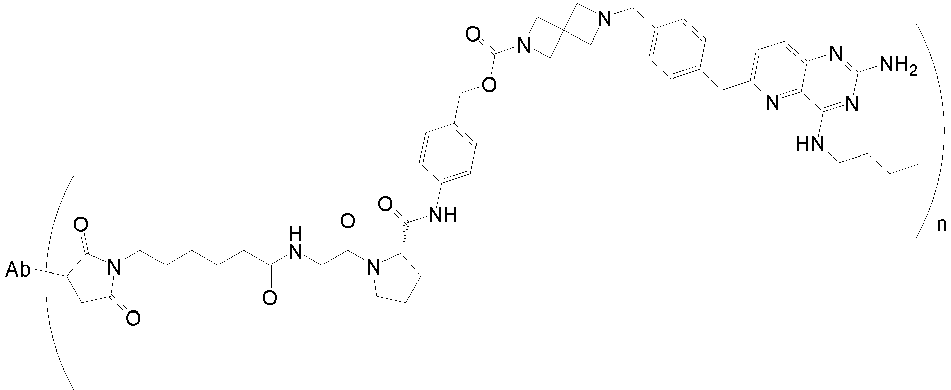 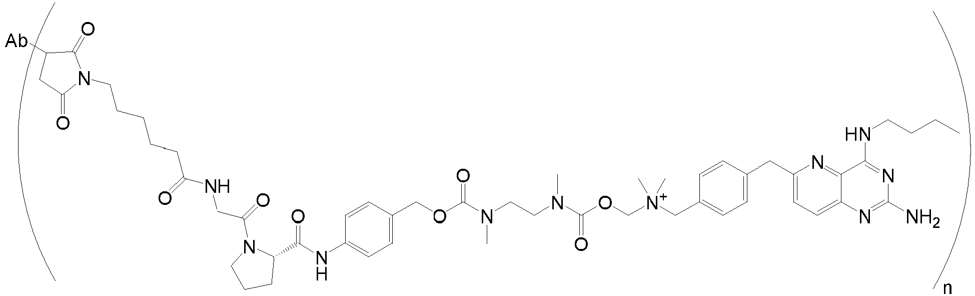 wherein Ab and n have the corresponding definitions described above in the present invention.

The present invention also provides a stereoisomer (shown as above) of the conjugate described above or a mixture thereof.

The present invention also provides a pharmaceutically acceptable salt, a solvate and a prodrug of the conjugate described above.

Specifically, the pharmaceutically acceptable salt may include organic salts or inorganic salts, e.g., one or more of hydrochloride, hydrobromide, sulfate, nitrate, phosphate, formate, acetate, trifluoroacetate, pantothenate, succinate, citrate, tartrate, fumarate, maleate, gluconate, glucuronate, saccharate, benzoate, lactate, methanesulfonate, ethanesulfonate, benzenesulfonate, *p*-toluenesulfonate, arginine, aspartate, glutamate, pantothenate, and ascorbate.

The present invention also provides a preparation method for the conjugate described above, which may comprise a step of coupling a conjugate (L-D) of a small molecule drug and a linking unit with an antibody.

Specifically, the preparation method further includes steps of reducing and oxidizing a disulfide bond of the antibody before the coupling step.

Specifically, the preparation method includes the following steps:
(1) incubating the antibody with a disulfide bond reducing agent;
(2) incubating the antibody obtained in the step (1) with an oxidant; and
(3) incubating the antibody obtained in the step (2) with the conjugate L-D.

Specifically, the step (1) may further include removing the excess disulfide bond reducing agent (e.g., by ultrafiltration). Specifically, the disulfide bond reducing agent in the step (1) may be tris(2-carboxyethyl)phosphine (TCEP), dithiothreitol (DTT), β-mercaptoethanol, or the like.

Specifically, in the step (1), the equivalent ratio of the antibody to the disulfide bond reducing agent is 1:8-15 (specifically, such as 1:8, 1:9, 1:10, 1:11, or 1:12).

Specifically, the temperature for incubating in the step (1) is 35-40°C (specifically, such as 35°C, 36°C, 37°C, 38°C, 39°C, or 40°C); in one embodiment of the present invention, the temperature is 37°C.

Specifically, the time for incubating in the step (1) is 0.5-6 hours (specifically, such as 0.5, 1, 2, 3, 4, 5, or 6 hours); in one embodiment of the present invention, the time is 3 hours.

Specifically, the step (2) may further include removing the excess oxidant (e.g., by ultrafiltration).

Specifically, the oxidant in the step (2) may be dehydroascorbic acid (DHAA), Cu(II), or the like.

Specifically, in the step (2), the equivalent ratio of the antibody to the oxidant is 1:40-60 (specifically, such as 1:40, 1:45, 1:50, 1:55, or 1:60).

Specifically, the temperature for incubating in the step (2) is 20-30°C (specifically, such as 20°C, 22°C, 24°C, 25°C, 26°C, 28°C, or 30°C); in one embodiment of the present invention, the temperature is room temperature.

Specifically, the time for incubating in the step (2) is 1-6 hours (specifically, such as 1, 2, 3, 4, 5, or 6 hours); in one embodiment of the present invention, the time is 3 hours.

Specifically, in the step (3), the equivalent ratio of the antibody to the small molecule L-D is 1:10-20 (specifically, such as 1:10, 1:12, 1:14, 1:15, 1:16, 1:18, or 1:20).

Specifically, the temperature for incubating in the step (3) is 20-30°C (specifically, such as 20°C, 22°C, 24°C, 25°C, 26°C, 28°C, or 30°C); in one embodiment of the present invention, the temperature is room temperature.

Specifically, the time for incubating in the step (3) is 6-48 hours (specifically, such as 6, 12, 18, 20, 22, 24, 26, 28, 30, 36, 42, or 48 hours); in one embodiment of the present invention, the time is 24 hours.

In one embodiment of the present invention, the preparation method further includes a step of preparing the conjugate (L-D) of a small molecule drug and a linking unit.

The present invention also provides a modified anti-PD-L1 antibody, which is obtained by mutating one or more amino acid residues of a known anti-PD-L1 antibody (e.g., atezolizumab, durvalumab, avelumab, cemiplimab, KN035, CS1001, BGB-A333, KL-A167, SHR-1316, STI-A1014, etc.) into cysteine by genetic engineering means (e.g., Thiomab technology), respectively, for example, by mutating amino acid(s) in heavy chains and/or light chains of avelumab into cysteine.

Specifically, the antibody described above comprises heavy chains and light chains;
wherein the heavy chain has an amino acid sequence set forth in SEQ ID NO: 4, or an amino acid sequence having at least 80%, 85%, 90%, 91%, 92%, 93%, 94%, 95%, 96%, 97%, 98% or 99% identity to SEQ ID NO: 4; and/or,
the light chain has an amino acid sequence set forth in SEQ ID NO: 9, or an amino acid sequence having at least 80%, 85%, 90%, 91%, 92%, 93%, 94%, 95%, 96%, 97%, 98% or 99% identity to SEQ ID NO: 9.

Specifically, the antibody described above has a light chain sequence set forth in SEQ ID NO: 9.

In one embodiment of the present invention, the antibody described above has a heavy chain sequence set forth in SEQ ID NO: 4 and a light chain sequence set forth in SEQ ID NO: 9.

The present invention also provides use of the modified anti-PD-L 1 antibody described above in the preparation of an antibody-drug conjugate (ADC).

The present invention also provides a small molecule compound and a pharmaceutically acceptable salt, a stereoisomer, a solvate and a prodrug thereof, wherein the compound has the following structure: 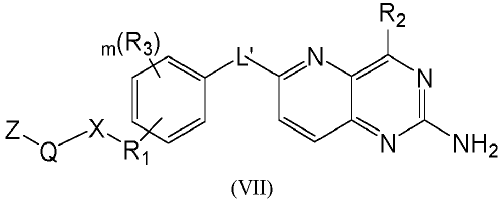
wherein m, X, L', R₁, R₂, R₃, and Q have the corresponding definitions described above in the present invention, and
Z is an active group.

In one embodiment of the present invention, the small molecule compound has the following structure: 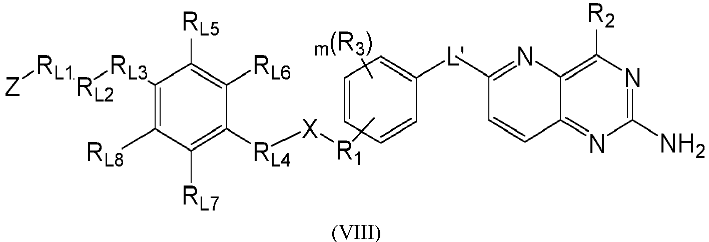
wherein m, X, L', R₁, R₂, R₃, R_{L1}, R_{L2}, R_{L3}, R_{L4}, R_{L5}, R_{L6}, R_{L7}, and R_{L8} have the corresponding definitions described above in the present invention, and
Z is an active group.

Specifically, Z is a reactive group for bonding with an active group of an amino acid (e.g., sulfydryl, amino, carboxyl, etc., in particular sulfydryl); specifically, for example, the sulfydryl-reactive group may be maleimide, carboxyl, amido, halogen, disulfide, or the like; the amino-reactive group may be an ester group, acyl halide, acid anhydride, carboxyl, epoxy, or the like; and the carboxyl/amino-reactive group may be hydroxyl, amino, halogen, acyl halide, or the like.

In some embodiments of the present invention, Z is

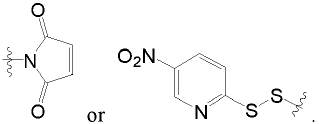

Specifically, the compound of formula VII has the following structure: 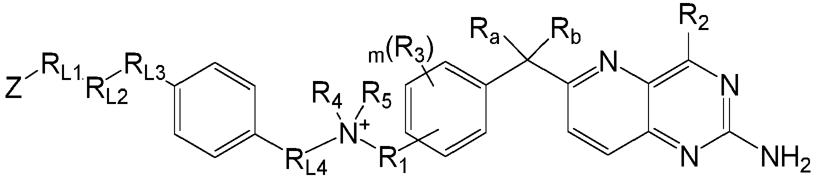 wherein Z, R_{L1}, R_{L2}, R_{L3}, R_{L4}, R₁, R₂, R₃, R₄, R₅, Rₐ, R_{b}, and m have the corresponding definitions described above in the present invention.

More specifically, the compound of formula VII has the following structure: 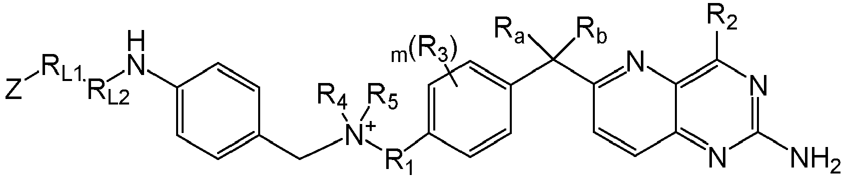 wherein Z, R_{L1}, R_{L2}, R₁, R₂, R₃, R₄, R₅, Rₐ, R_{b}, and m have the corresponding definitions described above in the present invention.

Further specifically, the compound of formula VII has the following structure: 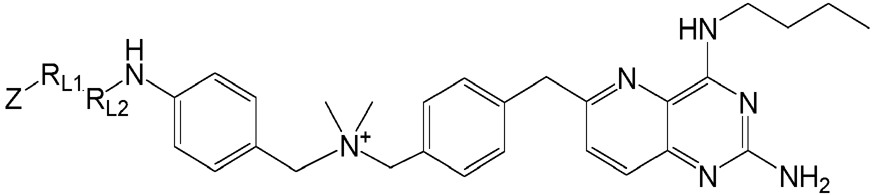 wherein Z, R_{L1} and R_{L2} have the corresponding definitions described above in the present invention.

In some embodiments of the present invention, the compound of formula VII has the following structure:

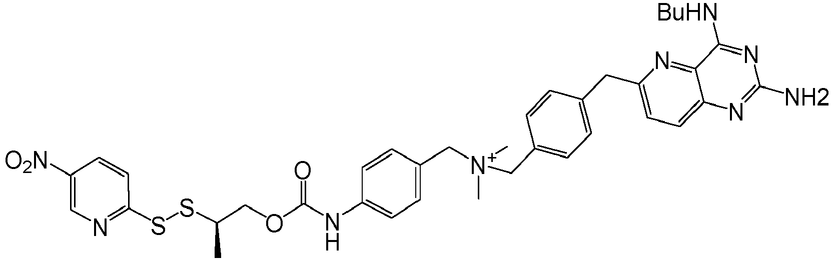

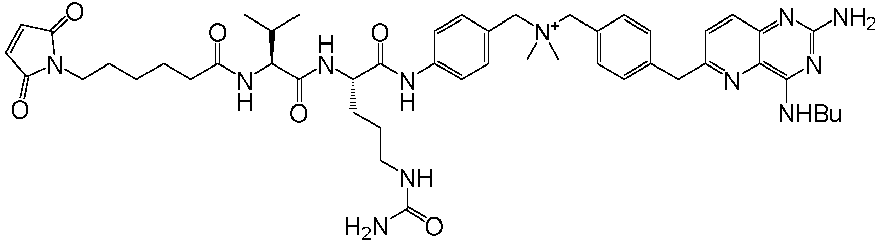

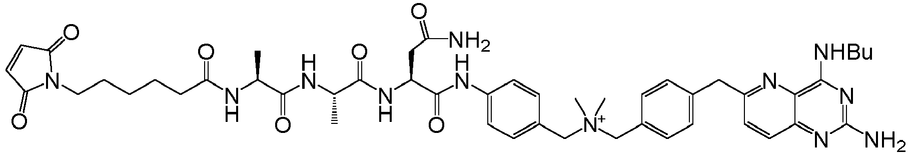

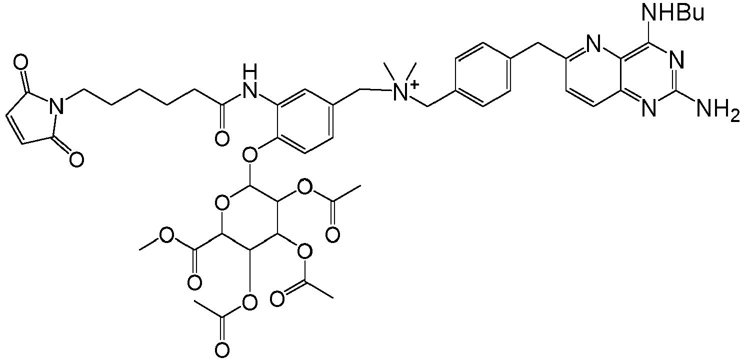

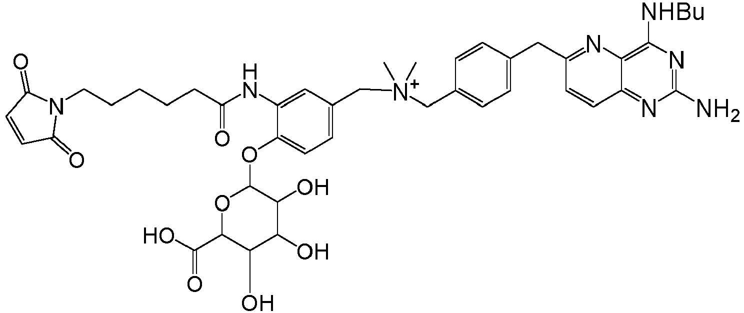

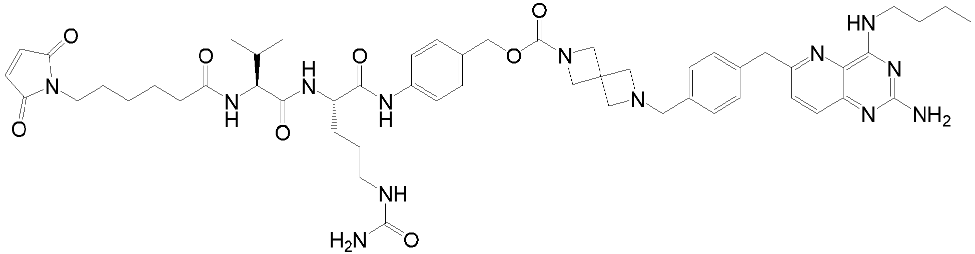

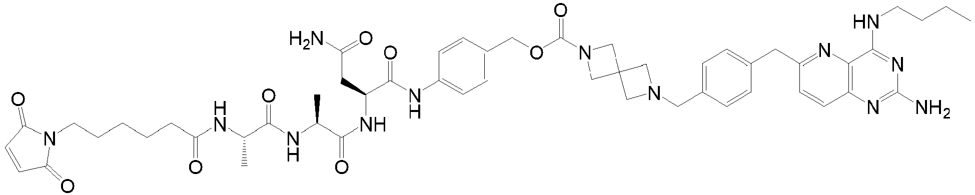

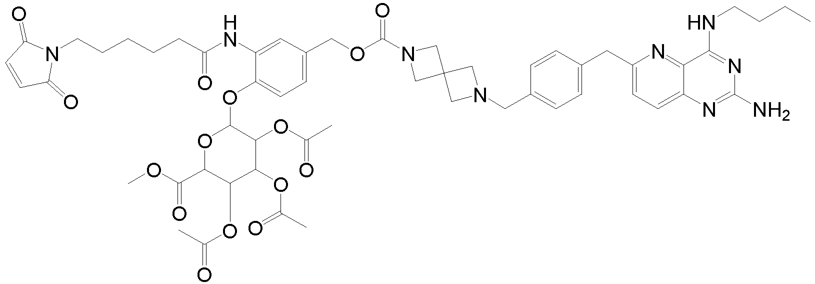

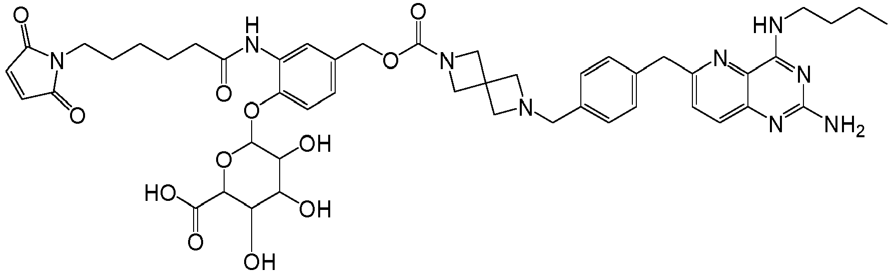

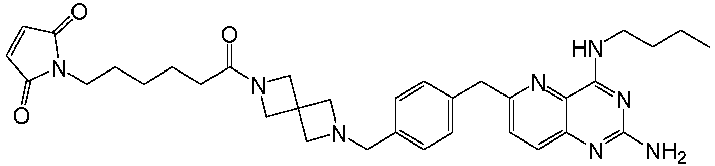

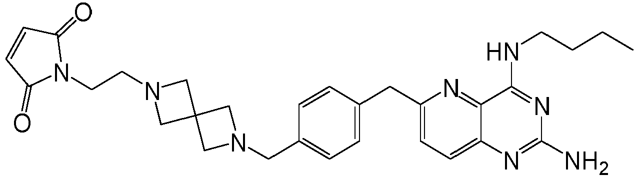

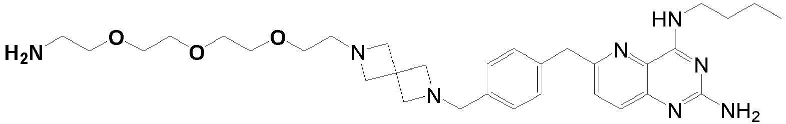

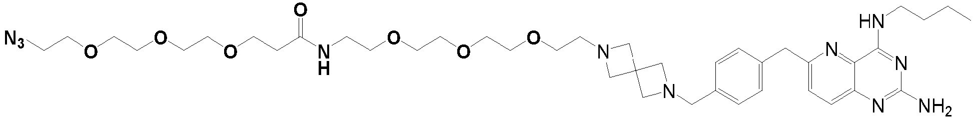

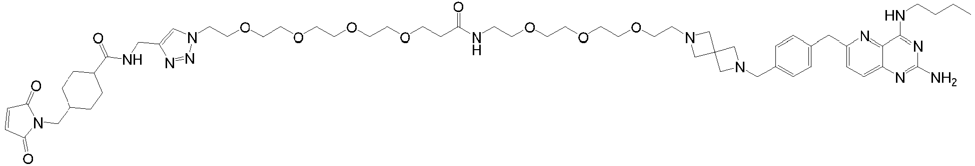

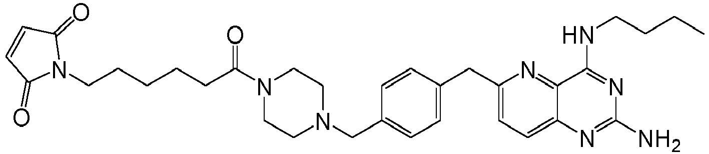

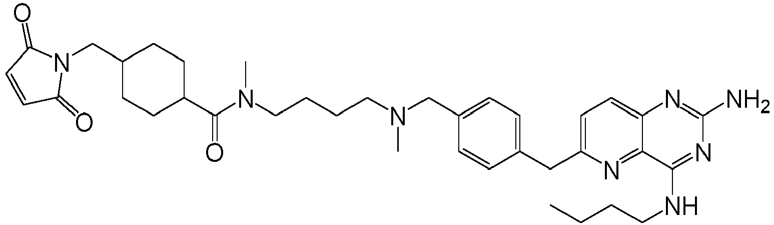

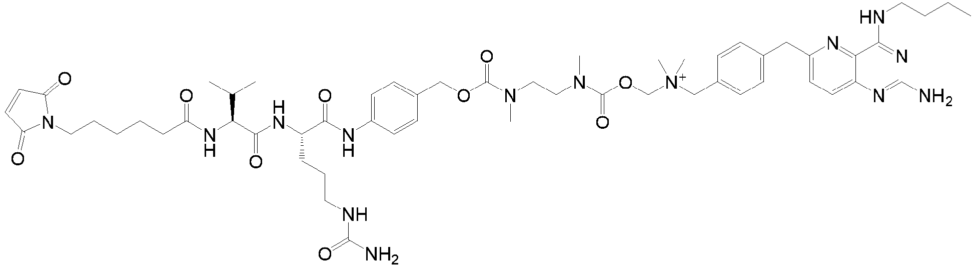

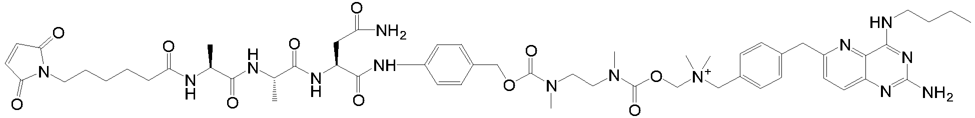

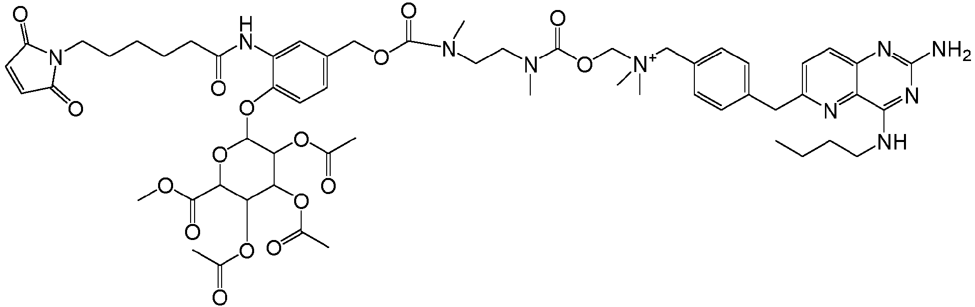

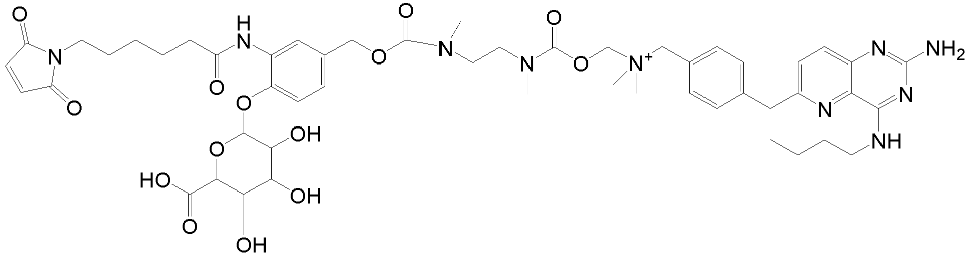

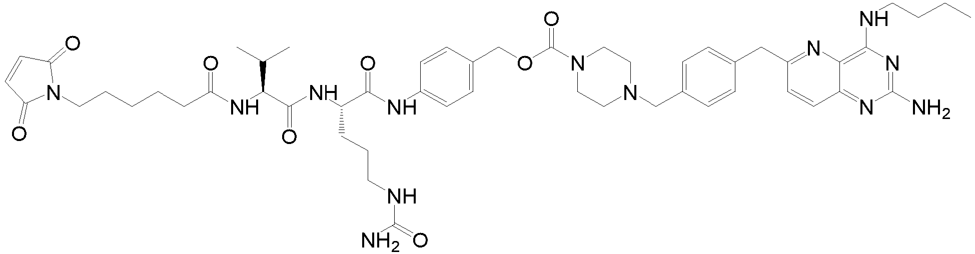

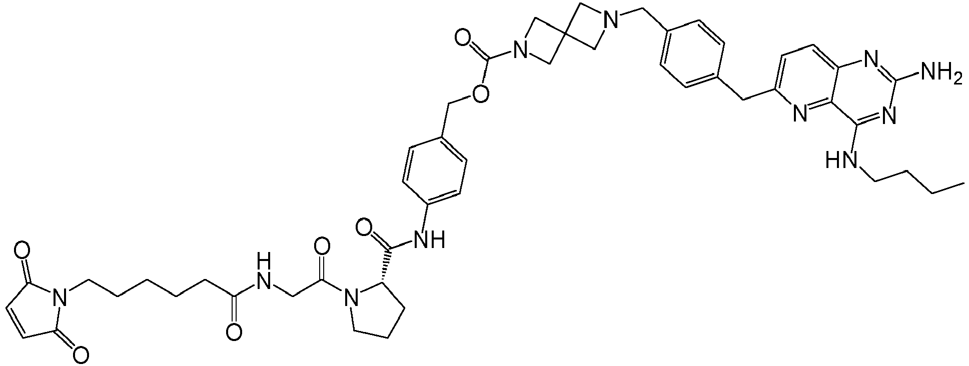

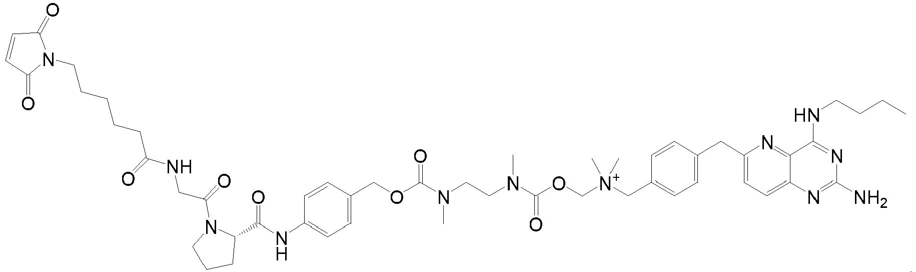

The present invention also provides a compound and a pharmaceutically acceptable salt and a stereoisomer thereof, which may be used as a linking unit moiety of the antibody-drug conjugate and has the following structure: 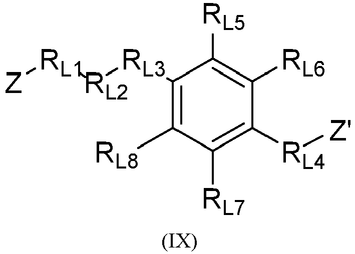
wherein Z, R_{L1}, R_{L2}, R_{L3}, R_{L4}, R_{L5}, R_{L6}, R_{L7}, and R_{L8} have the corresponding definitions described above in the present invention;
Z' is a reactive group.

Specifically, Z' may be halogen (e.g., chlorine), carboxyl, an ester group, hydroxyl, epoxy, amino, or the like.

Specifically, the compound of formula IX has the following structure: 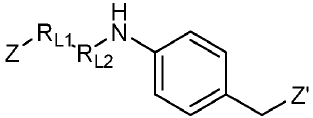 wherein Z, R_{L1}, R_{L2}, and Z' have the corresponding definitions described above in the present invention.

In some embodiments of the present invention, the compound of formula IX has the following structure: 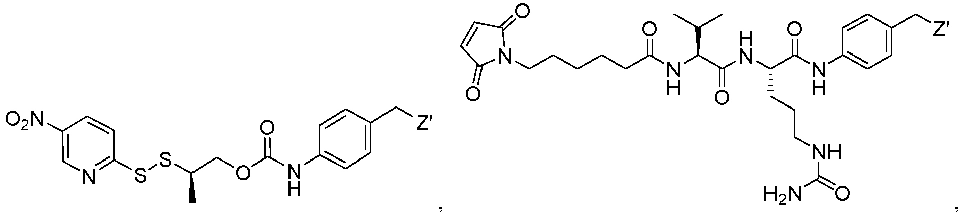 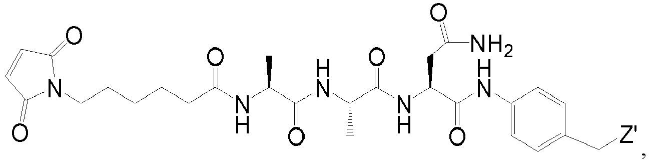 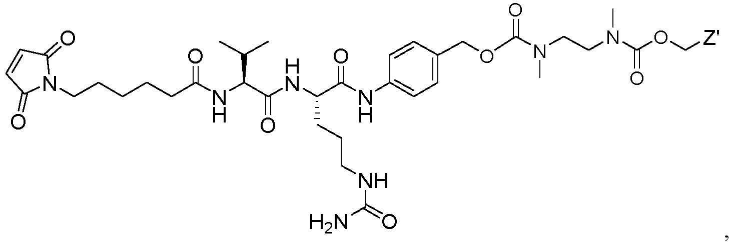 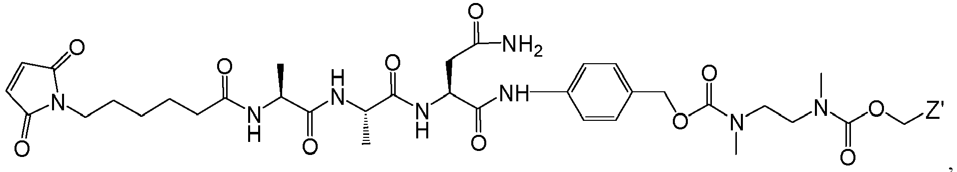 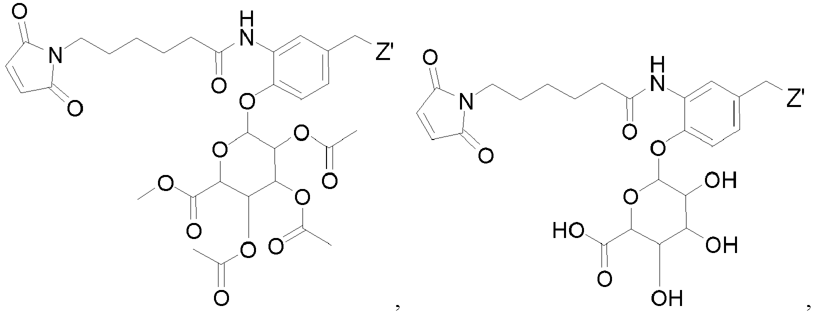 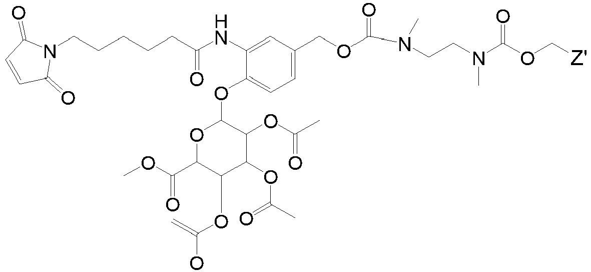 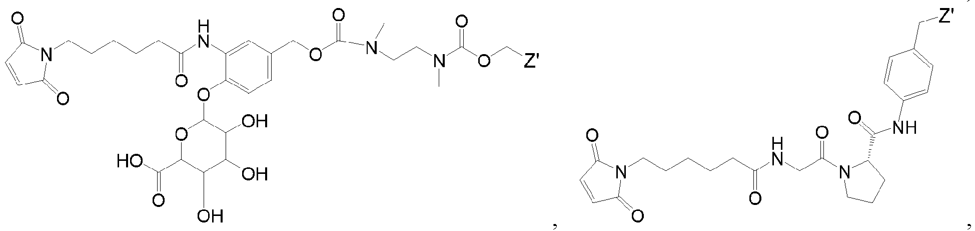 and

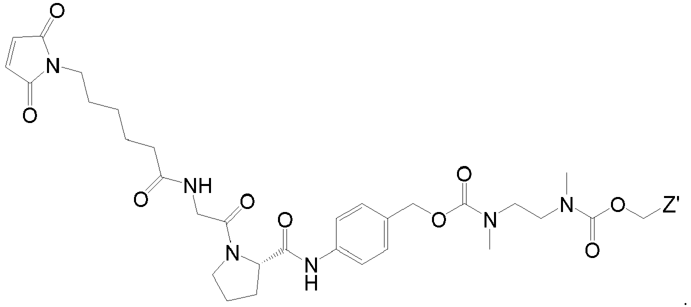

In some embodiments of the present invention, Z' is halogen (e.g., chlorine).

In some embodiments of the present invention, Z' is a carbonate group (e.g., 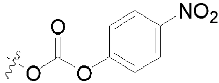 ).

The present invention also provides use of the compound of formula IX in the preparation of an antibody-drug conjugate (ADC).

The present invention also provides a pharmaceutical composition comprising the conjugate described above and a pharmaceutically acceptable auxiliary material.

Specifically, the pharmaceutically acceptable auxiliary material described above is a conventional pharmaceutical auxiliary material in the pharmaceutical field, for example, a diluent and an excipient, such as water, etc.; a filler such as starch and sucrose, etc.; a binder such as cellulose derivatives, alginates, gelatin, polyvinylpyrrolidone, etc.; a wetting agent such as glycerol, etc.; a disintegrant such as agar, calcium carbonate, sodium bicarbonate, etc.; an absorption promoter such as quaternary ammonium compounds, etc; a surfactant such as cetanol, etc.; an adsorption carrier such as kaolin, bentonite, etc.; a lubricant such as talc powder, calcium stearate, magnesium stearate, polyethylene glycol, etc. In addition, other adjuvants such as flavoring agents, sweetening agents, stabilizers, etc., may also be added to the pharmaceutical composition.

Specifically, the pharmaceutical composition may comprise about 1 wt% to about 99 wt% (specifically, such as 1%, 5%, 10%, 20%, 30%, 40%, 50%, 60%, 70%, 80%, 90%, 95%, or 99%) of the conjugate of the present invention, and the balance of suitable auxiliary materials, according to a desired mode of administration.

Specifically, the pharmaceutical composition may be used for administration by any mode of administration, either orally or parenterally, such as by pulmonary, nasal, rectal and/or intravenous injection. Thus, the formulation of the present invention may be suitable for local or systemic administration, in particular for dermal, subcutaneous, intramuscular, intra-articular, intraperitoneal, pulmonary, buccal, sublingual, nasal, transdermal puncture, vaginal, oral, or parenteral administration. The preferred form for rectal administration is suppositories.

The dosage forms suitable for oral administration are tablets, pills, chewing gums, capsules, granules, drops or syrups, and the like. The dosage forms suitable for parenteral administration are solutions, suspensions, reconstitutable driers or sprays, and the like.

The composition of the present invention may be formulated into a deposit or patch in a dissolved form for transdermal administration. Dermal applications include ointments, gels, creams, lotions, suspensions or emulsions, and the like.

The various dosage forms of the pharmaceutical composition of the present invention may be prepared according to conventional production methods in the pharmaceutical field, for example, by mixing active ingredients with one or more auxiliary materials and then formulating the resulting mixture into a desired dosage form.

Specifically, in the pharmaceutical composition described above, the conjugate of the present invention may be used as the sole active ingredient or may be used in combination with one or more additional active ingredients for the same indication, wherein the conjugate of the present invention and the additional active ingredients may be formulated for simultaneous, separate or sequential administration.

The present invention also provides a composition comprising the antibody and the small molecule drug of the present invention as active ingredients.

Specifically, in the composition described above, the antibody is an anti-PD-L1 antibody, e.g., atezolizumab, durvalumab, avelumab, cemiplimab, KN035, CS1001, BGB-A333, KL-A167, SHR-1316, STI-A1014, etc., and a modified anti-PD-L1 antibody obtained by mutating one or more amino acid residues of a known anti-PD-L 1 antibody (as described above) into cysteine by genetic engineering means, for example, by mutating amino acid(s) in heavy chains and/or light chains of avelumab into cysteine.

Specifically, the antibody described above comprises heavy chains and light chains;
wherein the heavy chain has an amino acid sequence set forth in SEQ ID NO: 4, or an amino acid sequence having at least 80%, 85%, 90%, 91%, 92%, 93%, 94%, 95%, 96%, 97%, 98% or 99% identity to SEQ ID NO: 4; and/or,
the light chain has an amino acid sequence set forth in SEQ ID NO: 9, or an amino acid sequence having at least 80%, 85%, 90%, 91%, 92%, 93%, 94%, 95%, 96%, 97%, 98% or 99% identity to SEQ ID NO: 9.

Specifically, the antibody described above has a light chain sequence set forth in SEQ ID NO: 9.

In one embodiment of the present invention, the antibody described above has a heavy chain sequence set forth in SEQ ID NO: 4 and a light chain sequence set forth in SEQ ID NO: 9.

Specifically, in the composition described above, the small molecule drug may be an immunomodulator, e.g., a Toll-like receptor (TLR) agonist, specifically, a TLR7 and/or TLR8 agonist, such as a pyridopyrimidine derivative and a salt thereof described in patent application publication No. WO2019/095455A1.

Specifically, the small molecule drug in the composition has the following structure: 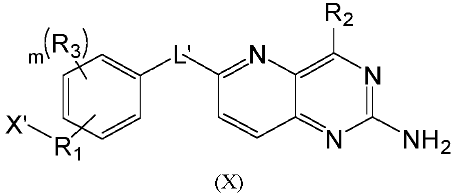
wherein R₁, R₂, R₃, m, and L' have the corresponding definitions described above in the present invention;
X' is selected from: H, halogen, -NR₄R₅, -NR₄R₅, -OR₄, -SR₄, azido, phosphonic acid group, phosphonate diethyl, haloalkyl, carboxyl, ester group, and -CN, wherein R₄ and R₅ are independently selected from: H, C₁-C₆ alkyl, C₁-C₆ alkoxy, amino acid residue, oligopeptide residue, and haloalkyl, or, R₄ and R₅, together with the nitrogen atom to which they are attached, form heterocyclyl.

More specifically, the small molecule drug in the composition may be selected from the following structures: 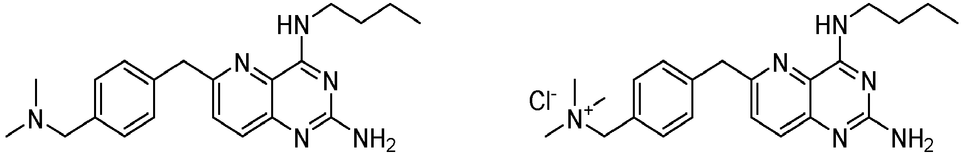 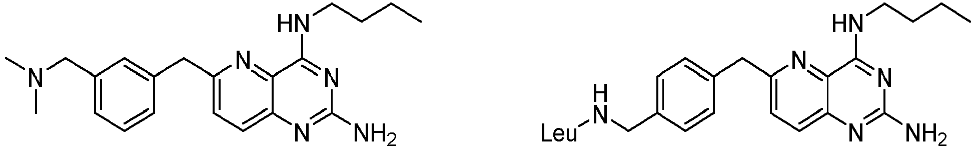 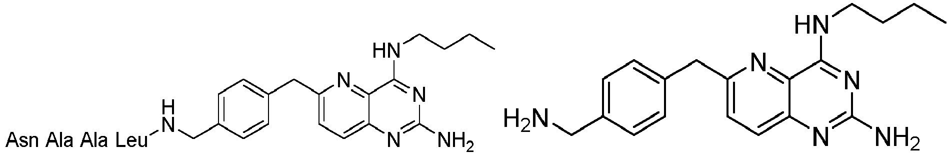 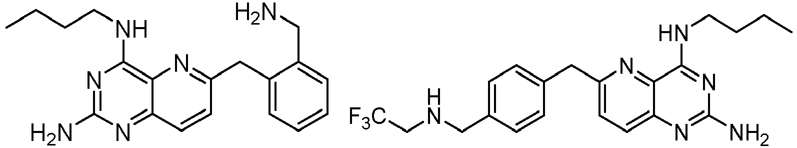 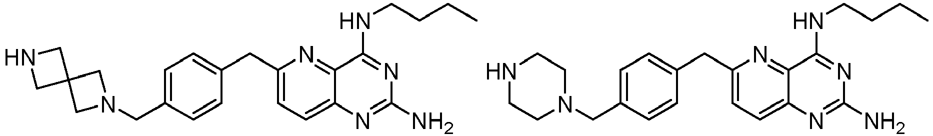 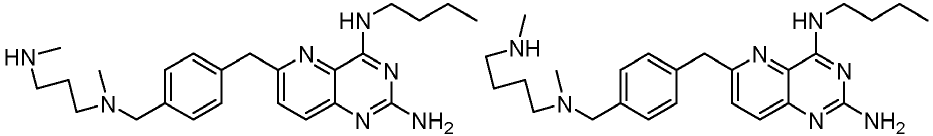 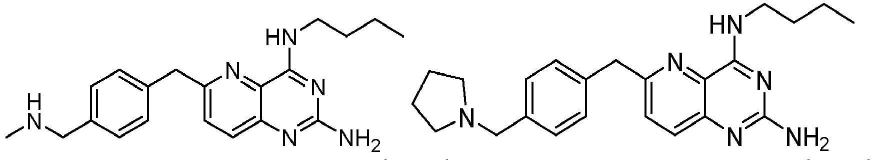 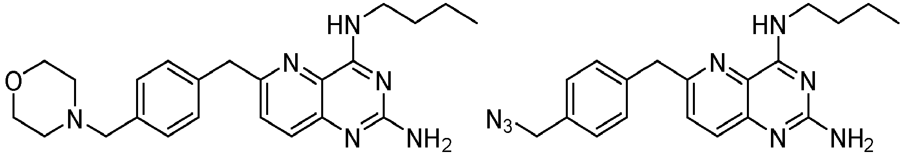 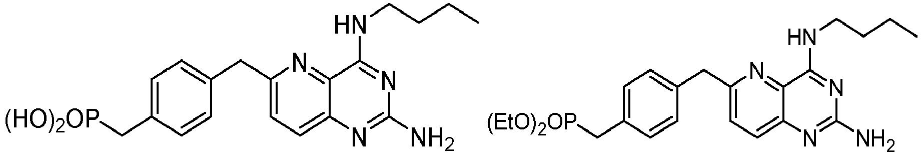 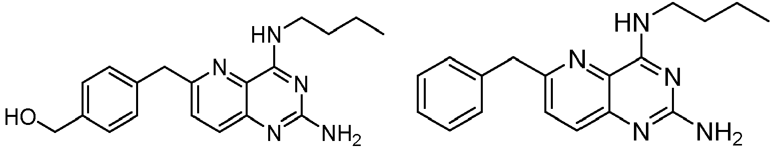 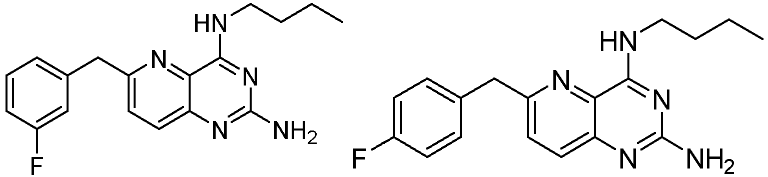 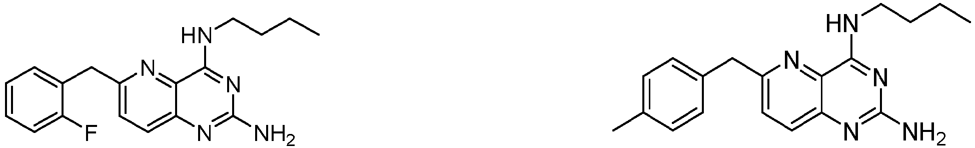 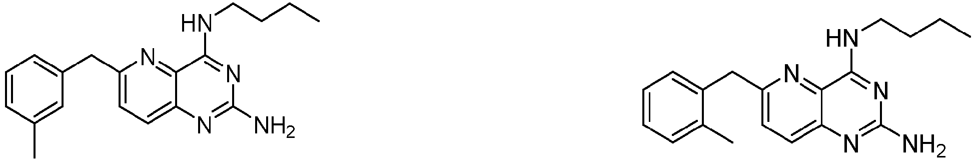 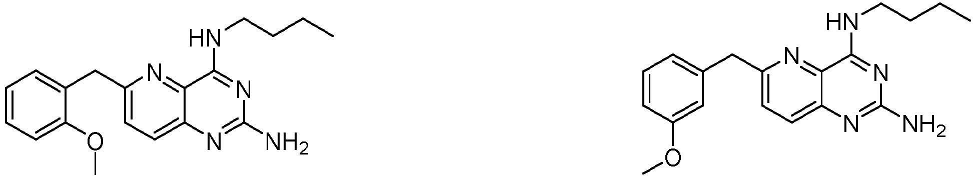 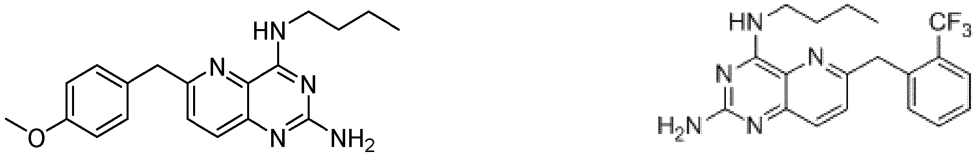 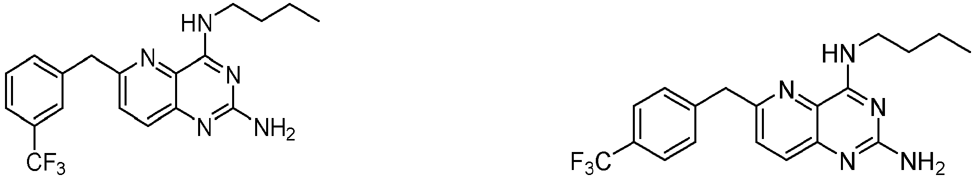 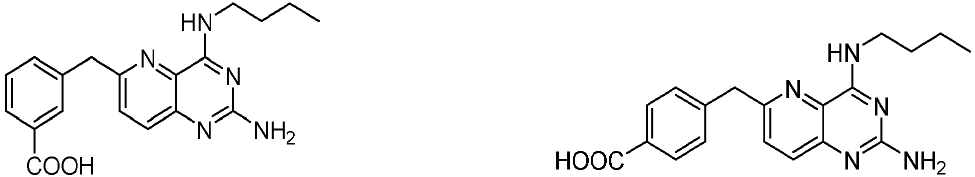 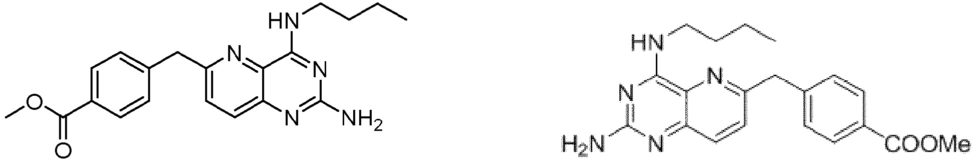 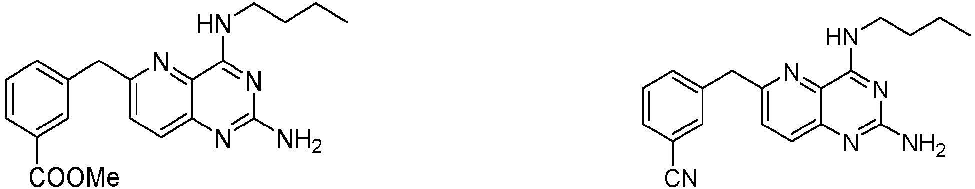 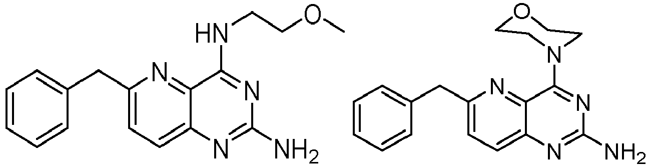 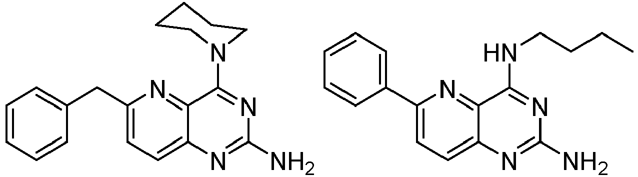 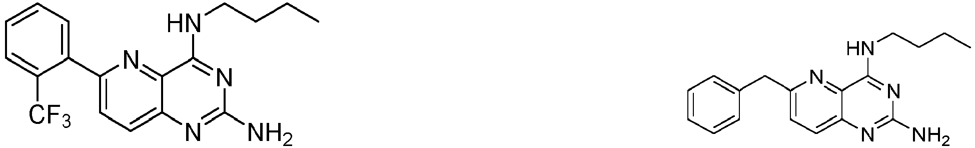 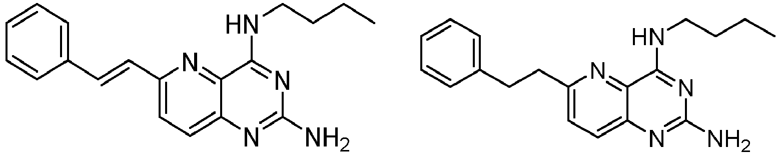 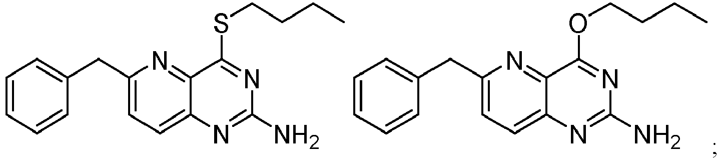 in particular:

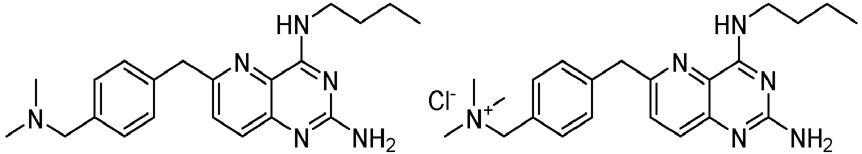

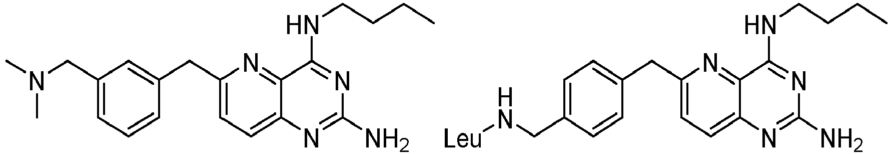

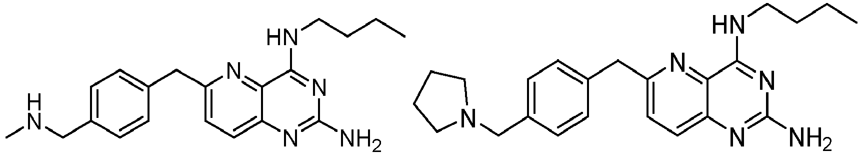

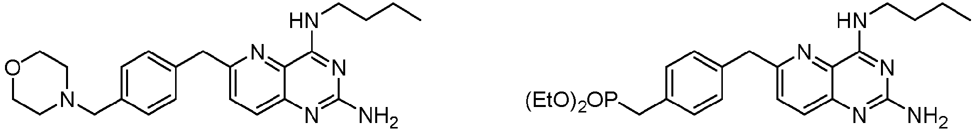

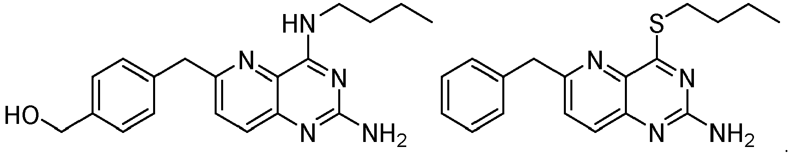

In one embodiment of the present invention, in the composition described above, the antibody is avelumab, and the small molecule drug has the following structure:

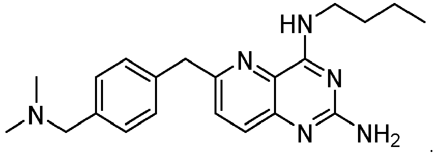

The present invention also provides use of the conjugate or the pharmaceutically acceptable salt, the solvate, the prodrug and the stereoisomer thereof, the antibody, the compound of formula VII, and the composition as described above in the preparation of a medicament for preventing and/or treating diseases.

Specifically, the diseases may be diseases associated with PD-L1 expression, and may also be diseases associated with TLR7 and/or TLR8 activity.

Specifically, the diseases may be one or more of respiratory diseases, immune diseases, viral diseases, and tumors.

Specifically, the respiratory diseases described above include, but are not limited to: asthma, chronic obstructive pulmonary disease, and adult respiratory distress syndrome.

Specifically, the immune diseases described above are autoimmune diseases, including but not limited to: systemic lupus erythematosus, rheumatoid arthritis, inflammatory bowel disease, Sjogren's syndrome, polymyositis, vasculitis, Wegener granulomatosis, sarcoidosis, ankylosing spondylitis, Reiter's syndrome, psoriatic arthritis, Behcet's syndrome, and the like.

Specifically, pathogens of the viral diseases described above include, but are not limited to: Adenoviridae (e.g., adenovirus), Herpesviridae (e.g., HSV1 (herpes of mouth), HSV2 (herpes of external genitalia), VZV (chicken pox), EBV (Epstein-Barr virus), CMV (cytomegalovirus)), Poxviridae (e.g., smallpox virus, vaccinia virus), Papovavirus (e.g., papilloma virus), Parvoviridae (e.g., B19 virus), Hepadnaviridae (e.g., hepatitis B virus), Polyomaviridae (e.g., polyomavirus), Reoviridae (e.g., reovirus, rotavirus), Picornaviridae (e.g., enterovirus, foot-and-mouth disease virus), Caliciviridae (e.g., Norwalk virus, hepatitis E virus), Togaviridae (e.g., rubella virus), Arenaviridae (e.g., lymphocytic choriomeningitis virus), Retroviridae (HIV-1, HIV-2, HTLV-1), Flaviviridae (e.g., Dengue virus, Zika virus, encephalitis B virus, Chikungunya virus, yellow fever virus, hepatitis C virus, West Nile virus, etc.), Orthomyxoviridae (e.g., influenza virus (e.g., influenza A virus, influenza B virus, influenza C virus, etc.)), Paramyxoviridae (e.g., human parainfluenza virus (HPV) type 1, HPV type 2, HPV type 3, HPV type 4, Sendai virus, mumps virus, measles virus, respiratory syncytial virus, Newcastle disease virus, etc.), Bunyaviridae (e.g., California encephalitis virus, Hantavirus), Rhabdoviridae (e.g., rabies virus), Filoviridae (e.g., Ebola virus, Marburg virus), Coronaviridae (e.g., HCoV-229E, HCoV-OC43, HCoV-NL63, HCoV-HKU1, SARS-CoV, MERS-CoV, SARS-CoV-2, etc.), Astroviridae (e.g., astrovirus), and Bomaviridae (e.g., Borna virus); more specifically, the viral diseases may be influenza, SARS, C0VID-19, viral hepatitis (e.g., hepatitis A, hepatitis B, hepatitis C, hepatitis D, etc.), AIDS, rabies, Dengue virus, Ebola virus disease, etc.

Specifically, the tumors described above are malignancies, including but not limited to: lymphoma, blastoma, medulloblastoma, retinoblastoma, liposarcoma, synovial cell sarcoma, neuroendocrine tumor, carcinoid tumor, gastrinoma, islet cell carcinoma, mesothelioma, schwannoma, acoustic neuroma, meningioma, adenocarcinoma, melanoma, leukemia and lymphoid malignancy, squamous cell carcinoma, epithelial squamous cell carcinoma, lung carcinoma (small cell lung carcinoma, non-small cell lung carcinoma, adenocarcinoma lung carcinoma, squamous lung carcinoma), peritoneal carcinoma, hepatocellular carcinoma, gastric carcinoma, intestinal carcinoma, pancreatic carcinoma, glioblastoma, cervical carcinoma, ovarian carcinoma, liver carcinoma, bladder carcinoma, breast carcinoma, metastatic breast carcinoma, colon carcinoma, rectal carcinoma, colorectal carcinoma, uterine carcinoma, salivary gland carcinoma, kidney carcinoma, prostate carcinoma, vulval carcinoma, thyroid carcinoma, anal carcinoma, penile carcinoma, Merkel cell carcinoma, esophageal carcinoma, biliary tract carcinoma, head and neck carcinoma, and hematological malignancies; in particular colon carcinoma, bladder carcinoma, melanoma, meningioma, lung carcinoma, and pancreatic carcinoma.

The present invention also provides a method for preventing and/or treating diseases, which comprises a step of administering to a system or a subject in need thereof a therapeutically effective amount of the conjugate or the pharmaceutically acceptable salt, the solvate, the prodrug, or the stereoisomer thereof, the antibody, the compound of formula VII, and the composition as described above.

Specifically, the diseases in the method have the definitions described above in the present invention.

The present invention also provides a method for enhancing a positive immune response in a body, which comprises a step of administering to a system or a subject in need thereof a therapeutically effective amount of the conjugate or the pharmaceutically acceptable salt, the solvate, the prodrug, or the stereoisomer thereof, the antibody, the compound of formula VII, and the composition as described above.

The present invention also provides a method for enhancing the effect of a chemotherapy, which comprises a step of administering to a system or a subject in need thereof a therapeutically effective amount of the conjugate or the pharmaceutically acceptable salt, the solvate, the prodrug, or the stereoisomer thereof, the antibody, the compound of formula VII, and the composition as described above.

The present invention also provides a method for enhancing the effect of an immunotherapy, which comprises a step of administering to a system or a subject in need thereof a therapeutically effective amount of the conjugate or the pharmaceutically acceptable salt, the solvate, the prodrug, or the stereoisomer thereof, the antibody, the compound of formula VII, and the composition as described above.

Specifically, the therapeutically effective amount described above may vary according to the mode of administration, the age, body weight and sex of the patient, the type and severity of the disease to be treated, and other factors, which is not particularly limited in the present invention.

In the present invention, a modified anti-PD-L1 antibody having two mutated cysteines is obtained by means of gene editing, which basically retains the structure of the original antibody, and is used for construction of ADCs. Experiments prove that the modified antibody and the obtained ADCs maintain the antigen recognition affinity of the original anti-PD-L1 antibody, can be internalized into cells by binding to PD-L1 on cell surfaces, and have good selectivity. It has been found in anti-tumor experiments that the obtained ADCs have strong anti-tumor activity, can significantly improve the survival rate of tumor-bearing animals, have toxicity significantly lower than that for single administration of the small molecule drug and combined administration of the small molecule drug and the antibody, and have small burden on the bodies of laboratory animals. The ADCs of the present invention greatly reduce the minimum effective dose of the small molecule drug when used alone, expand the therapeutic window thereof, are expected to be used in the development of treatment drugs for various diseases (such as tumors, viral diseases such as hepatitis B, etc.), and have good application prospects and value.

### BRIEF DESCRIPTION OF THE DRAWINGS

FIG. 1 shows the sequencing results of plasmid αPD-L1 THIOMAB LC-V205C, wherein, A. the comparison of the αPD-L1 THIOMAB LC-V205C sequencing sequence (upper) and the sequence of the template light chain 159-pFuse-αPDL1-LC plasmid (lower) shows that the GTG site on the template plasmid was mutated into TGC; B. the amino acid encoded at the mutation site of αPD-L1 THIOMAB LC-V205C was cysteine (Cys); and C. the amino acid encoded at the mutation site of the template plasmid was valine (Val).
FIG. 2 shows the results of identifying the expression of anti-PD-L1 antibody by reduced and non-reduced SDS PAGE analysis.
FIG. 3 shows the results of identifying the expression of anti-PD-L 1 THIOMAB by reduced and non-reduced SDS PAGE analysis.
FIG. 4 shows the mass spectrometry analysis results for THIO-S2.
FIG. 5 shows the coupling route for ADC HE-S2.
FIG. 6 shows the mass spectrometry detection results for ADC HE-S2.
FIG. 7 shows the coupling route for ADC VC.
FIG. 8 shows the coupling route for ADC Legumain.
FIG. 9 shows the mass spectrometry detection results for ADC VC and ADC Legumain.

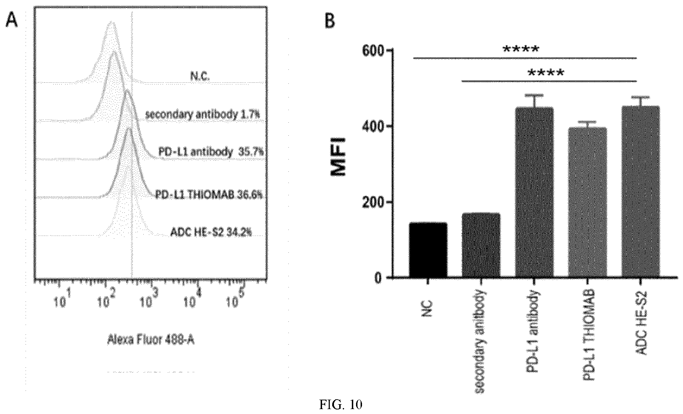

FIG. 10 shows the characterization results of antibody-antigen affinity of ADC HE-S2 and anti-PD-L1 THIOMAB. MC38 cells were treated with anti-PD-L1 antibody, anti-PD-L1 THIOMAB and ADC HE-S2, and then stained with FITC-labeled goat anti-human secondary antibody; the FITC signal intensity on MC38 surfaces was analyzed by FACS technique detection. A. The histograms of different colors represent, from top to bottom, the fluorescence signals measured for the negative control (NC) group, the secondary antibody group, the anti-PD-L1 antibody group, the anti-PD-L1 THIOMAB group, and the ADC HE-S2 group, respectively. B. Mean fluorescence intensity (MFI) measured after treatment with different antibody-based drugs on MC38. NC group: the negative control only added with the isotype antibody, with no secondary antibody added for staining; secondary antibody group: the control group added with the isotype antibody and stained with fluorescent secondary antibody. Student's t test was used to compare significant differences between the groups, and *p* < 0.0001 is represented by ****.

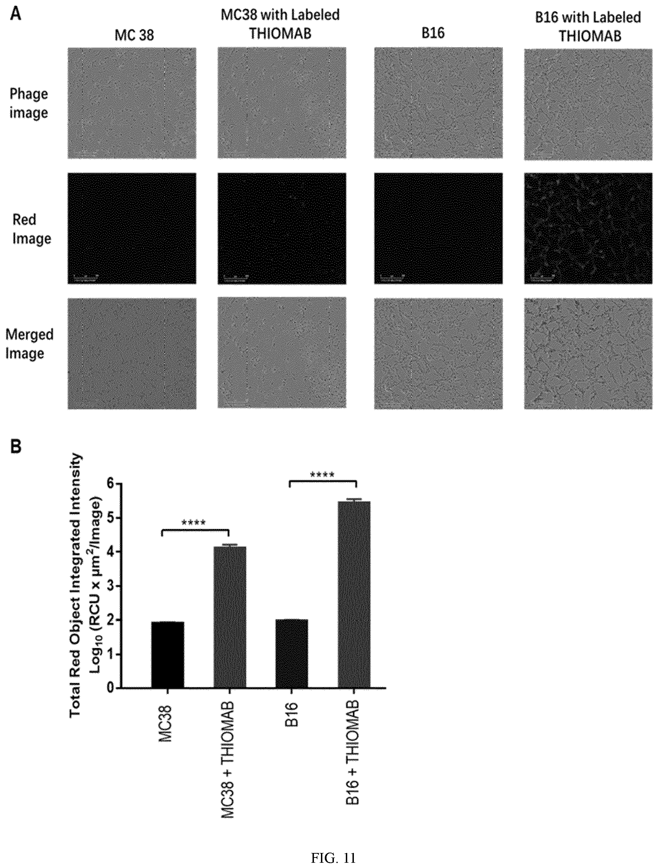

FIG. 11 shows the experimental results in which anti-PD-L 1 THIOMAB was internalized into cancer cells. After MC38 and B16 cells were treated for 24 hours with anti-PD-L 1 THIOMAB labeled with pHrodo^{™} red dye, A. high-definition phase images, red fluorescence images and combined images of cells were collected by using an IncuCyte^{®} viable cell system; B. a total red object integrated intensity histogram was obtained by analysis using IncuCyte^{®} software, wherein the logarithm of 10 was taken for the ordinate data. Student's t test was used to compare significant differences between the groups, and *p* < 0.0001 is represented by ****.
FIG. 12 shows the results of FACS detection of PD-L1 levels on the surfaces of *Cd274* knockout and wild-type DC 2.4 cells. KO: C*d274* knockout DC 2.4 cells, WT: wild-type DC 2.4 cells. Student's t test was used to compare significant differences between the groups, and *p* < 0.01 is represented by **.
FIG. 13 shows the results of a preliminary experiment of internalization of anti-PD-L 1 THIOMAB in DC cells. PD-L1 positive wild-type DC 2.4 cells and PD-L1 negative C*d274* knockout DC 2.4 cells were cultured in a 10% FBS culture medium, anti-PD-L1 THIOMAB labeled with pHrodo^{™} red dye was added after the cell state was stable, and the cells were incubated overnight. The intracellular red fluorescence intensity was then analyzed by FACS to characterize the internalization level of THIOMAB.

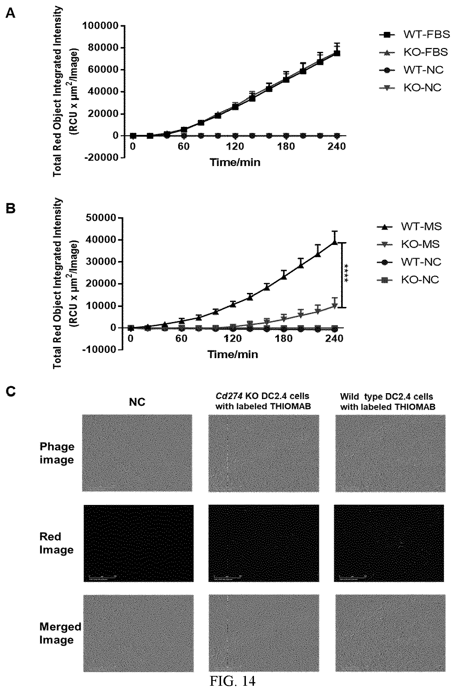

FIG. 14 shows the experimental results of the internalization capacity of the PD-L1/anti-PD-L1 antibody complex in DC cells. The total red object integrated intensity-time variation curves for anti-PD-L1 THIOMAB labeled with pHrodo^{™} red dye in the wild-type DC 2.4 cell line and the C*d274* knockout DC 2.4 cell line. The cells were incubated at 4°C for 30 minutes to transiently inhibit the internalization process of the receptors on the cell surfaces. After addition of labeled anti-PD-L1 antibody, incubation was performed for 30 minutes at 4°C to allow complete binding of antigen and antibody. The internalization of the antibodies was then analyzed by using the IncuCyte^{®} viable cell system. A. Cells were cultured in a culture medium containing 10% FBS. B. Cells were cultured in a culture medium containing 10% mouse serum. C. After 6 hours, high-definition phase images, red fluorescence images and combined images of DC 2.4 cells were collected by using the IncuCyte^{®} viable cell system. Student's t test was used to compare significant differences between the groups, and *p* < 0.0001 is represented by ****.

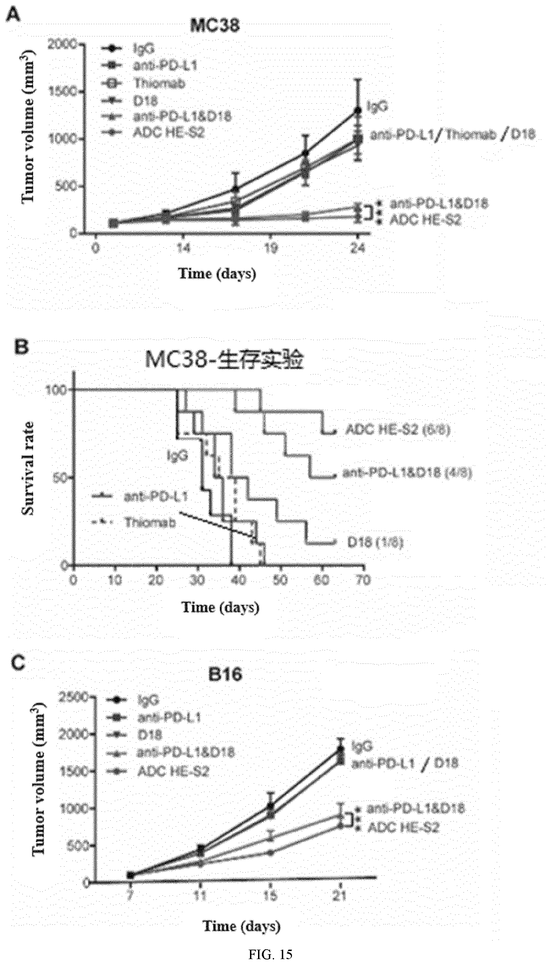

FIG. 15 shows the experimental results of verifying the anti-tumor activity of ADC HE-S2 in a mouse tumor model. A. Treatment with ADC HE-S2, single administration of anti-PD-L 1 antibody, D18, or anti-PD-L1 THIOMAB, and anti-PD-L1 antibody/D18 combined administration for inhibitory activity against mouse MC38 tumor growth. B. Whole animal survival study. The ordinate of the curve represents the percentage of tumor-bearing mice that remained alive after different days, and the abscissa thereof represents the number of experimental days. C. Treatment with ADC HE-S2, single administration of anti-PD-L1 antibody, D18, or anti-PD-L1 THIOMAB, and anti-PD-L1 antibody/D18 combined administration for inhibitory activity against mouse MC38 tumor growth. The dose and time of administration for each group of mice are shown in Table 1. Student's t test was used to compare significant differences between the groups, and *p* < 0.001 is represented by ***.

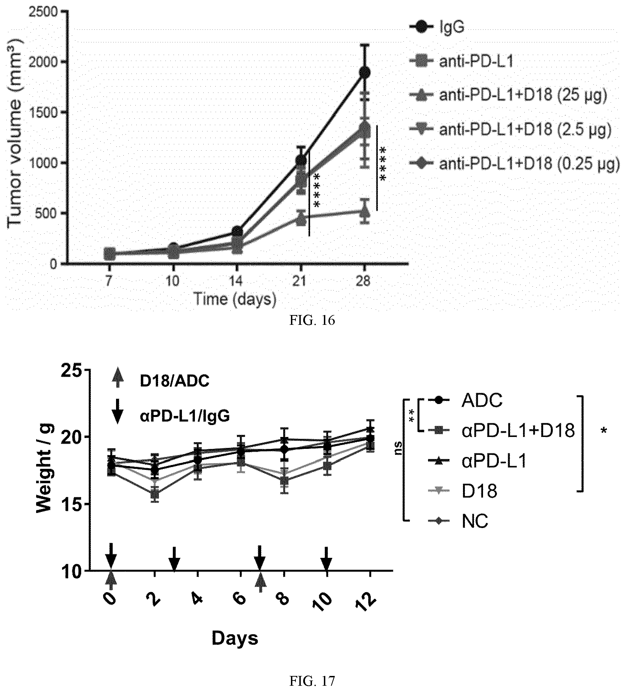

FIG. 16 shows the experimental results of a gradient dose of D18 in combination with anti-PD-L1 antibodies having different tumor inhibitory capacity. D18 was dosed once a week, and anti-PD-L1 antibody or IgG was dosed twice a week. When the antibody-based drugs were dosed, the administration was performed at 150 µg per mouse in each group. The combined administration groups were divided into: an anti-PD-L1 + D18 (25 µg) group, 25 µg of D18 dosed for each time per mouse; an anti-PD-L1 + D18 (2.5 µg) group, 2.5 µg of D18 dosed for each time per mouse; and an anti-PD-L1 + D18 (0.25 µg) group, 0.25 µg of D18 dosed for each time per mouse. Student's t test was used to compare significant differences between the groups, and *p* < 0.0001 is represented by ****.
FIG. 17 shows the effect of ADC HE-S2 on the body weight of mice. Mice were treated with ADC HE-S2, anti-PD-L1 antibody + D18 in combination, anti-PD-L 1 antibody alone, D18 alone, and isotype IgG, and their body weight variations were recorded. The time of administration for each drug is indicated by the arrow. Student's t test was used to compare significance differences between groups, wherein *p* < 0.05 is represented by *,. *p* < 0.01 is represented by **, and no significance difference is represented by ns.
FIG. 18 shows the mouse anti-tumor experimental results for ADC10.

### DETAILED DESCRIPTION

Unless otherwise defined, all scientific and technical terms used in the present invention have the same meaning as commonly understood by those skilled in the art to which the present invention relates.

In the context of the present invention, the following terms have the meanings detailed below.

In the present invention, the term "alkyl" refers to a hydrocarbon chain radical that is linear or branched and that does not contain unsaturated bonds, and that is linked to the rest of the molecule via a single bond. Typical alkyl groups contain 1 to 12 (e.g., 1, 2, 3, 4, 5, 6, 7, 8, 9, 10, 11, or 12) carbon atoms, such as methyl, ethyl, *n*-propyl, isopropyl, *n*-butyl, isobutyl, *tert*-butyl, *n*-pentyl, isopentyl, neopentyl, *tert*-pentyl, *n*-hexyl, isohexyl, etc. If alkyl is substituted with cycloalkyl, it is correspondingly "cycloalkylalkyl" radical, such as cyclopropylmethyl, cyclopropylethyl, cyclobutylmethyl, cyclopentylmethyl, or cyclohexylmethyl. If alkyl is substituted with aryl, it is correspondingly "aralkyl" radical, such as benzyl, benzhydryl or phenethyl. If alkyl is substituted with heterocyclyl, it is correspondingly "heterocyclylalkyl" radical.

"Alkenyl" refers to a hydrocarbon chain radical that is linear or branched and contains at least two carbon atoms and at least one unsaturated bond, and that is linked to the rest of the molecule via a single bond. Typical alkenyl groups contain 1 to 12 (e.g., 1, 2, 3, 4, 5, 6, 7, 8, 9, 10, 11, or 12) carbon atoms, such as ethenyl, 1-methyl-ethenyl, 1-propenyl, 2-propenyl, or butenyl.

"Alkoxy" refers to a substituent formed from a hydroxy group by substituting the hydrogen atom with alkyl, and typical alkoxy groups contain 1 to 12 (e.g., 1, 2, 3, 4, 5, 6, 7, 8, 9, 10, 11, or 12) carbon atoms, such as methoxy, ethoxy, propoxy, or butoxy.

"Aryl" refers to a monocyclic or polycyclic radical, including polycyclic radicals containing monoaryl and/or fused aryl groups. Typical aryl groups contain 1 to 3 monocyclic or fused rings and 6 to about 18 carbon ring atoms, preferably 6 to about 14 carbon ring atoms, such as phenyl, naphthyl, biphenyl, indenyl, phenanthryl or anthracyl radicals.

"Heterocyclyl" includes heteroaromatic and heteroalicyclic groups containing 1 to 3 monocyclic and/or fused rings and 3 to about 18 ring atoms. Preferred heteroaromatic groups and heteroalicyclic groups contain 5 to about 10 ring atoms. Suitable heteroaryl groups for the compound of the present invention contain 1, 2 or 3 heteroatoms selected from N, O and S atoms and include, for example, coumarin, including 8-coumarin, quinolyl, including 8-quinolyl, isoquinolyl, pyridinyl, pyrazinyl, pyrazolyl, pyrimidinyl, furyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuranyl, benzofurazanyl, benzothiophenyl, benzothiazolyl, benzoxazolyl, quinazolinyl, quinoxalinyl, naphthyridinyl, and furopyridinyl. Suitable heteroalicyclic groups for the compound of the present invention contain 1, 2 or 3 heteroatoms selected from N, O and S atoms and include, for example, pyrrolidinyl, tetrahydrofuryl, dihydrofuran, tetrahydrothienyl, tetrahydrothiopyranyl, piperidinyl, morpholinyl, thiomorpholinyl, oxathianyl, piperazinyl, azetidinyl, oxetanyl, thietanyl, homopiperidinyl, oxiranyl, thiiranyl, azepinyl, oxazepanyl, diazepinyl, triazepinyl, 1,2,3,6-tetrahydropyridinyl, 2-pyrrolinyl, 3-pyrrolinyl, indolinyl, 2*H*-pyranyl, 4*H*-pyranyl, dioxanyl, 1,3-dioxolanyl, pyrazolinyl, dithianyl, dithiolyl, dihydropyranyl, dihydrothienyl, pyrazolidinyl, imidazolinyl, imidazolidinyl, 3-azabicyclo[3.1.0]hexyl, 3-azabicyclo[4.1.0]heptyl, 3*H-*indolyl, and quinolizinyl.

"Halogen" or "halo" refers to bromo, chloro, iodo or fluoro.

Unless otherwise specified, the compounds of the present invention also include isotope-labeled forms, i.e., compounds that differ only in the presence of one or more isotope atoms. For example, compounds of existing structures with at least one hydrogen atom replaced by only deuterium or tritium, at least one carbon replaced by a ¹³C- or ¹⁴C-enriched carbon, or at least one nitrogen replaced by a ¹⁵N-enriched nitrogen are all included within the scope of the present invention. The term "PD-L1" includes isoforms, species homologs of mammalian, e.g., species homologs of human PD-L1, and analogs comprising at least one common epitope with PD-L1. The amino acid sequence of PD-L1, such as human PD-L1, is known in the art. In the present invention, the "anti-PD-L1 antibody" refers to an antibody that may specifically bind to PD-L1.

The term "antibody" refers to an immunoglobulin molecule comprising four polypeptide chains that are two heavy (H) chains and two light (L) chains interconnected via disulfide bonds (i.e., a "whole antibody molecule"), and a multimer thereof (e.g., IgM) or an antigen-binding fragment thereof. Each heavy chain comprises a heavy chain variable region ("HCVR" or "VH") and a heavy chain constant region (comprising domains CH1, CH2, and CH3). Each light chain comprises a light chain variable region ("LCVR" or "VL") and a light chain constant region (CL). The VH and VL regions may be further subdivided into hypervariable regions, which are called complementarity determining regions (CDRs) and are interspersed with regions that are more conserved, which are called framework regions (FRs). Each VH and VL is composed of three CDRs and four FRs, arranged from amino-terminus to carboxyl-terminus in the following order: FR1, CDR1, FR2, CDR2, FR3, CDR3, FR4.

As used herein, the terms "antigen-binding portion", "antibody fragment", and the like of an antibody include any natural, synthesized or genetically-engineered polypeptide or glycoprotein that can be obtained enzymatically and specifically binds to an antigen to form a complex. As used herein, the term "antigen-binding portion" or "antibody fragment" of a PD-L1 antibody refers to one or more fragments of an antibody that retain the ability to specifically bind to PD-L1. Antibody fragments may include Fab fragments, F(ab')₂ fragments, Fv fragments, dAb fragments, CDR-containing fragments, or isolated CDRs. Non-limiting examples of antigen-binding fragments include: (i) Fab fragments; (ii) F(ab')₂ fragments; (iii) Fd fragments; (iv) Fv fragments; (v) single-chain Fv (scFv) molecules; (vi) dAb fragments; (vii) minimal recognition units (e.g., isolated complementarity determining regions (CDRs)) consisting of amino acid residues that mimic the hypervariable region of an antibody. Other engineered molecules, such as bifunctional antibodies, trifunctional antibodies, tetrafunctional antibodies, and minibodies, etc., are also encompassed within the expression "antigen-binding fragment" as used herein. An antigen-binding fragment of an antibody may typically comprise at least one variable domain.

The term "therapeutically effective amount" refers to a dosage that achieves treatment, prevention, alleviation and/or amelioration of the diseases or conditions described herein in a subject.

The terms "patient", "subject", "individual", and the like are used interchangeably herein, and refer to any animal or cell thereof, whether in vitro or in situ, which in some non-limiting embodiments is a mammal, e.g., a human, monkey, dog, rabbit, mouse, etc., subject to the method described herein.

The term "treating" includes eradicating, removing, reversing, alleviating, altering or controlling a disease or condition after its onset.

The term "preventing" refers to the ability to avoid, minimize, or make difficult the onset or progression of a disease or condition prior to its onset through treatment.

The term "disease" refers to a physical condition of the subject that is associated with the disease of the present invention. The disclosures of the various publications, patents, and published patent specifications cited herein are hereby incorporated by reference in their entireties.

The technical schemes of the present invention will be clearly and completely described below with reference to the examples of the present invention, and it is obvious that the described examples are only a part of the examples of the present invention but not all of them. Based on the examples of the present invention, all other examples obtained by those of ordinary skills in the art without creative work shall fall within the protection scope of the present invention.

### Example 1: Preparation of Small Molecule Loading

### 1. Preparation of (R)-2-((5-5-nitropyridin-2-yl)dithiocyclohexyl)propan-1-ol (Compound 24)

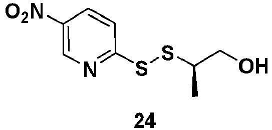

SOCl₂ (316 µL, 3.94 mmol) was added dropwise to a solution of 5-nitropyridine-2-thiol (560 mg, 3.59 mmol) in anhydrous DCM (8 mL) at 0°C under argon atmosphere. The resulting mixture was stirred at room temperature for 2 h, and after completion of the reaction, the reaction mixture was concentrated under reduced pressure to provide Intermediate 24 as a yellow powder. To a solution of Intermediate 4 in dry DCM (10 mL) was added dropwise a solution of methyl (*R*)-2-mercaptopropan-1-ol (prepared according to the procedure described in WO2013055987, 360 mg, 3.88 mmol) in dry DCM (5 mL). The reaction mixture was stirred at room temperature under argon atmosphere overnight. After completion of the reaction, the reaction mixture was filtered, and the filtrate was concentrated under reduced pressure to give a yellow solid. The residue was suspended in water, alkalized with a sodium bicarbonate solution, and then extracted with DCM (3 × 100 mL). The organic phase was dried over anhydrous Na₂SO₄, filtered, and concentrated under reduced pressure. The residue was purified by flash silica gel chromatography (PE:EA = 10:3) to give Compound 24 (yield: 76%). 1H NMR (400 MHz, chloroform-d) δ9.23 (dd, J = 2.7, 0.8 Hz, 1H), 8.33 (dd, J = &9, 2.6 Hz, 1H), 7.71 (dd, J = 8.8, 0.7 Hz, 1H), 4.48 (t, J = 6.7 Hz, 1H), 3.62 (ddd, J = 11.8, 7.1, 4.3 Hz, 1H), 3.38 (ddd, J = 11.9, 7.3, 4.4 Hz, 1H), 3.11 (pd, J = 7.0, 4.4 Hz, 1H), 1.28 (d, J = 6.9 Hz, 3H). 13C NMR (101 MHz, chloroform-d) δ167.95, 145.12, 142.45, 131.48, 120.85, 64.00, 49.68, 16.73. C₈H₁₁N₂O₃S₂ MS (ESI) M/z [M+H]⁺, calculated 247.0, found 247.2.

### 2. Preparation of (R)-2-((5-nitropyridin-2-yl)dithiocycloheayl)propyl(4-(hydroxymethyl)phenyl)carbamate (Compound 26)

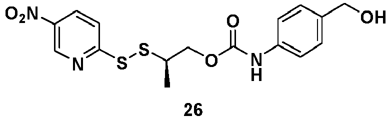

Compound **24** (246 mg, 1 mmol) and pyridine (79 mg, 1 mmol) were dissolved in dry DCM (5 mL) and stirred at 0°C. To the mixture at 0°C was added dropwise a solution of triphosgene (145 mg, 0.5 mmol) in anhydrous DCM (5 mL) under argon atmosphere, and the resulting mixture was stirred for 1 h. After completion of the reaction, the resulting reaction solution was concentrated by rotary evaporation under reduced pressure to give (*R*)-2-((5-nitropyridin-2-yl)disulfanyl)propyl carbon chloride (Compound **25**), which could be directly used without further purification. Subsequently, Compound **25** (308 mg, 1.0 mmol) was dissolved in dry DCM (5 mL), and a mixed solution of (4-aminophenyl) methanol (148 mg, 1.2 mmol) and pyridine (79 mg, 1.0 mmol) in DCM (5mL) was added slowly & dropwise. The reaction mixture was stirred at room temperature for 2 h under argon atmosphere. After completion of the reaction, the resulting solution was transferred to a saturated ammonium chloride solution and extracted with DCM (3 × 50 mL). The organic layer was washed with water and brine, then dried over anhydrous Na₂SO₄, filtered, and concentrated by rotary evaporation under reduced pressure to give a crude product. The crude product was purified by flash silica gel chromatography (PE:EA = 2:1) to give Compound **26** as a white solid (yield: 70%). 1H NMR (400MHz, Chloroform-*d*) δ9.23 (d, J = 2.6Hz, 1H), 8.33 (dd, J = 8.8, 2.6Hz, 1H), 7.91 (d, J = 8.8Hz, 1H), 7.34 (m, 4H), 6.82 (s, 1H), 4.67 (s, 2H), 4.31-4.23 (m, 2H), 3.37 (h, J = 6.8Hz, 1H), 1.43 (d, J = 7.0Hz, 3H).13C NMR (101 MHz, Chloroform-*d*) δ168.63, 152.87, 145.03, 142.06, 136.88, 136.37, 131.61, 128.04, 119.47, 118.73, 67.26, 64.86, 45.57, 17.10. Mass (ESI): C₁₆H₁₈N₃O₅S₂ m/z [M+H]⁺, calculated 396.1, found 396.2.

### 3. Preparation of (R)-2-((5-nitropyridin-2-yl)dithiocyclohexyl)propyl(4-(benzylchloro)phenyl)carbamate (Compound 19)

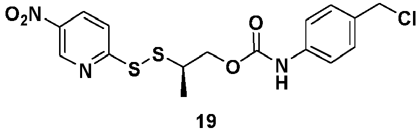

Thionyl chloride (22 gL, 0.304 mmol) was added dropwise to a solution of Compound **26** (100 mg, 0.253 mmol) in dry DCM (5 mL) at 0°C. The reaction mixture was taken out at 0°C, and stirred at room temperature for 30 min. After completion of the reaction, excess thionyl chloride was removed by rotary evaporation under reduced pressure to give a desired product, Compound **19,** as a white solid (yield: 96%). 1H NMR (400 MHz, chloroform-d) δ9.24 (d, J = 2.6 Hz, 1H), 8.35 (dd, J = 8.9, 2.7 Hz, 1H), 7.89 (d, J = 8.9 Hz, 1H), 7.35 (m, 4H), 6.75 (s, 1H), 4.56 (s, 2H), 4.27 (qd, J = 11.5, 6.1 Hz, 2H), 3.35 (h, J = 6.7 Hz, 1H), 1.41 (d, J = 7.0 Hz, 3H). 13C NMR (101 MHz, chloroform-d) δ168.59, 152.71, 145.06, 142.11, 137.56, 132.88, 131.60, 129.59, 119.47, 118.71, 67.34, 45.90, 45.51, 17.07. C₁₆Hl₇CIN₃O₄S₂ MS (ESI): m/z [M+H]⁺, calculated 414.0, found 414.2.

### 4. Preparation of Compound 20

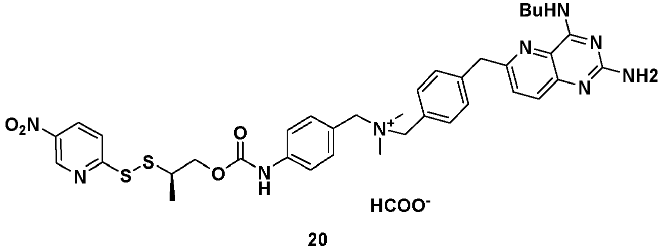

To a solution of compound *N*⁴-butyl-6-(4-((dimethylamino)methyl)benzyl)pyridine[3,2-*d*]pyrimidine-2,4-diamine (40 mg, 0.11 mmol) in DMF (0.5 mL) were added Compound **19** (50 mg, 0.12 mmol), TBAB (8 mg, 0.022 mmol) and DIEA (18 µL, 0.11 mmol). The reaction mixture was stirred at room temperature, and monitored by LC-MS. After completion of the reaction, DMF was removed by rotary evaporation under reduced pressure. The residue was redissolved in 10% methanol solution, and stirred at room temperature for 2 h. The resulting solution was concentrated by rotary evaporation, and purified by phase preparative high performance liquid chromatography (RPHPLC) to give Compound **20** as a formate (yield: 68.0%).

1H NMR (400 MHz, methanol-d4) δ9.09 (d, J = 2.6 Hz, 1H), 8.32 (s, 1H), 8.31 (dd, J = 8.7, 2.6 Hz, 1H), 8.09 (d, J = 8.8 Hz, 1H), 7.79 (d, J = 8.6 Hz, 1H), 7.66 (d, J = 8.5 Hz, 1H), 7.62-7.44 (m, 8H), 4.69 (s, 2H), 4.57 (s, 2H), 4.35 (s, 2H), 4.26-4.16 (m, 2H), 3.70 (t, J = 7.3 Hz, 2H), 3.44 (pd, J = 6.9, 4.8 Hz, 1H), 2.97 (s, 6H)), 1.73 (p, J = 7.5 Hz, 2H), 1.50-1.40 (m, 5H), 1.00 (t, J = 7.5 Hz, 3H),¹³C NMR (101 MHz, methanol-d4) δ168.46, 165.88, 160.00, 157.48, 155.18, 153.27, 144.32, 142.10, 141.94, 141.18, 133.68, 133.63, 133.31, 131.74, 129.62, 129.31, 126.30, 125.85, 125.76, 121.22, 120.11, 118.01, 68.23, 67.23, 67.2043.08, 40.56, 30.66, 19.77, 15.98, 12.78. C₃₇H₄₄N₉O₄S₂ MS (ESI): m/z [M]⁺, calculated 742.3, found 742.3, HRMS (ESI) M⁺, calculated 742.2958, found 742.2968.

### 5. Preparation of MC-Ual-Cit-PAB-Cl (Compound 29)

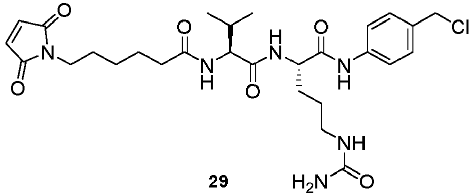

To a solution of MC-Ual-Cit-PAB-OH (57 mg, 0.1 mmol) in anhydrous DCM (5 mL) was added dropwise thionyl chloride (8.7 gL, 0.304 mmol) at 0°C. The reaction mixture was taken out at 0°C, and stirred at room temperature for 12 h. After completion of the reaction, excess thionyl chloride was removed by rotary evaporation under reduced pressure to give a desired product **29** as a brown solid, which was directly used without purification.

### 6. Preparation of Compound 30

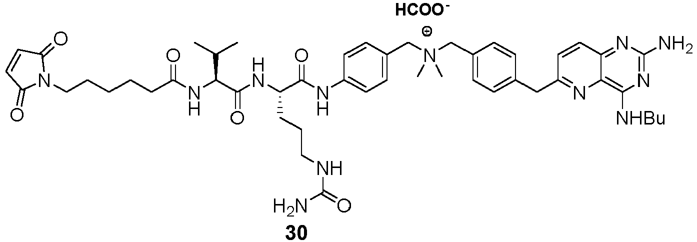

To a solution of compound N⁴-butyl-6-(4-((dimethylamino)methyl)benzyl)pyridine[3,2-d]pyrimidine-2,4-diamine (40 mg, 0.11 mmol) in DMF (1 mL) were added Compound **29** (71 mg, 0.12 mmol), TBAB (8 mg, 0.022 mmol), and DIEA (18 µL, 0.11 mmol). The reaction mixture was stirred at room temperature, and monitored by LC-MS. After completion of the reaction, DMF was removed by rotary evaporation under reduced pressure. The residue was redissolved in 10% methanol solution, and stirred at room temperature for 2 h. The resulting solution was concentrated by rotary evaporation, and purified by phase preparative high performance liquid chromatography (RPHPLC) to give compound **30** as a formate (yield: 55.0%). ¹H NMR (400 MHz, Chloroform) δ 10.34 (bs, 1H), 9.40 (bs, 1H), 8.13 (d, *J* = 8.6 Hz, 1H), 7.82 - 7.68 (m,4H), 7.61 - 7.39 (m, 4H), 7.25 - 6.96 (m, 3H), 4.51 - 4.14 (m, 5H), 4.25 (s, 2H), 4.00 (m, 2H), 3.46 (t, *J* = 6.8 Hz, 2H), 3.27 (s, 6H), 2.95 (m, 2H), 2.73 (m, 1H), 2.49 (m, 1H), 2.10 - 1.85 (m, 6H), 1.74 - 1.53 (m, 4H), 1.60 - 1.38 (m, 2H), 1.41 - 1.19 (m, 6H), 1.14 - 0.80 (m, 9H). MS (ESI) [M]⁺, calculated 920.15, found 920.30.

### 7. Preparation of (S)-N¹-(4-(chloromethyl)phenyl)-2-((R)-2-((S)-2-(6-(2,5-dioxo-2,5-dihydro-1H-pyrrol-1-yl)hexanamido)propionamido)propionamido)succinamide (Compound 31)

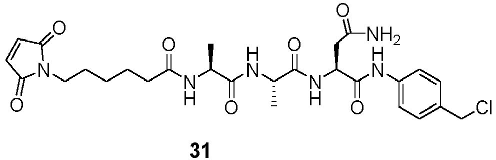

To a solution of (*S*)-2-((*R*)-2-((*S*)-2-(6-(2,5-dioxo-2,5-dihydro-1*H*-pyrrol-1-yl)hexanamido)propionamido)propionamido)-N¹-(4-(hydroxymethyl)phenyl)succinamide (100 mg, 0.174 mmol) in anhydrous DCM (5 mL) was added dropwise thionyl chloride (15 gL, 0.210 mmol) at 0°C. The reaction mixture was taken out at 0°C, and stirred at room temperature for 30 min. After completion of the reaction, excess thionyl chloride was removed by rotary evaporation under reduced pressure to give a desired product as a brown solid. The product was directly used without purification.

### 8. Preparation of Compound 32

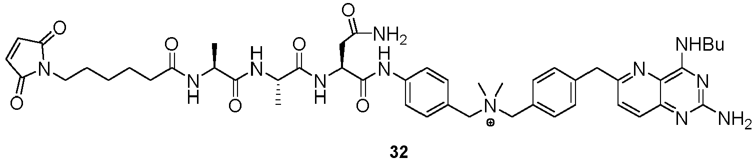

To a solution of compound N⁴-butyl-6-(4-((dimethylamino)methyl)benzyl)pyridine[3,2-d]pyrimidine-2,4-diamine (40 mg, 0.11 mmol) in DMF (1 mL) were added Compound **31** (71 mg, 0.12 mmol), TBAB (8 mg, 0.022 mmol) and DIEA (18 µL, 0.11 mmol). The reaction mixture was stirred at room temperature, and monitored by LC-MS. After completion of the reaction, DMF was removed by rotary evaporation under reduced pressure. The residue was redissolved in 10% methanol solution, and stirred at room temperature for 2 h. The resulting solution was concentrated by rotary evaporation, and purified by phase preparative high performance liquid chromatography (RPHPLC) to give Compound **32** as a formate (yield: 48.0%). LC-MS (ESI) [M]⁺, calculated 920.10, found 920.25.

### 9. Preparation of Compound 33

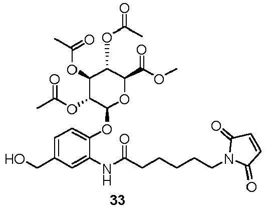

(2*S*,3*R*,4*S*,5*S*,6*S*)-2-(2-amino-4-(hydroxymethyl)phenoxy)-6-(methoxycarbonyl)tetrahydro-2*H*-pyran-3,4,5-triol triacetate, commercially available, (455 mg, 1 mmol) was dissolved in DMF (5 mL). N-succinimidyl 6-maleimidohexanoate (340 mg, 1.1 mmol), 1-hydroxybenzotriazole (135 mg,1 mmol), and *N*,*N*-diisopropylethylamine (210 µL, 1.2 mmol) were added in sequence, and the resulting mixture was stirred at room temperature for 2 h. The reaction solution was extracted with EA/H₂O, and the organic phase was concentrated to give a crude product. The crude product was purified by silica gel column chromatography (DCM:MeOH = 10:1) to give the target product (278 mg). LC-MS (ESI) [M]⁺: 649.3.

### 10. Preparation of Compound 34

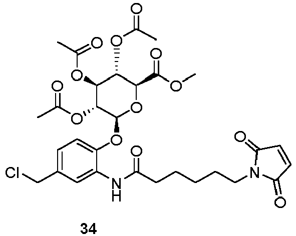

Thionyl chloride (8 µL, 0.12 mmol) was added dropwise to a solution of (2*S*,3*R*,4*S*,5*S*,6*S*)-2-(2-(6-(2,5-dioxo-2,5-dihydro-1*H*-pyrrol-1-yl)hexanamide)-4-(hydroxymethyl)phenoxy)-6-(methoxycarbonyl)tetrahydro-2*H*-pyran-3,4,5-triacyltriacetate (compound 33) (65 mg, 0.1 mmol) in anhydrous DCM (2 mL) at 0°C. The reaction mixture was stirred at 0°C for 30 min. After completion of the reaction, the organic solvent was removed by rotary evaporation under reduced pressure to give the target product. The product was directly used without purification.

### 11. Preparation of Compound 35

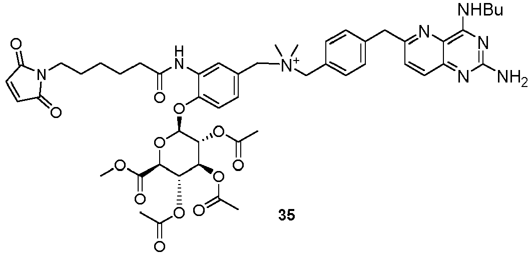

To a solution of compound (2*S*,3*R*,4*S*,5*S*,6*S*)-2-(4-(chloromethyl)-2-(6-(2,5-dioxo-2,5-dihydro-1*H*-pyrrol-1-yl)hexanamide)phenoxy)-6-(methoxycarbonyl)tetrahydro-2*H*-pyran-3,4,5-triacyltriacetate (67 mg, 0.1 mmol) (compound 34) in DMF (1 mL) were added N⁴-butyl-6-(4-((dimethylamino)methyl)benzyl)pyridine[3,2-d]pyrimidine-2,4-diamine (40 mg, 0.11 mmol), TBAB (8 mg, 0.022 mmol), and DIEA (18 µL, 0.11 mmol). The reaction mixture was stirred at room temperature, and monitored by LC-MS. After completion of the reaction, the reaction solution was filtered, and then purified by phase preparative high performance liquid chromatography (RPHPLC) to give the target product (30 mg, yield: 28%). LC-MS (ESI) [M]⁺: 995.4.

### 12. Preparation of Compound 36

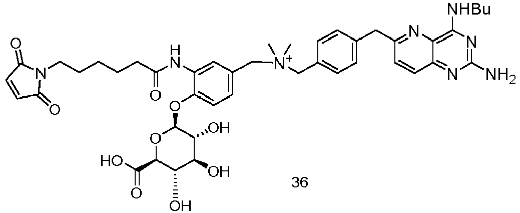

Compound 35 (30 mg, 0.03 mmol) was dissolved in a mixed solvent (THF:H₂O = 2:1). Lithium hydroxide monohydrate (15 mg, 0.3 mmol) was added, and after the resulting mixture was stirred at room temperature for 30 min, acetic acid was added to adjust pH to 7. The reaction solution was concentrated under reduced pressure to remove the organic solvent, methanol was added for dissolving, and the residue was subjected to reversed-phase chromatography to give the target product (12 mg, yield: 46%). LC-MS (ESI) [M]⁺: 855.4.

### 13. Preparation of Compound 37

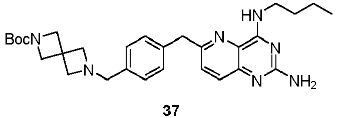

N⁴-butyl-6-(4-(chloromethyl)benzyl)pyridine[3,2-d]pyrimidine-2,4-diamine (356 mg, 1 mmol, the synthesis method was referred to the preparation method for Compound 10 in the example of Chinese patent CN 108069963 B**)** was dissolved inDMF. 2-boc-2,6-diazaspiro[3.3]heptane hemioxalate (980 mg, 0.2 mmol) and potassium carbonate (420 mg, 0.3 mmol) were added. The resulting mixture was stirred overnight at 35°C. After completion of the reaction, the reaction solution was extracted with ethyl acetate and saturated brine, and washed with water for 3 times. The organic phase was dried over anhydrous sodium sulfate, and concentrated to dryness by rotary evaporation to give a crude product. The crude product was purified by silica gel column chromatography (DCM-MeOH system) to give the target product (340 mg, yield: 65%). LC-MS (ESI) [M+H]⁺: 518.3.

### 14. Preparation of Compound 38

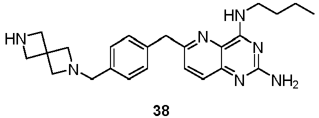

Tert-butyl 6-(4-((2-amino-4-(butylamino)pyridine[3,2-d]pyrimidin-6-yl)methyl)benzyl)-2,6-diazaspiro[3.3]heptane-2-carboxylate (compound 37) (260 mg, 0.5 mmol) was dissolved in a mixed solvent (DCM:TFA = 10:1). The reaction solution was stirred at 0°C for 1 h, and concentrated under reduced pressure to remove the organic solvent to give a crude product. Diethyl ether was added to the crude product. The resulting product was subjected to ultrasonic treatment, and filtered to give the target product. LC-MS (ESI) [M+H]⁺: 418.3.

### 15. Preparation of Compound 39

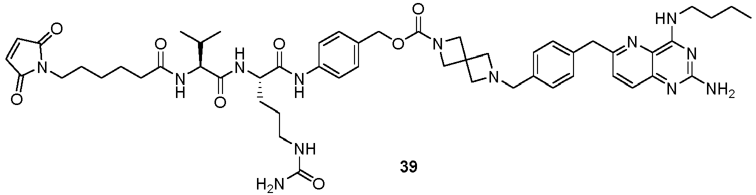

4-((*S*)-2-((*S*)-2-(6-(2,5-dioxo-2,5-dihydro-1*H*-pyrrol-1-yl)hexanamide)-3-methylbutyramide)-5-uridylpentanamide)benzyl(4-nitrobenzene)carbonate (81.0 mg, 0.11 mmol) was dissolved in DMF (0.5 mL). 6-(4-((2,6-diazaspiro[3.3]heptan-2-yl)methyl)benzyl)-N⁴-butylpyridine[3,2-d]pyrimidine-2,4-diamine (compound 38) (42.0 mg, 0.10 mmol) and *N*,*N*-diisopropylethylamine (21 µL, 0.12mmol) were added. The resulting mixture was stirred at room temperature, and the reaction was monitored by LCMS. After completion of the reaction, the reaction solution was subjected to RP-HPLC to give the target product (20 mg, yield: 20%). LC-MS (ESI) [M+H]⁺: 1016.4. ¹H NMR (400 MHz, DMSO-*d6*) δ 7.78 (d, J = 8.5 Hz, 1H), 7.60-7.55 (m, 4H), 7.29 - 7.17 (m, 5H), 4.00 (s, 2H), 3.66 (s, 2H), 3.46 (t, J = 13.2 Hz, 2H), 3.07 (s, 2H), 2.95 (t, J = 12.4 Hz, 1H), 2.76-2.67 (m, 1H), 2.08-2.01 (m, 2H), 1.75-1.59 (m, 6H), 1.50-1.43(m, 3H), 1.38-1.27 (m, 6H), 1.01-0.87 (m, 9H).

### 16. Preparation of Compound 40

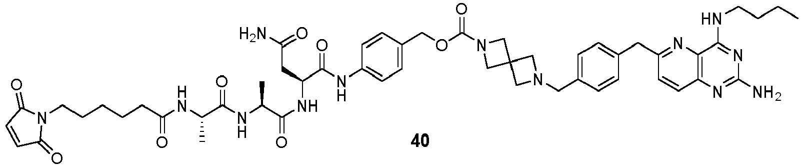

4-((*S*)-4-amino-2-((*S*)-2-(6-(2,5-dioxo-2,5-dihydro-1*H*-pyrrol-1-yl)hexanamido)propionamido)-4-oxobutanamido)benzyl(4-nitrobenzene)carbonate, commercially available, (81.0 mg, 0.11 mmol) was dissolved in DMF (0.5 mL). 6-(4-((2,6-diazaspiro[3.3]heptan-2-yl)methyl)benzyl)-N⁴-butylpyridine[3,2-d]pyrimidine-2,4-diamine (compound 38) (42.0 mg, 0.10 mmol) and *N*,*N*-diisopropylethylamine (21 µL, 0.12mmol) were added. The resulting mixture was stirred at room temperature, and the reaction was monitored by LCMS. After completion of the reaction, the reaction solution was subjected to RP-HPLC to give the target product (15 mg, yield: 16%). LC-MS (ESI) [M+H]⁺; 1016.4.

### 17. Preparation of Compound 41

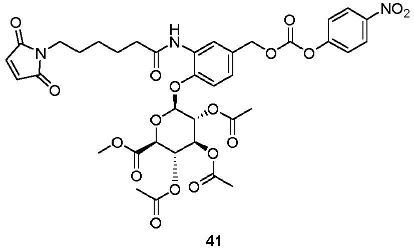

(2*S*,3*R*,4*S*,5*S*,6*S*)-2-(2-(6-(2,5-dioxo-2,5-dihydro-1*H*-pyrrol-1-yl)hexanamide)-4-(hydroxymethyl)phenoxy)-6-(methoxycarbonyl)tetrahydro-2*H*-pyran-3,4,5-triacyltriacetate (260 mg, 0.4 mmol) was dissolved in DMF (2 mL). Bis(p-nitrophenyl)carbonate (182 mg, 0.6 mmol) and *N*,*N*-diisopropylethylamine (105 µL, 0.6 mmol) were added in sequence, and the resulting mixture was reacted for 6 h. The reaction solution was extracted with EA/H₂O for three times. The organic phases were combined, dried over anhydrous sodium sulfate, and concentrated under reduced pressure to give a crude product. The crude product was purified by silica gel column chromatography (DCM:MeOH = 15:1) to give the target product (250 mg, yield: 78%). LC-MS (ESI) [M+H]⁺: 814.4.

### 18. Preparation of Compound 42

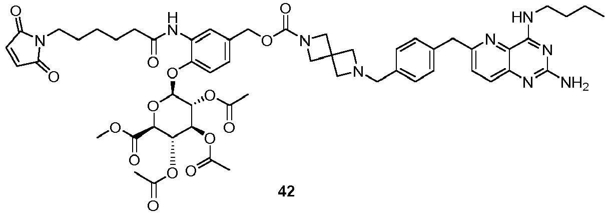

(2*S*,3*R*,4*S*,5*S*,6*S*)-2-(2-(6-(2,5-dioxo-2,5-dihydro-1*H*-pyrrol-1-yl)hexanamide)-4-((((4-nitrophenoxy)carbonyl)methyl)phenoxy)-6-(methoxycarbonyl)tetrahydro-2*H*-pyran-3,4,5-triyltriacetate (Compound 41) (82 mg, 0.1 mmol) was dissolved in DMF (1 mL). 6-(4-((2,6-diazaspiro[3.3]heptan-2 yl)methyl)benzyl)-N⁴-butylpyridine[3,2-d]pyrimidine-2,4-diamine (compound 38) (42.0 mg, 0.10 mmol) and *N*,*N*-diisopropylethylamine (21 µL, 0.12 mmol) were added. The reaction solution was stirred at room temperature, and the reaction was monitored by LCMS. After completion of the reaction, the reaction solution was subjected to RP-HPLC to give the target product (30 mg, yield: 27%). LC-MS (ESI) [M+H]⁺: 1092.4.

### 19. Preparation of Compound 43

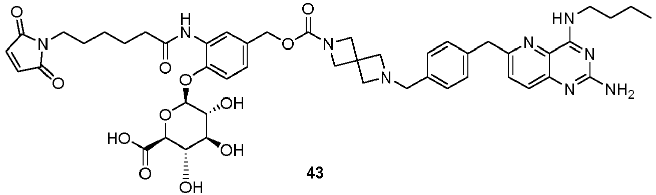

Compound 42 (30 mg, 0.027 mmol) was dissolved in a mixed solvent (THF:H₂O = 2:1). Lithium hydroxide monohydrate (15 mg, 0.27 mmol) was added, and after the resulting mixture was stirred at room temperature for 30 min, acetic acid was added to adjust pH to 7. The reaction solution was concentrated under reduced pressure to remove the organic solvent. Methanol was added for dissolving, and the residue was subjected to reversed-phase chromatography to give the target product (15mg, yield: 51%). LC-MS (ESI) [M+H]⁺: 952.3. ¹H NMR (400 MHz, CD₃OD) 8.06 (bs, 1H), 7.82(d, 1H), 7.31 - 7.18 (m, 5H), 7.15- 7.03 (m, 2H), 3.98 (td, ***J* =** 13.4, 4.8 Hz, 2H), 3.63 (s, 2H), 3.46 (t, *J* = 13.2 Hz, 2H), 3.05 (s, 2H), 2.37 (m, 2H), 1.77-1.25 (m, 12H), 0.89 (t, J = 7.4 Hz, 3H).

### 20. Preparation of Compound 44

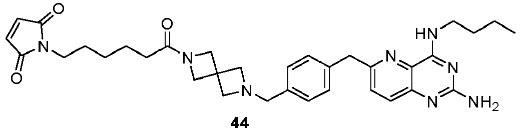

Compound 38 (41.8 mg, 1.0 mmol) was dissolved in DMF (2 mL). *N*-succinimidyl 6-maleimidohexanoate (34 mg, 0.11 mmol) was added, and the resulting mixture was reacted at room temperature for 3 h. The reaction solution was extracted with ethyl acetate/saturated brine. The organic phase was washed with water for 3 times, dried over anhydrous sodium sulfate, and concentrated under reduced pressure to remove the organic phase to give a crude product. The crude product was subjected to silica gel column chromatography (DCM:MeOH = 5:1) to give the target product (28 mg, yield: 48%). LC-MS (ESI) [M+H]⁺: 611.4. ¹H NMR (400 MHz, Chloroform) δ 7.67 (d, *J* = 8.4Hz, 1H),7.62 (br,2H), 7.31 - 7.16 (m, 5H), 4.21(s,2H), 3.89 (t*, J* = 10.4 Hz, 2H), 3.62 (s, 2H), 3.45 (t*, J* = 13.4 Hz, 2H), 3.01 (s, 2H), 2.23 (td, *J* = 13.2, 4.8 Hz, 2H), 1.63 - 1.27 (m, 12H), 0.89 (t, J = 7.4 Hz, 3H).

### 21. Preparation of Compound 45

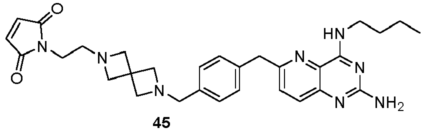

Compound 38 (21 mg, 0.5 mmol) was dissolved in methanol (1 mL). 2-(2,5-dioxo-2,5-dihydro-1*H*-pyrrol-1-yl)acetaldehyde (28 mg, 2 mmol) was added. The resulting mixture was stirred at room temperature for 30 min. Sodium cyanoborohydride (8 mg, 13 mmol) was added in portions, and the reaction was continued for 1 h. The reaction solution was filtered, and subjected to reversed-phase chromatography to give the product (6 mg, yield: 23%). LC-MS (ESI) [M+H]⁺: 541.3. ¹H NMR (400MHz, CDCI₃): δ7.62 (br, 2H), 7.56 (d, J = 8.6 Hz, 1H), 7.45(d, J = 8.6 Hz, 1H),7.43-7.32 (m, 4H), 4.24 (s, 2H), 4.70 (s, 4H), 4.12(s, 2H), 3.53 (td, J = 13.2, 5.9 Hz, 2H), 3.46-3.33 (s, 4H), 3.33 (s, 4H), 2.50 (t, 3H), 1.70-1.65 (m, 2H), 1.53-1.44 (m, 2H), 1.00 (t, J = 7.3 Hz, 3H).

### 22. Preparation of Compound 46

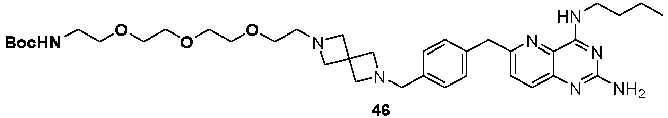

Compound 38 (210 mg, 0.5 mmol) was dissolved in acetonitrile (5 mL). *Tert*-butyl(2-(2-(2-iodoethoxy)ethoxy)ethoxy)ethyl)carbamate (305 mg, 0.75 mmol) and potassium carbonate (216 mg, 1.5 mmol) were added in sequence, and the reaction solution was heated to 65°C for 5 h. After completion of the reaction, the reaction solution was filtered, and the filter residue was washed with acetonitrile. The filtrate was collected, and concentrated under reduced pressure to give a crude product. The crude product was purified by silica gel column chromatography (DCM:MeOH = 4:1) to give the target product (218 mg, yield: 62%). LC-MS (ESI) [M+H]⁺: 693.5.

### 23. Preparation of Compound 47

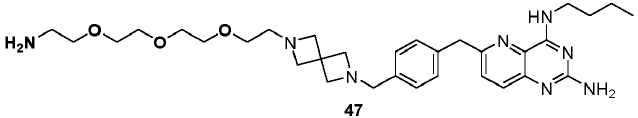

Compound 46 (218 mg) was dissolved in a mixed solvent (DCM:TFA = 20:1, 10 mL), and the reaction solution was left at 0°C for 1 h. After completion of the reaction, the reaction solution was concentrated under reduced pressure to remove the organic solvent to give the target product, which was directly used without purification.

### 24. Preparation of Compound 48

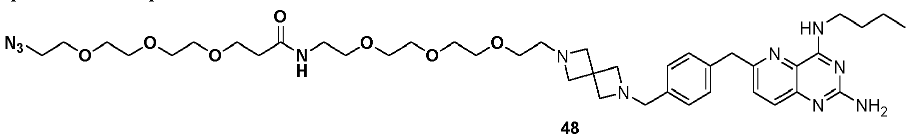

Compound 47 (184 mg, 0.3 mmol) was dissolved in DMF (1 mL). 2,5-dioxopyrrolidin-1-yl 3-(2-(2-azidoethoxy)ethoxy)ethoxy)propionate (106 mg, 0.3 mmol) and triethylamine (135 µL, 0.9 mmol) were added in sequence. The reaction solution was left at room temperature and reacted for 3 h. After completion of the reaction, dichloromethane and water were added for extraction. The organic phase was washed with a saturated ammonium chloride aqueous solution and water, dried over anhydrous sodium sulfate, and concentrated under reduced pressure to give a crude product. The resulting crude product was directly used without purification. LC-MS (ESI) [M+H]⁺: 822.3.

### 25. Preparation of Compound 49

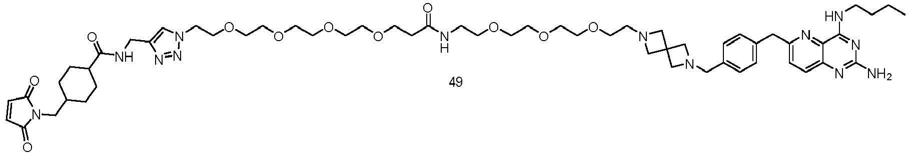

Compound 48 (100 mg, 0.12 mmol) was dissolved in a mixed solvent (DMSO:H₂O = 1:1, 1 mL). 4-((2,5-dioxo-2,5-dihydro-1*H*-pyrrol-1-yl)methyl)-*N*-(prop-2-yn-1-yl)cyclohexane-1-carboxamide (66 mg, 0.24 mmol) and cuprous bromide (51 mg, 0.36mmol) were added. The reaction solution was reacted at room temperature for 1 h. After completion of the reaction, the reaction solution was filtered, and the filtrate was subjected to reversed-phase chromatography to give the target compound (22 mg, yield: 16%). LC-MS (ESI) [M+H]⁺: 1140.4. ¹H NMR (400 MHz, DMSO) δ 9.43 (t, J = 5.8 Hz, 1H), 8.21 (t, J = 5.6 Hz, 1H), 7.91 (t, 1H), 7.82 (bs, 2H), 7.79 (d, J = 8.6 Hz, 1H), 7.68 (d, J = 8.6 Hz, 1H), 7.44 (d, J = 8.1 Hz, 2H), 7.36 (d, J = 8.1 Hz, 2H), 7.01 (s, 1H), 4.48 (t, J = 5.2 Hz, 2H), 4.26 (m, 4H), 3.82 - 3.76 (m, 4H), 3.67 (t, J = 6.4 Hz, 1H), 3.62 - 3.55 (m, 6H), 3.55 - 3.43 (m, 22H), 3.39 (t, J = 5.0 Hz, 4H), 3.33 (t, J = 6.3 Hz, 2H), 3.20 (m, 7H), 2.31 (t, J = 6.4 Hz, 4H), 2.06 (t, J = 11.3 Hz, 2H), 1.67 (m, 8H), 1.34 (m, 6H), 0.91 (t, J = 7.3Hz, 3H).

### 26. Preparation of Compound 50

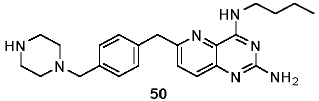

Compound 50 was prepared according to the synthesis method for compound 38.

### 27. Preparation of Compound 51

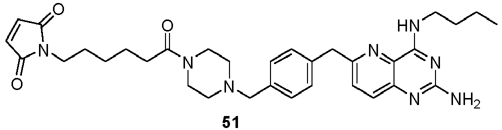

Compound 51 was prepared according to the synthesis method for compound 45, where the yield was 56%. LC-MS (ESI) [M+H]⁺: 599.4.

### 28. Preparation of Compound 52

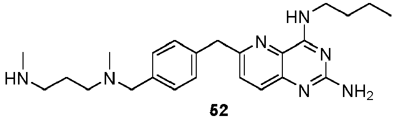

Compound 52 was prepared according to the synthesis method for compound 38.

### 29. Preparation of Compound 53

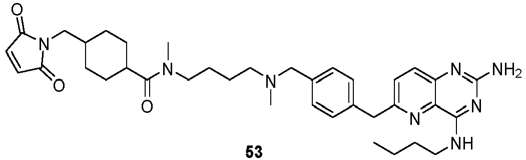

Compound 53 was prepared according to the synthesis method for compound 45, with replacing 6-maleimidohexanoic acid *N*-hydroxysuccinimide ester with 4-(*N*-maleimidomethyl)cyclohexanecarboxylic acid *N*-hydroxysuccinimide ester, , where the yield was 42%. LC-MS (ESI) [M+H]⁺: 655.3.

### 30. Preparation of Compound 54

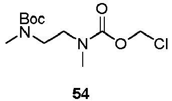

Tert-butyl methyl(2-(methylamino)ethyl)carbamate (1 g, 5.3 mmol) was dissolved in dichloromethane (10 mL). The reaction solution was left at 0°C. Chloromethyl chloroformate (0.95 mL) and pyridine (0.86 mL) were added in sequence. The reaction was continued at 0°C for 2 h. After completion of the reaction, dichloromethane and water were added for layering. The organic phase was washed with a saturated sodium chloride aqueous solution, and dried over anhydrous sodium sulfate. The organic layers were combined, and concentrated to give a crude product. The crude product was purified by silica gel column chromatography (PE:EA = 5:1) to give the target product as a colorless oil. LC-MS (ESI) [M+H]⁺: 281.3.

### 31. Preparation of Compound 55

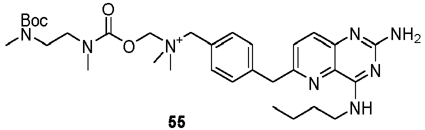

Compound **54** (1080 mg, 3.9 mmol) and TBAI (1420 mg, 3.9 mmol) were added to N⁴-butyl-6-(4-((dimethylamino)methyl)benzyl)pyridine[3,2-d]pyrimidine-2,4-diamine (400 mg, 1.1 mmol) in chloroform (10 mL), and the reaction solution was heated to 60°C for 6 h. After completion of the reaction, the reaction solution was concentrated, and then purified by column chromatography (DCM:MeOH = 4:1) to give the target compound (480 mg, yield: 72%). LC-MS (ESI) [M]⁺: 609.3.

### 32. Preparation of Compound 56

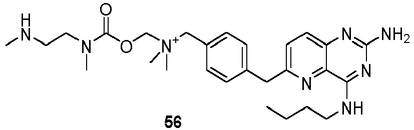

Compound 55 (480 mg) was dissolved in a mixed solvent (DCM:TFA = 10:1), and the reaction solution was left at 0°C for 1 h. After completion of the reaction, the organic solvent was removed by concentration under reduced pressure to give the target product trifluoroacetate. Diethyl ether was added to the yellow oil. The resulting mixture was subjected to ultrasonic treatment to precipitate a solid, and filtered. The filter residue was the target product trifluoroacetate. LC-MS (ESI) [M]⁺: 509.3.

### 33. Preparation of Compound 57

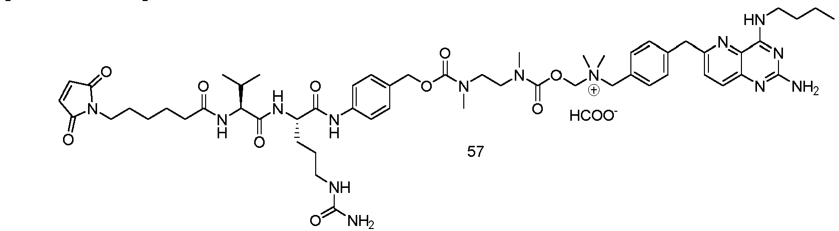

4-((*S*)-2-((*S*)-2-(6-(2,5-dioxo-2,5-dihydro-1*H*-pyrrol-1-yl)hexanamide)-3-methylbutyramide)-5-uridylpentanamide)benzyl(4-nitrobenzene)carbonate (MC-VC-PAB-NPC) (81.0 mg, 0.11 mmol) was dissolved in DMF (0.5 mL). Compound 56 (51 mg, 0.10 mmol), HOBT (13.5 mg, 0.10 mmol), and *N*,*N*-diisopropylethylamine (53 µL, 0.3 mmol) were added. The resulting mixture was stirred at room temperature, and the reaction was monitored by LCMS. After completion of the reaction, the reaction solution was subjected to RP-HPLC to give the target product (30 mg, yield: 24%). LC-MS (ESI) [M]⁺: 1107.4. ¹H NMR (400 MHz, DMSO) δ 10.16 (s, 1H), 8.38 (bs, 2H), 8.24 (bs, 1H), 7.90 (d, J = 7.7 Hz, 1H), 7.75 (t, 1H), 7.66 - 7.37 (m, 7H), 7.26 (m, 2H), 7.00 (s, 2H), 6.24 (s, 2H), 5.50 (s, 2H), 5.26 - 5.06 (m, 2H), 5.04 - 4.87 (m, 2H), 4.61 - 4.12 (m, 8H), 3.60 - 3.27 (m, 8H), 2.89 (m, 12H), 2.25 - 2.05 (m, 2H), 1.97 (m, 1H), 1.79 - 1.09 (m, 13H), 1.00 - 0.71 (m, 8H).

### 34. Preparation of Compound 58

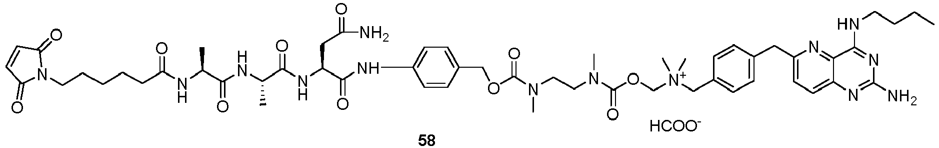

Compound 58 was prepared according to the synthesis method for compound 57, with replacing MC-VC-PAB-NPC with MC-AAN-PAB-NPC. LC-MS (ESI) [M]⁺: 1107.4. ¹H NMR (400 MHz, DMSO) δ 9.75 (s, 1H), 8.33 (bs, 2H), 8.24 (s, 1H), 8.08 (d, 1H), 7.76 (t, 1H), 7.63 (d, J = 7.6 Hz, 2H), 7.55 - 7.38 (m, 7H), 7.27 (m, 2H), 7.00 (s, 2H), 6.94 (s, 1H), 6.26 (s, 2H), 5.14 (m, 2H), 4.96 (m, 2H), 4.65 - 4.42 (m, 3H), 4.32 - 4.14 (m, 5H), 3.50-3.35 (m, 10H), 3.04 - 2.77 (m, 12H), 2.58 (d, J = 6.3 Hz, 2H), 2.09 (t, J = 7.5 Hz, 3H), 1.67 - 1.55 (m, 2H), 1.53 - 1.27 (m, 6H), 1.24-1.18 (m, 10H), 0.93 (t, J = 7.3 Hz, 3H).

### 35. Preparation of Compound 59

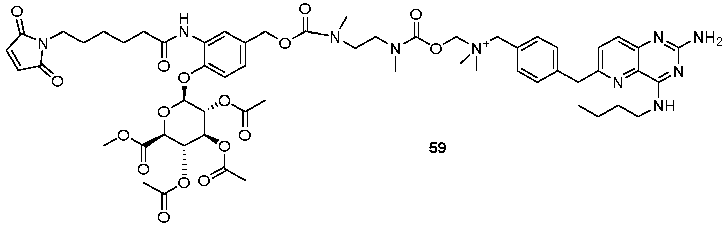

Compound 59 was prepared according to the synthesis method for compound 57, with replacing MC-VC-PAB-NPC with compound 41. LC-MS (ESI) [M]⁺: 1183.5.

### 36. Preparation of Compound 60

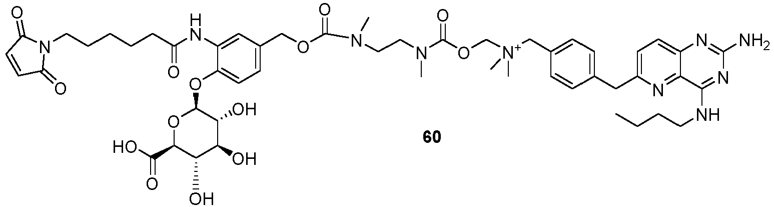

Compound 59 (35 mg, 0.03 mmol) was dissolved in a mixed solvent (THF:H₂O = 2:1). Lithium hydroxide monohydrate (16 mg, 0.3 mmol) was added, and after the resulting mixture was stirred at room temperature for 30 min, acetic acid was added to adjust pH to 7. The reaction solution was concentrated under reduced pressure to remove the organic solvent. Methanol was added for dissolving. The residue was subjected to reversed-phase chromatography to give the target product (5mg, yield: 15%). LC-MS (ESI) [M+H]⁺: 1043.4.

### Example 2: Preparation of Anti-PD-L1 THIOMAB

### 1. Gene editing for anti-PD-L1 THIOMAB

Avelumab (MSB0010718C, trade name: Bavencio) is a fully humanized IgG1 type monoclonal anti-PD-L1 antibody drug developed by Merck KGaA and Pfizer, which was approved by the FDA in 2017 for the treatment of Merkel-cell carcinoma (Apolo et al., 2017; Shirley, 2018), and whose encoding nucleotide sequences for the light and heavy chains are set forth in SEQ ID NOs: 1 and 2, respectively, and amino acid sequences of the light and heavy chains are set forth in SEQ ID NOs: 3 and 4, respectively. Avelumab, in addition to binding to human PD-L1, can also cross-react with murine PD-L1 (Deng et al., 2016). Therefore, by using avelumab as a starting material to develop an anti-PD-L1 ADC drug, the antibody itself could be ensured to have sufficiently excellent biological activity and druggability, and the obtained ADC drug was also suitable for early mouse model tests and human clinical tests in subsequent development. The expression plasmid of avelumab consists of a light chain plasmid 159-pFuse-αPDL1-LC and a heavy chain plasmid 162-pFuse-αPDL1-hIgG-Fc2, and the nucleotide sequences thereof are set forth in SEQ ID NOs: 5 and 6. The two plasmids were co-transfected into cells for expression, and after protein A resin purification, the anti-PD-L1 antibody avelumab could be obtained.

The inventors completed the construction of the THIOMAB expression plasmid by using the PCR site-directed mutagenesis method. A pair of fully complementary primers containing mutation sites and having opposite directions was designed, a complete plasmid to be mutated was taken as a template, and high-fidelity PCR enzyme was used to replicate and amplify the whole plasmid sequence having the mutation sites. The methylated template plasmid was then removed with the DpnI significant endonuclease, while the unmethylated PCR product remained. Further, through plasmid transformation and culture, the complete plasmid having mutation sites could be amplified.

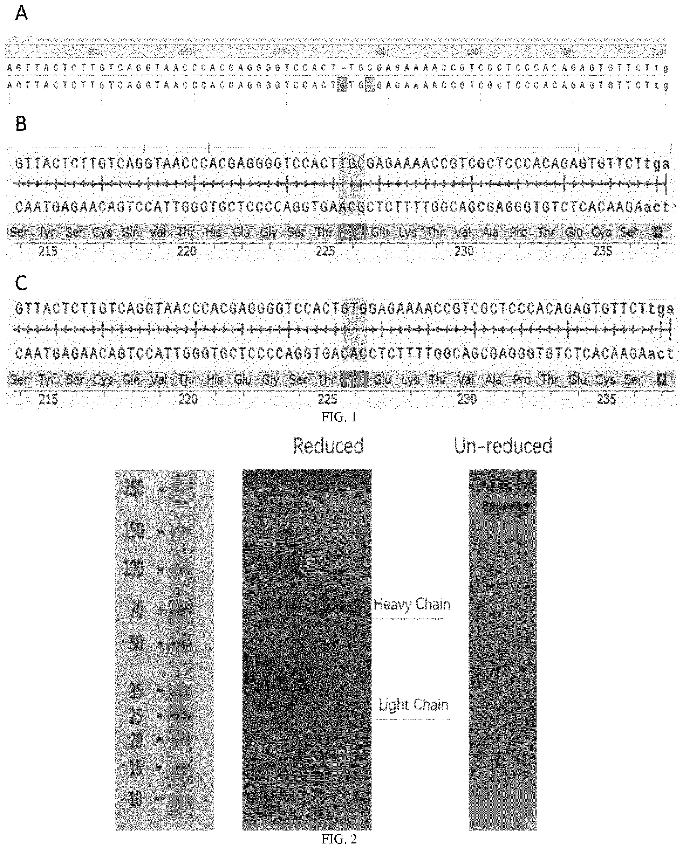

After the site-directed mutagenesis experiment was completed, the obtained plasmid was extracted for sequencing to identify whether the mutagenesis was successful. The plasmid, obtained after site-directed mutagenesis of the light chain plasmid 159-pFuse-αPDL1-LC, was named as αPD-L1 THIOMAB LC-V205C, and the nucleotide sequence thereof was set forth in SEQ ID NO: 7. The sequencing comparison results are shown in FIG. 1. After compared with the template plasmid, the sequence at the predetermined mutation site was mutated from GTG to TGC, while the other bases were unchanged, so the 205^{th} amino acid of the translated light chain was mutated from valine to cysteine. These two artificially introduced cysteines would be used for subsequent coupling reactions of the ADC drug. The light chain encoding nucleotide sequence of the antibody anti-PD-L1 THIOMAB obtained by mutation was set forth in SEQ ID NO: 8, and the amino acid sequence of the light chain was set forth in SEQ ID NO: 9.

### 2. Expression of anti-PD-L1 antibody and THIOMAB

The anti-PD-L1 antibody obtained after expression and purification was identified by reduced and non-reduced SDS PAGE analysis. As shown in FIG. 2. The antibody was reduced to light chains (~25 KD) and heavy chains (~50 KD) in reduced SDS PAGE, and still remained an intact antibody (~150 KD) in non-reduced SDS PAGE.

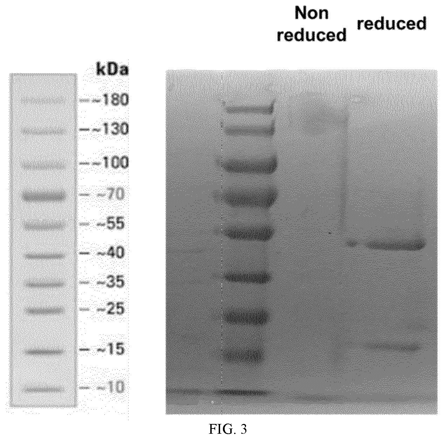

The anti-PD-L1 THIOMAB obtained after expression and purification was identified by reduced and non-reduced SDS PAGE analysis. As shown in FIG. 3, the SDS staining results of anti-PD-L1 THIOMAB (shown in FIG. 3) were substantially identical to those of anti-PD-L1 antibody (shown in FIG. 2): the antibody was reduced to light chains (~25 KD) and heavy chains (~50 KD) in reduced SDS PAGE, and still remained an intact antibody (~150 KD) in non-reduced SDS PAGE. Experiments prove that the anti-PD-L1 THIOMAB obtained by gene editing basically retained the original structure of the antibody, which indicates that the THIOMAB expression process and the self-assembly process were not influenced by artificial mutation. Meanwhile, under the same conditions, the expression amount of the antibody and THIOMAB was not greatly different, and the intracellular expression and assembly processes of the antibody were not greatly influenced.

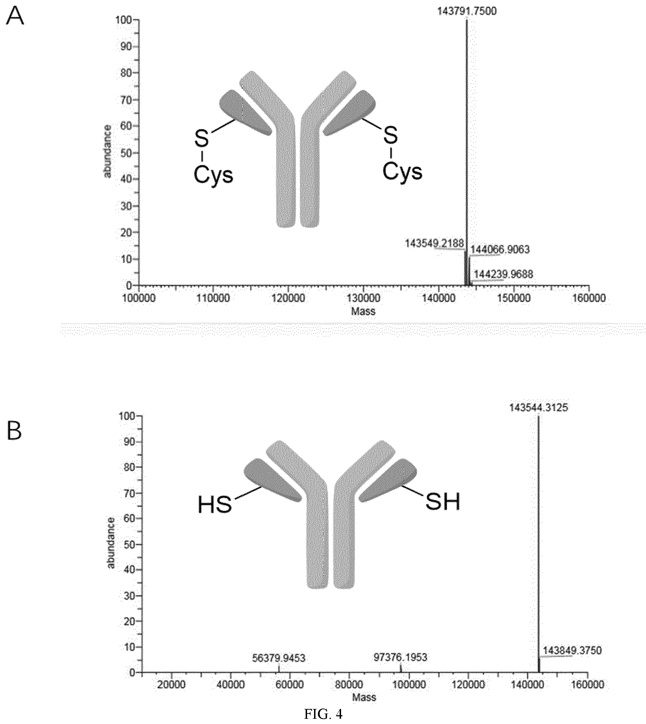

To further identify the resulting THIOMAB, the inventors treated the intact THIOMAB according to the "TCEP reduction-purification-DHAA reoxidation-purification" procedure to give free-sulfydryl-containing THIOMAB (THIOMAB-S2). The change in molecular weight thereof was detected by Exactive Plus EMROrbitrap LC-MS. The results are shown in FIG. 4. The molecular weight of intact anti-PD-L1 THIOMAB was about 143791.75, while the molecular weight of THIOMAB-S2 having a free sulfydryl group was about 143544.31, and the difference therebetween was 247.44. Theoretically, the sulfydryl group of the intact anti-PD-L1 THIOMAB was generally modified with a free cysteine, so the molecular weight thereof was about 238 in excess of that of the residue obtained from the condensation of two cysteines with the sulfydryl group, relative to THIOMAB-S2. This error was acceptable for antibody macromolecules. Therefore, it could be determined that the change in the molecular weight of the obtained THIOMAB satisfied the experimental expectation. This result shows that the inventors successfully expressed homogeneous anti-PD-L1 THIOMAB having two mutated cysteines by gene editing, which could be used for subsequent construction of homogeneous ADC drugs. In subsequent experiments, the inventors also needed to investigate whether such artificial mutations would affect the antigen-binding capacity and other activities of the antibodies by comparing the results of their *in-vivo* and *in-vitro* experiments with those of natural anti-PD-L1 antibodies.

As shown in FIG. 4, reduced and dialyzed THIOMAB lost two cysteines with exposing two free sulfydryl groups, compared with cysteine-capped native anti-PD-L1 THIOMAB. Thus, the predicted molecular weight of THIOMAB was decreased by 238 at this point, which became 143553.75, while the actual molecular weight of THIOMAB detected was 143544.31, which was decreased by 247.44.

### Example 3: Synthesis Route and Identification of THIOMAB-Based ADCs

### 1. Synthesis and identification of ADC HE-S2

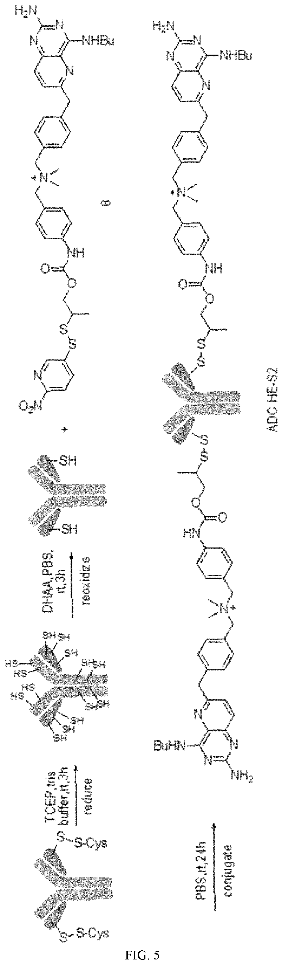

A schematic synthesis diagram of ADC HE-S2 is shown in FIG. 5. The inventors synthesized a conjugate of anti-PD-L 1 antibody with a disulfide bond linker and N⁴-butyl-6-(4-((dimethylamino)methyl)benzyl)pyridine[3,2-d]pyriproxyfen-2,4-diamine (hereinafter referred to as Compound D18) by taking anti-PD-L1 THIOMAB and Compound 20 (prepared in Example 1) as starting materials, named as ADC HE-S2. The linking site on the antibody was the cysteine at position 226 of the amino acid sequence (light chain sequence) set forth in SEQ ID NO: 9.

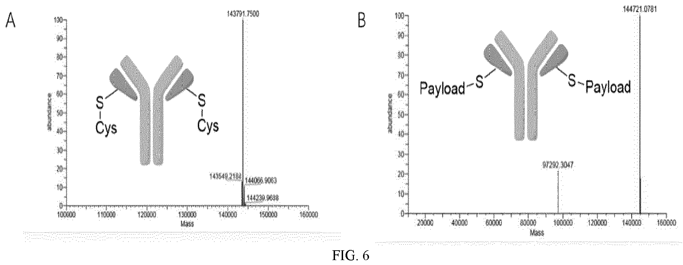

After the concentration and purity of ADC HE-S2 obtained from the reaction were determined by means of Nonadrop, the yield was estimated (about 30%-50%). A portion of the sample was sent to the National Center for Protein Science for detection of changes in molecular weights of antibodies by means of macromolecular MS, and the results are shown in FIG. 6. Theoretically, the change in the overall molecular weight of the ADC with DAR of 2 was increased by about 934 compared with THIOMAB after the sulfydryl group in ADC HE-S2 was modified with S2-D18. The molecular weight of ADC obtained by MS detection was 144721.08, while the molecular weight of THIOMAB was 143791.75, and the difference therebetween was 929.33. This error was acceptable for the protein mass spectrometry (~147 K). Therefore, it could be determined that a homogeneous ADC drug with DAR of 2 which was ADC HE-S2 (ADC1) was successfully prepared, as shown below.

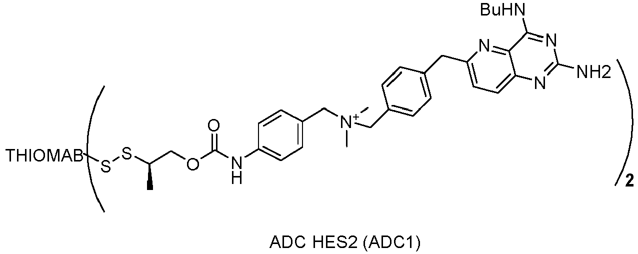

The mass spectrometry detection results for ADC HE-S2 are shown in FIG. 6, wherein the molecular weight of the MS-derived ADC was 144721.08, and the molecular weight of THIOMAB was 143791.75.

### 2. Synthesis and identification of other ADC drugs

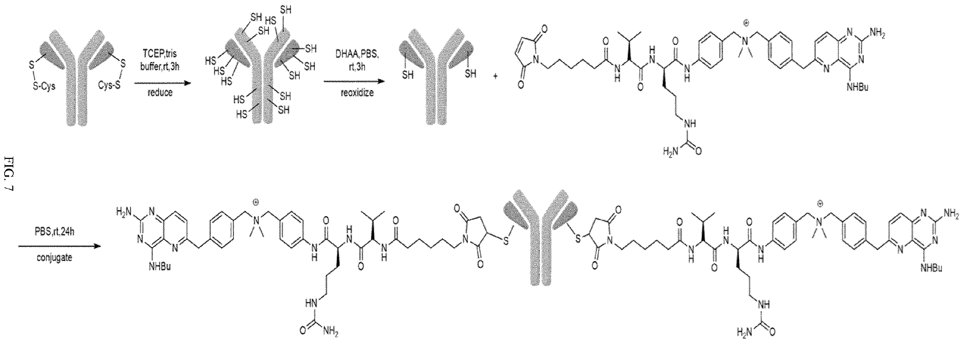

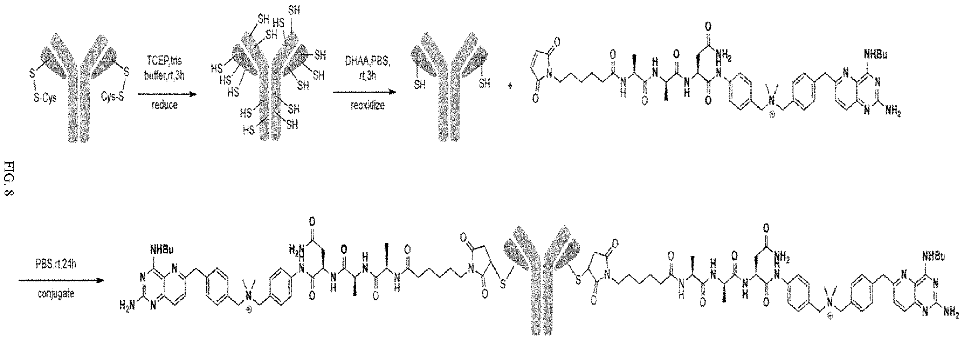

After the protocol for the synthesis of ADCHE-S2 was mature, the inventors used this conjugation strategy (by taking anti-PD-L1 THIOMAB, Compound 30, Compound 32, and Compound 57 (prepared in Example 1) as starting materials) to synthesize two ADCs with different linkers, ADC VC (ADC2) and ADC Legumain (ADC3), and ADC4. The synthesis flowcharts are shown in FIGs. 7 and 8, respectively.

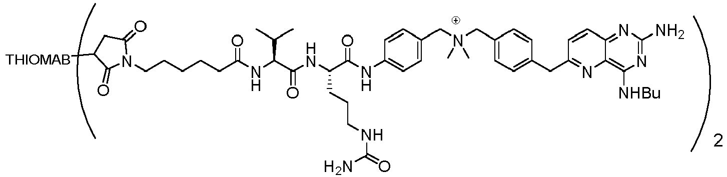

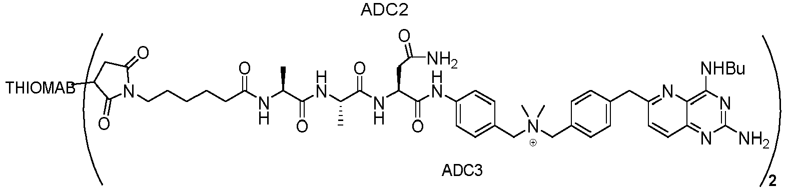

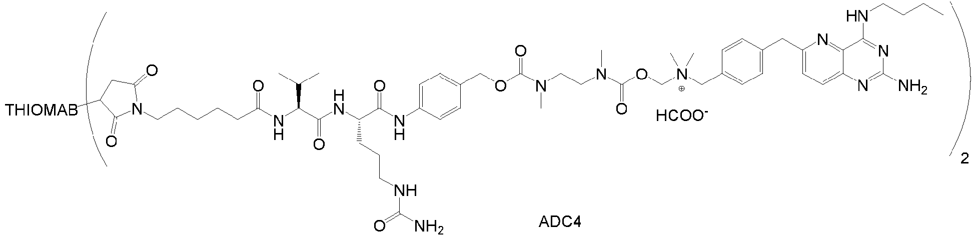

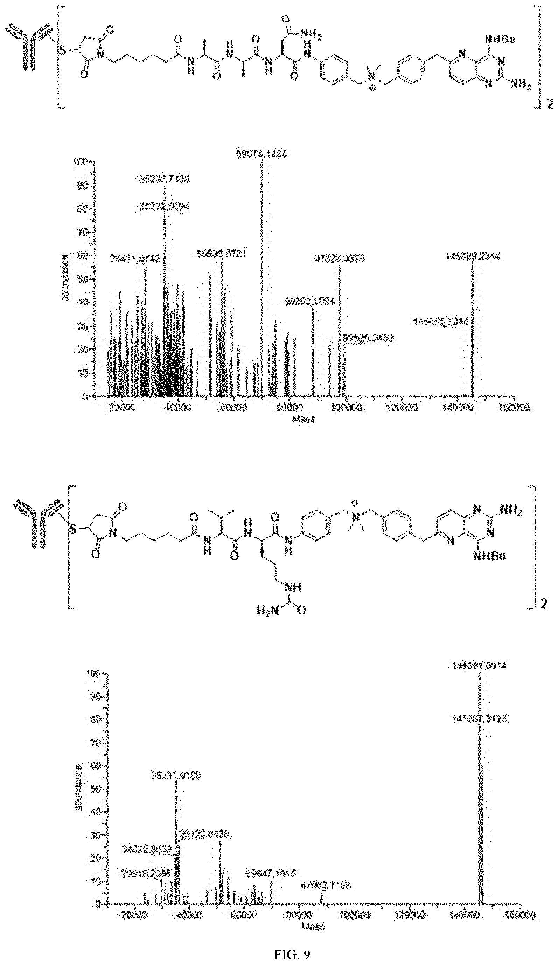

Through MS identification (as shown in FIG. 9), it could be calculated and verified that the theoretical molecular weight of ADC VC should be about 1600 greater than that of THIOMAB according to the previous calculation and analysis method. The MS results showed that the difference between the two was that 145399.23 - 143791.75 = 1607.48, which was very close to the theoretical value. Similarly, it could also be verified that the theoretical molecular weight of ADC Legumain should be increased by about 1600 over THIOMAB. The MS results showed that the difference between the two was that 145391.09 - 143791.75 = 1599.34, which was also very close to the theoretical calculation value. It could therefore be concluded that two homogeneous ADCs with DAR of 2 were obtained, ADC VC and ADC Legumain.

### Example 4: Synthesis Route and Identification of Wild-Type PD-L1 Antibody-Based ADCs

### 1. ADC coupling procedure and method

a. Preparation of reaction starting materials and reagents: an antibody (Avelumab, as described in Example 2) was formulated into a PBS solution with a concentration of 5-10 mg/mL; a TCEP aqueous solution with a concentration of 10 mM was prepared; conjugated small molecules (Compounds 39, 40, 43, 44, 51, 45, 49, 53, 57, 58, 60, prepared in Example 1) were formulated into DMSO solutions with a concentration of 10 mM; and a 20 mM cysteine PBS solution was prepared;
b. Antibody reduction: 10 or 20 equivalents of TCEP (10 mM, shaking at 37°C, 220 rpm/min, for 2-3 h) were added;
c. Coupling reaction: after the antibody was reduced, 10-20 equivalents of small molecule were added, followed by shaking at room temperature, 220 rpm/min, for 2-4 h;
d. Quenching reaction: a cysteine solution with a quantity equivalent to the small molecule was added, and incubated at 4°C or 10°C for 30 min to stop the reaction; and
e. Desalting: after the reaction was stopped, desalting was carried out using a 5 mL pre-packed desalting column of Cytiva.

### 2. ADC DAR value determination

The method for calculating DAR values by using ultra high performance liquid chromatography (UHPLC) and mass spectrometry (MS) was as follows:
a. Instruments: Waters Acquity-Class ultra-high performance liquid chromatograph (UHPLC) in series with Waters Synapt G2-Si Q-TOF high-resolution mass spectrum system, with high-purity nitrogen as atomizing gas and high-purity argon as collision gas.
b. Liquid chromatography conditions: chromatographic column: ACQUITY UPLC Protein BEH SEC chromatographic column (200 Å, 2.1 mm × 150 mm, 1.7 µm), column temperature: 25°C, and sample injection volume: 15 µL. Isocratic elution: 50 mM ammonium acetate as mobile phase, flow rate: 0.065 mL/min, collection time: 10 min, and ultraviolet detection wavelength: 280 nm.
   Mass spectrometry conditions: capillary voltage was set to be 3.0 kV for ESI source positive ionization mode scanning; cone voltage: 120 V, and source temperature and desolvation temperature were set to be 120°C and 500°C, respectively; cone gas flow and desolvation gas flow were set to be 20 L/h and 600 L/h, respectively, and primary mass spectral range was m/z 400 to m/z8000.
c. Data analysis: data were analyzed using Waters Masslynx software, and mass spectral data were deconvoluted using the MaxEnt 1 plug-in. ADC DAR values were determined by analysis of mass-spectrum deconvolution spectra. First, peaks were integrated and peak area percentages were calculated, with peak area percentage sum being 100. According to the formula: DAR = Σ weighted peak area/100, mean weighted drug coupling ratio for ADC was calculated.

### 3. Specific ADC product structure information

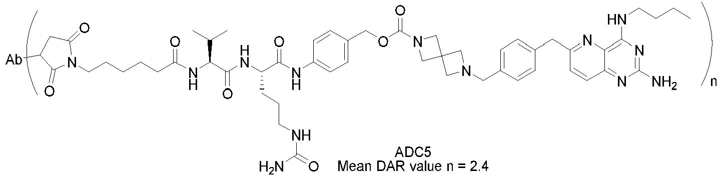

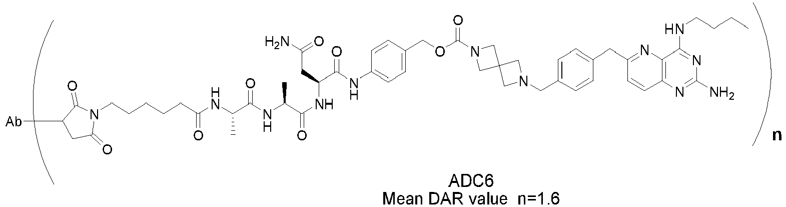

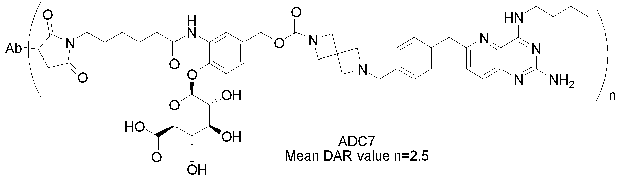

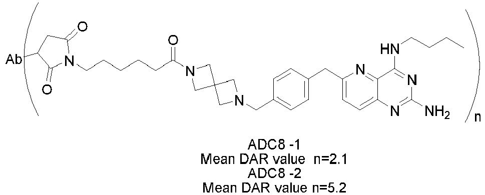

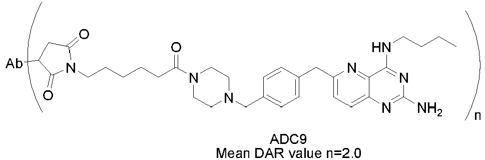

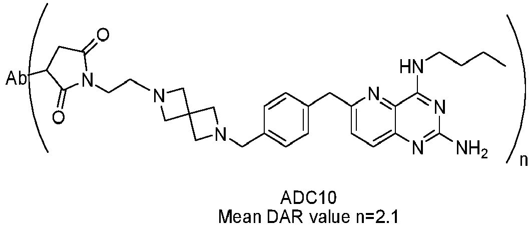

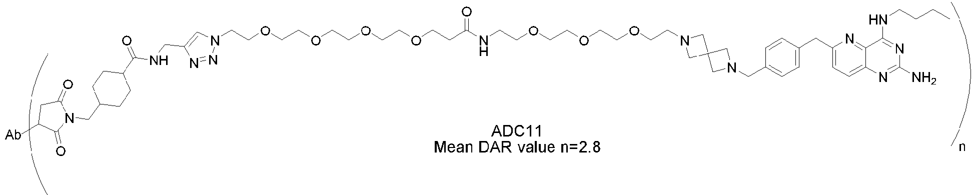

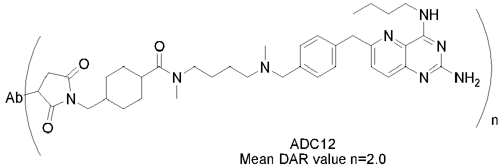

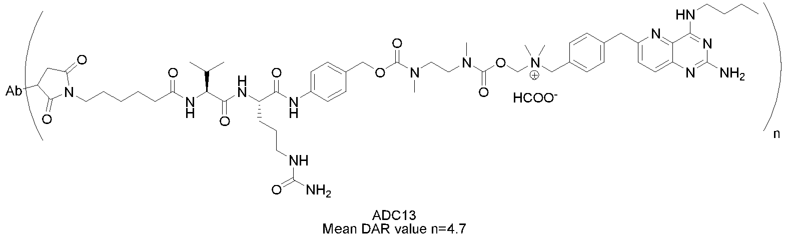

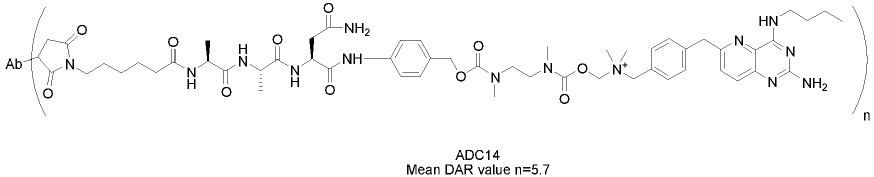

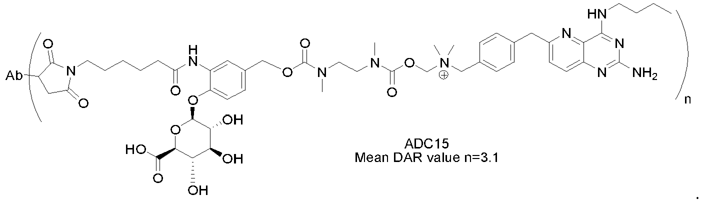

### Example 5: Biological Activity Study

### 1. Detection for antigen-binding capacity of ADC HE-S2 (ADC1)

Sufficient specific antigen affinity is the basis of the biological activity for antibodies and is crucial to the activity and efficacy of antibody-based drugs. Since the ADC drug preparation process involves modification, chemical treatment and small molecule conjugation for the antibodies, these may destroy or affect the original antigen affinity and stability of the antibodies, resulting in impaired antigen-binding capacity of the antibodies. Therefore, in the antibody treatment process, the conditions used need to be as mild as possible; the choice of the modification and conjugation sites should also avoid the antigenic determinants of the original antibody in order to avoid impairing the antigen-binding capacity of the antibody. In addition, the antigen affinity of the resulting ADC drugs should also be verified to ensure that the ADC drugs retain sufficient targeting capacity and specificity.

The antibody-antigen affinity of ADC HE-S2 designed and prepared in the present invention is an important basis for achieving the anti-tumor activity thereof. ADC HE-S2 requires specific binding of antibodies to highly expressed PD-L1 in tumors to achieve targeted delivery for the immunomodulator D18. Meanwhile, ADC HE-S2 can also bind to PD-L1 on the cell surfaces to block an immunosuppressive signal pathway of tumor PD-1/PD-L1, thereby inducing a T-cell anti-tumor response. Thus, after successfully preparing ADC HE-S2, the inventors firstly demonstrated their capacity to recognize and bind to PD-L1 antigen *in vitro.*

With PD-L1 positive MC38 as a platform, the inventors verified whether ADC HE-S2 and the gene-modified anti-PD-L1 THIOMAB retained the original antibody-antigen binding capacity by using a fluorescence activated cell sorting (FACS) technique. In the experiment, ADC HE-S2, anti-PD-L1 antibody (i.e., avelumab as described in Example 2) and anti-PD-L1 THIOMAB (i.e., mutant antibody prepared in Example 2) serving as primary antibodies were incubated with MC38 cells, such that the antibodies could be sufficiently contacted and bind to PD-L1 on the cell surfaces. After washing with PBS, the cells were treated with an FITC-labeled goat anti-human secondary antibody, and incubated in the dark for 30 min to allow the secondary antibody to bind fully to the primary antibodies. Simultaneously, two groups of negative controls were set, wherein one group was marked as a negative control (NC), only isotype control IgG was added to treat the cells, no staining was carried out, and the fluorescent background of the cells was represented; the other group was marked as a secondary antibody, and after addition of isotype control IgG, the secondary antibody was stained with FITC-labeled secondary antibody along with other experimental groups to characterize false positive fluorescence signals generated by non-specific binding of secondary antibodies to the cells. The stained MC38 cells were analyzed on a BD FACSAria^{™} III flow cytometer, and the strength of FITC fluorescence signals on MC38 cells treated with different antibody-based drugs was detected and compared, so as to characterize the affinity of ADC, THIOMAB and antibodies to PD-L1. All results were processed through FlowJo software (TreeStarInc.) and are shown in FIG. 10.

The FACS results are shown in FIG. 10, wherein the control group stained with a fluorescent secondary antibody produced a false positive signal with a shift rate of only 1.7% based on the blank control (NC) group with IgG isotype antibody; the mean fluorescence intensity (MFI) calculation results showed that the MFI of the NC group was 142.00 while the MFI detected after MC38 were stained with the secondary antibody was 166.75, although the difference was significant (*p* < 0.0001) but the difference value was relatively small. This significant and weak difference was a false positive signal produced by the non-specific binding of FITC secondary antibody to MC38 cells. Experiments proved that as the phenomenon of false positive binding was relatively weak, the results were not influenced. Thus, the NC control group was used as a reference basis to evaluate the antibody-antigen binding capacity of anti-PD-L 1 antibody-based drugs.

Similar FITC positive signals were observed on MC38 cells treated with anti-PD-L1 antibody, anti-PD-L1 THIOMAB and ADC HE-S2, and the shift rates of the FITC positive signals relative to the NC group were 35.7%, 36.6% and 34.2%, respectively, which were much higher than the shift rate (1.7%) of the false positive signal produced by non-specific binding of the fluorescent secondary antibody. The MFI calculations also showed that the evaluated fluorescence intensities of the anti-PD-L1 antibody, anti-PD-L1 THIOMAB, and ADC HE-S2 were 445.3, 449.0, and 392.5, respectively, which were also significantly higher than those of the two control groups (p < 0.0001), which indicated that all three antibodies could specifically bind to PD-L1. Meanwhile, the analysis from the Student's t test proved that the MFI detected by the gene-edited anti-PD-L1 THIOMAB and the further chemically modified ADC HE-S2 had no significant difference compared with the natural anti-PD-L1 antibody. This indicates that the gene editing and chemical modification of the inventors neither cause impairment of the original antigen recognition affinity of the antibody, nor cause non-specific off-target phenomenon of the antibody.

Therefore, the inventors could confirm that ADC HE-S2 still maintained the antigen recognition affinity of the original anti-PD-L1 antibody, and could be further applied to the following experiments and development.

### 2. Internalization capability of ADC HE-S2 (ADC1)

Drug release from the ADC is widely thought to be dependent on the internalization capability of the corresponding antibody. The antibody-antigen complex formed after the ADC bound to the antigen protein on the cell surface needed to enter the target cells along with the internalization of the antigen protein, thereby releasing the conjugated drug under the action of intracellular enzymes or other intracellular physiological environments. Thus, the internalization capability of the formed complex upon binding of the selected antigen to the ADC is critical to the function realization of the ADC drug. Since ADC HE-S2 designed by the inventors should theoretically act both in cancer cells and DC cells, the inventors verified the capacity of anti-PD-L1 antibody-based drugs to internalize into tumor cells and DC cells through *in-vitro* experiments.

The pHrodo^{™} red dye is known to be a pH sensitive fluorescent dye, which rapidly increases in molecular fluorescence intensity when the surrounding environment becomes acidic (Lehrman et al., 2018). When the dye was internalized into the cells along with the antigen-antibody complex, the pH of the environment around the dye was reduced to acidity, thereby producing an observable fluorescence signal. This property allowed the internalization process of the antibody to be monitored by using the pHrodo^{™} red dye. Through the IncuCyte^{®} viable cell analysis system, the inventors could perform image-based monitoring and quantitative analysis on the internalization process of the anti-PD-L1 antibody labeled with the pHrodo^{™} red dye. In 0.1 M sodium carbonate (pH 8.3) buffer, 1 mg/mL of anti-PD-L1 THIOMAB and 3-5 equivalents of pHrodo^{™} red dye were incubated together for about 1 h, dialyzed and purified to remove residual small molecules so as to obtain fluorescent dye-labeled anti-PD-L1 THIOMAB. The labeled antibody should be stored in the dark at 4°C for later use; the antibody should be stored in the dark in an environment at -20°C for long-term storage to avoid repeated freezing and thawing. To characterize the internalization process of the PD-L1/anti-PD-L1 antibody complex, the inventors chose to label anti-PD-L1 THIOMAB with the pHrodo^{™} red dye. In the foregoing, the inventors have verified that anti-PD-L1 THIOMAB had the same antigen recognition ability as ADC HE-S2, and therefore the internalization capacity of anti-PD-L1 THIOMAB could also reflect the internalization capacity of ADC HE-S2.

### (1) Internalization experiment for anti-PD-L1 THIOMAB on cancer cells

The internalization of the PD-L1/anti-PD-L1 antibody complex was verified by taking two PD-L1 positive tumor cells of MC38 and B16 as materials. After fluorescently-labeled anti-PD-L1 THIOMAB was added into the cells, the cells were incubated in the dark for 24 h, the distribution of red fluorescence signals in the cells was photographed by using an IncuCyte^{®} viable cell analysis system, and the results are shown in FIG. 11A.

It was clearly observed that red signals were clearly generated in the cells after the addition of labeled THIOMAB, whereas little red fluorescence was observed in the control group without the antibody. As could be seen from the synthesized images, little red fluorescence was produced extracellularly, demonstrating the correlation of the fluorescence signals with antibody internalization. Thus, the inventors could evaluate the internalization level of fluorescently-labeled THIOMAB by analyzing the total red object integrated intensity obtained by analysis using IncuCyte^{®} software, as shown in FIG. 11B. After the addition of labeled THIOMAB, the total red object integrated intensity in MC38 was 13701.63, which was significantly higher than 84.88 in the control group, and the signal intensity in B16 was even as high as 283613.60, which was also significantly higher than 98.53 in the control group. The fluorescence signals of B16 cells were also stronger than those of MC38 cells, which could be caused by the internalization rate of PD-L1 of different cells and the difference of pH values in the cells.

### (2) Internalization and selectivity experiments for anti-PD-L1 THIOMAB on DC cells

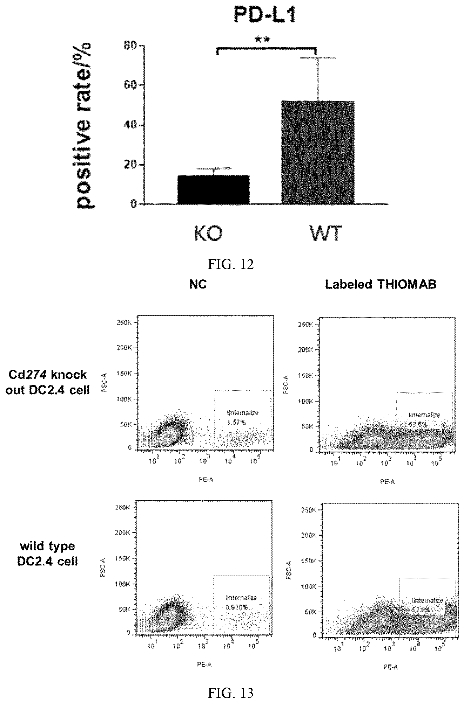

To verify internalization of anti-PD-L1 THIOMAB on DC cells, the inventors acquired two kinds of DC cells, which were C*d274* knockout DC 2.4 cells and wild-type DC 2.4 cells, from the teacher named Tang Haidong of the School of Pharmaceutical Sciences, Tsinghua University. The C*d274* gene was a PD-L1 encoding gene, and the knocked-out DC 2.4 could not express PD-L1 protein, so the first kind of cells was PD-L1 negative DC cells; while wild-type DC 2.4 could express PD-L1, so the second kind of cells was PD-L1 positive DC cells (as shown in FIG. 12). According to the hypothesis, the inventors could observe that anti-PD-L1 THIOMAB could be internalized into PD-L1-positive DC cells, but not into PD-L1 negative C*d274* knockout DC 2.4 cells. Therefore, through the anti-PD-L1 THIOMAB internalization experiments for the two kinds of cells, the inventors could simultaneously verify the internalization capacity of the anti-PD-L1 THIOMAB on DC cells and the selectivity of the antibody.

In preliminary experiments, the inventors added 10% heat-inactivated fetal bovine serum (FBS) as a blocking agent to a culture medium of DC 2.4 cells according to a conventional procedure, and detected the red fluorescence signals in the cells by FACS after incubation for 8 h, and the results are shown in FIG. 13. Surprisingly, FACS results showed that the same proportion of red fluorescence positive cells was observed in both PD-L1 positive wild-type DC 2.4 cells and PD-L1 negative C*d274* knockout DC 2.4 cells, with the proportions being 53.6% and 52.9%, respectively. Few red fluorescence-positive cells (1.57% and 0.92%) were observed in the negative control group to which no fluorescently-labeled anti-PD-L1 THIOMAB was added. Similar results were obtained by multiple reuses, which suggested that: the anti-PD-L1 THIOMAB labeled with the fluorescent dye could be successfully internalized into PD-L1 positive DC 2.4 cells and also into PD-L1 negative C*d274* knockout DC 2.4 cells. This unexpected result was contrary to the inventors' hypothesis.

The inventors found in literature investigations that the binding capacity of the Fc fragment of human IgG to the mouse Fcy receptor was very similar to its binding capacity to the human orthologous Fcy receptor (Dekkers et al., 2017). The inventors therefore speculated that the murine Fcy receptor, which was highly expressed and highly active on the surfaces of DC 2.4 cells, could also bind to and internalize the humanized anti-PD-L1 antibody, thereby allowing both kinds of cells to internalize the anti-PD-L1 antibody.

To verify this hypothesis, the inventors used 10% mouse serum and 10% FBS as blocking agents which were added to cell culture media in groups, respectively. Relative to FBS, murine IgG enriched in mouse serum could block Fcy receptors on the surface of DC 2.4 more effectively, thereby inhibiting the antibody internalization process mediated by Fcy receptors on DC cells. Before addition of THIOMAB, the DC cells should firstly be incubated at 4°C for 30 min to allow sufficient binding of IgG in the mouse serum to Fcy receptors on the surfaces of the DC cells while halting the internalization process of the receptors on the cell surfaces. Thereafter, the fluorescently-labeled anti-PD-L 1 THIOMAB was added to the cells in each group. Then, the cells were incubated at 4°C in the dark for 30 min to allow the antibody and the antigen to contact and bind sufficiently under the condition that the internalization of the cells was stopped.

The total red object integrated intensity-time variation curves obtained from the internalization experiments for anti-PD-L1 THIOMAB are shown in FIGs. 14A and B. The results in FIG. 14A showed that very similar fluorescence intensity variation curves could be observed in PD-L1 positive wild-type DC 2.4 cells and PD-L1 negative C*d274* knockout DC 2.4 cells after addition of 10% FBS for blocking; while no variation in fluorescence signals was observed in the negative control group to which no fluorescently-labeled anti-PD-L1 THIOMAB was added.

As shown in FIG. 14B, in the experimental group to which 10% mouse serum was added, the speed for increase in the total red object integrated intensity in the PD-L1 positive wild-type DC cells was significantly higher than that of the PD-L1 negative C*d274* knockout DC cells. Especially in the initial stage of detection (0-60 min), PD-L1 negative cells produced very few fluorescence signals, the variation curve was similar to that of the negative control, then the fluorescence signal produced by the antibody began to appear in the PD-L1 negative DC cells, but the rate (slope) of signal enhancement still lagged behind that of the wild-type DC 2.4 cells, and finally t-test analysis proved that the difference between the two kinds of cells was significant (*p* < 0.0001). After the cells were cultured for 6 h, the blockage by the mouse serum in the experimental group was photographed by using the IncuCyte^{®} viable cell analysis system, and the distribution of red fluorescence signals in cells in each group was observed. The results are shown in FIG. 14A, and the red fluorescence signals of wild-type DC 2.4 cells were significantly stronger than those of C*d274* knockout DC cells. This phenomenon could be explained by the fact that PD-L1 on the surfaces of DC cells could bind fully to the anti-PD-L1 antibody and the Fcy receptor was also well blocked by homologous IgG in mouse serum, since the cellular receptor internalization process was almost stopped and antigen-antibody binding was not affected during the incubation at 4°C for 30 min prior to detection. When the detection began, the cell surface receptor internalization process gradually resumed as the culture temperature gradually returned to 37°C, and anti-PD-L1 THIOMAB, which had previously bound fully to PD-L1 on the surfaces of wild-type DC cells, was also rapidly internalized, whereas the internalization could not occur in PD-L1 negative cells. With the prolonged incubation time, PD-L1 negative DC cells could internalize anti-PD-L1 THIOMAB through the recycled Fcy receptor, thereby producing a fluorescence signal. However, this internalization process was competitively inhibited by mouse homologous IgG in the culture medium. The rate of internalization of THIOMAB in C*d274* knockout DC cells thus still lagged behind the rate of internalization of THIOMAB binding to PD-L1 in wild-type DC 2.4 cells.

This result verified the inventors' hypothesis and demonstrated the capacity of the anti-PD-L1 antibody to be internalized into cells by binding to PD-L1 on the cell surfaces. Meanwhile, the difference in internalization rates of anti-PD-L1 THIOMAB in C*d274* knockout cells and wild-type DC 2.4 cells after blocking with mouse serum also demonstrated the good selectivity of the anti-PD-L1 antibody.

### Example 6: Anti-tumor Activity Study

### 1. Study on anti-tumor activity of ADC HE-S2 (ADC1)

To verify the anti-cancer activity of ADC HE-S2, the inventors first made a mouse model with MC38. After the tumor was loaded, a visible tumor could be observed on the mouse surface for about one week. When the mean tumor tissue volume of the mice reached 100 mm³, the mice were randomly grouped as follows according to the final type of drug administered: IgG isotype control antibody group (abbreviated as IgG), anti-PD-L1 antibody group (anti-PD-L1), anti-PD-L1 THIOMAB group (Thiomab), D18 group (D18), D18/anti-PD-L1 antibody combination treatment group (anti-PD-L1 & D18), and ADC HE-S2 group (ADC HE-S2), with 7-8 mice per group. The IgG isotype control antibody group, the anti-PD-L1 antibody (i.e., avelumab as described in Example 2) group, and the anti-PD-L1 THIOMAB (i.e., mutant antibody prepared in Example 2) group were administered twice a week via intraperitoneal injection of 150 µg per mouse for each time. The D18 group was administered twice a week via intraperitoneal injection of 25 µg per mouse for each time. In the D 18/anti-PD-L 1 antibody combination treatment group, the anti-PD-L1 antibody was dosed twice a week, 150 µg per mouse for each time; the D18 was dosed once, 25 µg per mouse for each time; in the ADC HE-S2 group, ADC HE-S2 was dosed once a week via intraperitoneal injection of 150 µg per mouse for each time; the anti-PD-L1 antibody was dosed once a week, staggered from the administration of the ADC, via intraperitoneal injection of 150 µg per mouse for each time.

The experimental results are shown in FIG. 15A. For MC38 tumors, the efficacies of the three drugs, anti-PD-L1 antibody, anti-PD-L1 THIOMAB and D18, on single treatment were all very similar: the inhibitory activities on tumor growth were not significantly different from each other, but were slightly superior to the efficacy of the IgG isotype antibody as the negative control. This result indicated that both the D18 and the anti-PD-L1 antibody had certain anti-cancer activity, while anti-PD-L1 THIOMAB also retained the biological function of the original antibody. The combination treatment ofD18 and the anti-PD-L1 antibody showed very good inhibitory activity against tumor growth, and the efficacy was far better than that of monotherapy of any group. This result again demonstrated the synergistic effect between D18 and anti-PD-1/PD-L1 antibody for blocking treatments. ADC HE-S2 showed very surprising anti-cancer activity, and the inhibitory activity against tumor growth was significantly superior to that of the combination treatment group (p < 0.001). When the experiment was carried out to day 24, the tumor volumes of the mice in the combination treatment group began to greatly increase; while in the ADC treatment group, the tumor sizes of the mice were kept low.

In order to further study the survival rate of the mice after long-term administration, the inventors carried out a mouse survival experiment. The experiment finally continued until day 70 (as shown in FIG. 15B). The survival rate of mice in the ADC HE-S2 group was 6/8, and it was worth emphasizing that tumors in the last six alive mice were barely detectable. The survival rate of the mice in the D18/anti-PD-L1 antibody combined administration group was 4/8; the survival rate of the mice in the D18 group was 1/8; while all the mice in the other two single administration groups and the negative control group died. This result also demonstrated the strong anti-cancer activity of ADC HE-S2.

In addition, the inventors also used a B 16 melanoma mouse model to further demonstrate the efficacy of ADC HE-S2 (as shown in FIG. 15C). The dosage and time for administration are also shown in Table 1. B16 melanoma is a tumor with low PD-L1 level and certain drug resistance to PD-1/PD-L1 blocking treatment. As a result, as expected by the inventors, the inhibitory effect of each drug on B16 melanoma was reduced relative to the MC38 tumor. The efficacies of three of the anti-PD-L1 antibody, anti-PD-L1 THIOMAB and D18 in the single administration groups were almost comparative to that of the IgG isotype antibody. Only the anti-PD-L1 antibody + D18 combination treatment and ADC HE-S2 still showed some tumor inhibitory effect. The results that compared to mice after the combination treatment, the growth rate of B16 tumors in mice treated with ADC HE-S2 was significantly slow down (p < 0.05) demonstrated that the anti-tumor activity of ADC HE-S2 in the B16 tumor model was also stronger than that of the anti-PD-L1 antibody/D 18 combination treatment.

**Table 1. Medication schedule for treatment of mice**

| Group | 10^{th} day | 13^{th} day | 17^{th} day | 21^{th} day | 24^{th} day* |
|---|---|---|---|---|---|
| Control | IgG | IgG | IgG | IgG | IgG |
| αPD-L1 | αPD-L1 | αPD-L1 | αPD-L1 | αPD-L1 | αPD-L1 |
| THIO MAB | THIO MAB. | THIO MAB | THIO MAB | THIO MAB | THIO MAB |
| D18 | D18 | - | D18 | - | D18 |
| αPD-L1 /D18 | αPD-L1 /D18 | αPD-L1 | αPD-L1 /D18 | αPD-L1 | αPD-L1 /D18 |
| ADC HE-S2 | ADC HE-S2 | αPD-L1 | ADC HE-S2 | αPD-L1 | ADC HE-S2 |

Dosage: Antibody drugs, administered 150 µg per mouse for each time, and D18, administered 25 µg per mouse for each time. - means no administration on the day. * Only mice with MC38 tumor model were allowed to be administered until the 24th day, and mice with B16 tumor model were no longer administered after the 21st day.

### 2. ADC coupling strategy expands the therapeutic window of D18

It was noted that since the ADC HE-S2 had a DAR of 2 and a molecular weight of about 150 K, the content of D18 in the ADC per dose could be estimated simply as 150 µg/150000 × 2 × 364 = 0.748 µg, much less than the dose given in combination (25 µg per mouse per dose). Therefore, the inventors wanted to investigate the effect of D18 dose variation on the synergistic anti-cancer effect between D18 and the anti-PD-L 1 antibody.

Thus, the inventors used different doses of D18 (0.25 µg, 2.5 µg, and 25 µg) in combination with 150 µg of anti-PD-L1 antibody to treat MC38 tumors (as shown in FIG. 16). Mice were grouped as follows according to type of drug administered: IgG isotype antibody control group (abbreviated as IgG), anti-PD-L1 antibody group (anti-PD-L1), anti-PD-L1 antibody + D18 (25 µg) group (anti-PD-L1 + D18 (25 µg)), anti-PD-L1 antibody + D18 (2.5 µg) group (anti-PD-L1 + D18 (2.5 µg)), and anti-PD-L1 antibody + D18 (0.25 µg) group (anti-PD-L1 + D18 (0.25 µg)). Dosing was started after the mean tumor volume of mice in each group reached 100 mm³. The IgG isotype antibody and the anti-PD-L1 antibody were dosed twice a week via intraperitoneal injection of 150 µg per mouse for each time; D18 was dosed once a week via intraperitoneal injection of 25 µg, 2.5 µg and 0.25 µg per mouse for each time, respectively, per group; mice in the IgG isotype group were intraperitoneally injected weekly with the same dose of DMSO as given for D18 as a control.

The anti-tumor results of the gradient dose ofD18 in combination with the anti-PD-L1 antibody are shown in FIG. 16. The experimental results proved that the D18 had the function of enhancing the anti-tumor activity of the anti-PD-L1 antibody and showed the characteristic of dose dependence. The combination treatment with the anti-PD-L1 antibody showed significant tumor inhibitory activity when D18 was dosed at a dose of 25 µg per mouse for each time. The anti-tumor effect of the D18/anti-PD-L1 antibody combination treatment was also reduced with the decrease in the dose of D18 used. In fact, when the dose used was reduced to 2.5 µg or 0.25 µg per mouse, the tumor inhibitory effect was not significantly different from that of the anti-PD-L1 antibody alone.

It was noted that the loaded D18 dose (about 0.75 µg) at each administration of ADC HE-S2 was much less than 2.5 µg. Therefore, it was determined that through ADC targeting administration, the inventors utilized the D18 dose (about 0.75 µg) which was not enough to exert activity in combined administration, and acquired a more remarkable tumor inhibitory effect than that of combined treatment of 25 µg of D18 and anti-PD-L1 antibody.

Then, the inventors examined the effect of each group of drug treatment on the body weight of mice in a mouse model to determine the toxic and side effects of the drugs used, and the results are shown in FIG. 17. Healthy mice were divided into an ADC group, an anti-PD-L1 antibody + D18 group, an anti-PD-L1 antibody group, a D18 group and an IgG group according to the administration, with 5 mice per group. The mode and dose of administration for each group of mice were the same as those in the mouse tumor model experiment: in the ADC group, ADC HE-S2 was dosed once a week, and the anti-PD-L1 antibody was injected once, 150 µg for each time; D18 was dosed once a week, 25 µg for each time; the anti-PD-L1 antibody and the IgG isotype antibody were dosed twice a week, 150 µg for each time.

The body weight change of the mice in the anti-PD-L1 antibody group was almost consistent with that of the blank control IgG group, demonstrating that the toxic and side effects of the anti-PD-L1 antibody on the mice were small. The anti-PD-L1 antibody + D18 group and the D18 group showed some decreases in body weight of the mice after each D18 injection. The result that the body weight loss of the mice in the anti-PD-L1 antibody + D18 group was particularly significant (*p* < 0.05) relative to the administration ofD18 alone verified the previous hypothesis of the inventors; namely the anti-PD-L1 antibody as an immune checkpoint inhibitor would increase the toxic and side effects of the TLR7/8 agonist D18. Although certain drop in body weight of mice in the ADC group would be caused by each injection of ADC HE-S2, the drop amplitude of this group was significantly less than that of the D18 group (*p* < 0.05) and the combined administration group (*p* < 0.01). After administration, the body weight of the mice also quickly returned to normal, and finally the change in body weight of the mice in the ADC group was not significantly different from that in the control group. These results demonstrated that ADC HE-S2, while also exhibiting some toxicity, was significantly less toxic than D18 alone and in combination, and brought less burden on the bodies of the mice. The lower amount of D18 actually loaded in the ADC should be the main reason for the reduced toxicity of D18.

Finally, it could be concluded that ADC HE-S2 exhibited better tumor inhibitory activity than when administered in combination with a lower actual D18 dosage, while also significantly reducing the toxic and side effects of the combination treatment. By the ADC strategy proposed by the inventors, the lowest effective dose of D18 in use was greatly reduced, and the therapeutic window of D18 was expanded.

### 2. Anti-tumor experiment in mice for antibody-conjugated drug ADC10 based on wild-type PD-L1 antibody

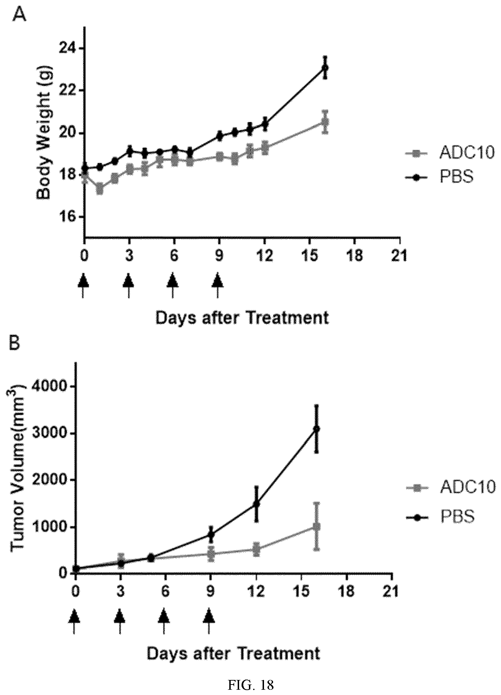

MC38 cells (1 × 10⁶ cells/mouse) were inoculated subcutaneously into the right abdominal region of the mice. When the mean tumor tissue volume of the mice reached 100 mm³, the mice were randomly grouped as follows according to the final type of drug administrated: PBS control group, and ADC10 group, with 5-6 mice per group. The dosing was performed twice a week via intraperitoneal injection of 10 mg/kg per mouse for each time, the change in body weight of each mouse was measured every day (FIG. 18A), and the tumor volume of the mice was measured every 3 days (FIG. 18B). As shown in FIG. 18B, the tumor inhibitory rate of the ADC was 70%.

The above description is only for the purpose of illustrating the preferred examples of the present invention, and is not intended to limit the scope of the present invention. Any modifications, equivalents, and the like made without departing from the spirit and principle of the present invention shall fall in the protection scope of the present invention.

The foregoing examples and methods described herein may vary based on the abilities, experience, and preferences of those skilled in the art.

The certain order in which the steps of the method are listed in the present invention does not constitute any limitation on the order of the steps of the method.

## Claims

1. An antibody-drug conjugate, or a pharmaceutically acceptable salt, a solvate, a prodrug, or a stereoisomer thereof, wherein the antibody-drug conjugate has the following structure: 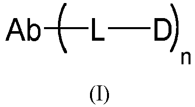 wherein,
Ab is an antibody or an antigen-binding fragment thereof;
D is a small molecule drug;
L is a linking unit connecting Ab and D; and
n is an integer of 1 to 100.

2. The antibody-drug conjugate according to claim 1, wherein an antigen targeted by the antibody is selected from: Claudin 18.2, GPC3, HER-2/neu, carbonic anhydrase IX, B7, CCCL19, CCCL21, CSAp, BrE3, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD44, CD45, CD46, CD52, CD54, CD55, CD59, CD64, CD67, CD70, CD74, CD79a, CD80, CD83, CD95, CD126, CD133, CD138, CD147, CD154, CEACAM-5, CEACAM-6, alpha-fetoprotein (AFP), VEGF, ED-B fibronectin, EGP-1, EGP-2, EGF receptor (ErbB1), ErbB2, ErbB3, factor-H, FHL-1, Flt-3, folate receptor, Ga 733, GROB, HMGB-1, hypoxia inducible factor (HIF), HM1.24, insulin-like growth factor (ILGF), IFN-γ, IFN-α, IFN-β, IL-2R, IL-4R, IL-6R, IL-13R, IL-15R, IL-17R, IL-18R, IL-2, IL-6, IL-8, IL-12, IL-15, IL-17, IL-18, IL-25, IP-10, IGF-1R, Ia, HM1.24, ganglioside, HCG, HLA-DR, CD66a-d, MAGE, mCRP, MCP-1, MIP-1A, MIP-1B, macrophage migration inhibitory factor (MIF), MUC1, MUC2, MUC3, MUC4, MUC5, PD-1, PD-L1, placental growth factor (PIGF), PSA, PSMA, PSMA dimer, PAM4 antigen, NCA-95, NCA-90, A3, A33, Ep-CAM, KS-1, Le(y), mesothelin, S100, tenascin, TAC, Tn antigen, Thomas-Friedenreich antigen, tumor necrosis antigen, tumor angiogenesis antigen, TNF-α, TRAIL receptor (R1 and R2), VEGFR, RANTES, T101, cancer stem cell antigen, complement factors C3, C3a, C3b, C5a and C5, and oncogene products.

3. The antibody-drug conjugate according to claim 1, wherein the antibody is an anti-PD-L1 antibody.

4. The antibody-drug conjugate according to claim 1, wherein the antibody comprises heavy chains and light chains;
wherein the light chain has an amino acid sequence set forth in SEQ ID NO: 9, or an amino acid sequence having at least 80%, 85%, 90%, 91%, 92%, 93%, 94%, 95%, 96%, 97%, 98%, or 99% identity to SEQ ID NO: 9;
preferably, the antibody has a light chain sequence set forth in SEQ ID NO: 9;
more preferably, the antibody has a heavy chain sequence set forth in SEQ ID NO: 4 and the light chain sequence set forth in SEQ ID NO: 9; or the antibody has a heavy chain sequence set forth in SEQ ID NO: 4 and a light chain sequence set forth in SEQ ID NO: 3.

5. The antibody-drug conjugate according to any one of claims 1 to 4, wherein n is an integer of 1 to 10, preferably an integer of 1 to 6; preferably, n = 2 or 4.

6. The antibody-drug conjugate according to claim 4, wherein in the antibody-drug conjugate, a linking site on the antibody for bonding with L is the free sulfydryl group on the cysteine residue at position 226 of the amino acid sequence set forth in SEQ ID NO: 9.

7. The antibody-drug conjugate according to any one of claims 1 to 6, wherein the moiety D in general formula I has the following structure: 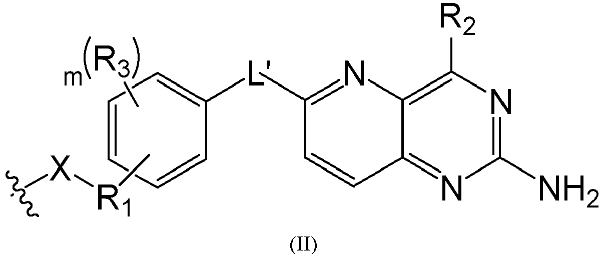 wherein,
L' is a linking group and is selected from: a single bond, C₁-C₆ alkylene, C₁-C₆ alkenylene, and C₃-C₆ cycloalkylene, wherein the groups can be optionally substituted with C₁-C₄ alkyl;
R₁ is selected from: a single bond, C₁-C₆ alkylene, C₁-C₆ alkenylene, and C₁-C₆ alkyleneoxy, wherein the groups can be optionally substituted with C₁-C₄ alkyl;
X is selected from: 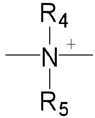 , -NR₄-, -O-, -S-, a single bond, -OCO-, -COO-, -NR₄-(C₁-C₆ alkylene)-NR₅-, and heterocyclylene, wherein R₄ and R₅ are independently selected from: H, C₁-C₆ alkyl, C₁-C₆ alkoxy, amino acid residues, oligopeptide residues, haloalkyl, carboxyl-substituted alkyl, ester-substituted alkyl, and 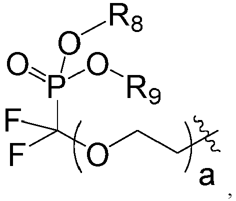 wherein a is an integer of 1 to 10, R₈ and R₉ are independently selected from: H, and C₁-C₆ alkyl, or R₈ and R₉, together
with atoms to which they are attached, form 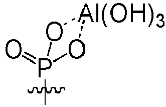 ; or R₄ and R₅, together with the nitrogen atom to which they are attached, form heterocyclyl;
R₂ is selected from: -NR₆R₇, -OR₆, and -SR₆, wherein R₆ and R₇ are independently selected from: H, C₁-C₆ alkyl, C₁-C₆ alkoxy, and C₁-C₆ alkoxyalkyl, or R₆ and R₇, together with the nitrogen atom to which they are attached, form heterocyclyl, or R₆, together with the oxygen atom to which it is attached, forms heterocyclyl, or R₆, together with the sulfur atom to which it is attached, forms heterocyclyl;
R₃ is one or more independent substituents on the phenyl ring and is selected from: H, C₁-C₆ alkyl, C₁-C₆ alkoxy, and Ci-Ce alkoxyalkyl; and
m is an integer of 0 to 4.

8. The antibody-drug conjugate according to claim 7, wherein L' has the following structure: 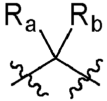 , wherein Rₐ and R_{b} are independently selected from: H, C₁-C₃ alkyl, or Rₐ and R_{b}, together with the carbon atom to which they are attached, form C₃-C₆ cycloalkylene; preferably, L' is selected from: -CH₂-,

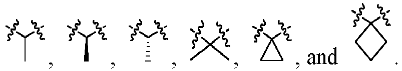

9. The antibody-drug conjugate according to claim 7, wherein R₁ is C₁-C₆ alkylene, such as C₁-C₃ alkylene, e.g., -CH₂-, or,
R₁ is a single bond; or,
R₁ is C₁-C₆ alkyleneoxy, such as, C₁-C₃ alkyleneoxy, e.g., -CH₂O-, -CH₂CH₂O-, or -CH₂CH₂CH₂O-.

10. The antibody-drug conjugate according to claim 7, wherein R₃ is one or more independent substituents on the phenyl ring and is independently selected from: H, methyl, and methoxy.

11. The antibody-drug conjugate according to claim 7, wherein m is 0 or 1.

12. The antibody-drug conjugate according to claim 7, wherein R₈ and R₉ are independently selected from: H, methyl, ethyl, n-propyl, and isopropyl.

13. The antibody-drug conjugate according to claim 7, wherein R₄ and R₅ are independently selected from: H, methyl, ethyl, *n*-propyl, isopropyl, *n*-butyl, *tert*-butyl, amino acid residues, oligopeptide residues, -CF₃, -CH₂CF₃, 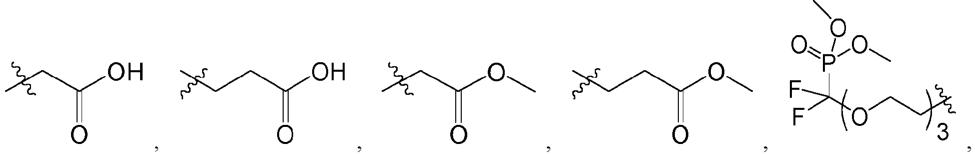 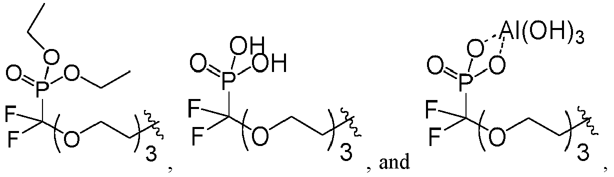 or, R₄ and R₅, together with the nitrogen atom to which they are attached, form substituted or unsubstituted heterocyclyl; preferably, the substituted or unsubstituted heterocyclyl is selected from: 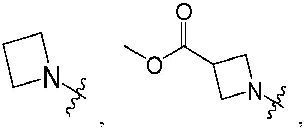 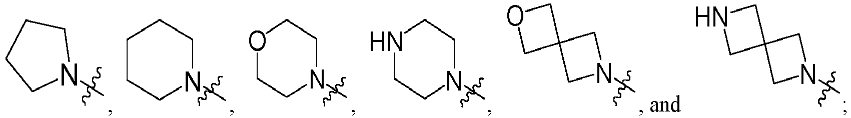
preferably, the amino acid residue is selected from: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine residues;
preferably, amino acid residues in the oligopeptide residue are independently selected from one or more of: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine residues;
more preferably, X is 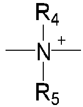 , and R₄ and R₅ are independently selected from C₁-C₆ alkyl, such as methyl;
more preferably, X is -NR₄-, and R₄ is selected from: H, and C₁-C₆ alkyl, for example, R₄ is H or methyl;
more preferably, X is -NR₄-(C₁-C₆ alkylene)-NR₅-, and R₄ and R₅ are independently selected from C₁-C₆ alkyl, for example, R₄ and R₅ are both methyl;
more preferably, X is heterocyclylene, in particular nitrogen-containing heterocyclylene, e.g., 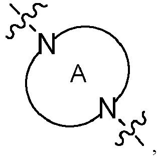 ring A is a 4- to 10-membered nitrogen-containing heterocyclic ring, and ring A can be monocyclic, bicyclic, or tricyclic (including fused, spiro, bridged); further preferably, X is 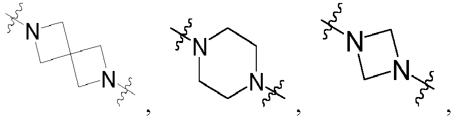 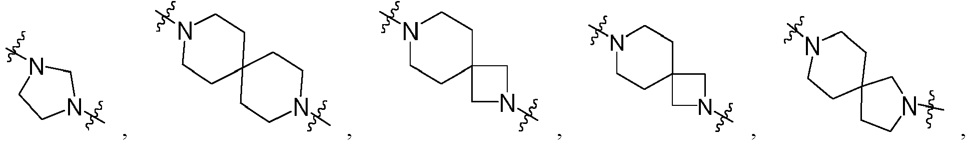 or

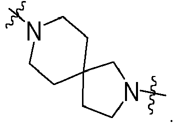

14. The antibody-drug conjugate according to claim 7, wherein R₂ is selected from: -NHR₆, -OR₆, and -SR₆, wherein R₆ is C₁-C₆ alkyl or C₁-C₆ alkoxyalkyl; or,
R₂ is -NR₆R₇, wherein R₆ and R₇, together with the nitrogen atom to which they are attached, form heterocyclyl;
preferably, the heterocyclyl is selected from:

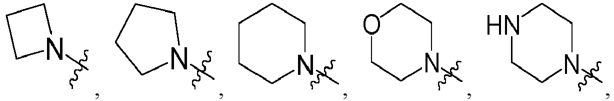

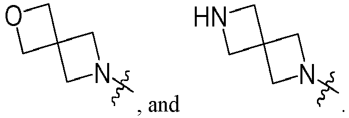

15. The antibody-drug conjugate according to claim 7, wherein R₂ is selected from:

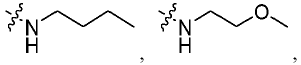

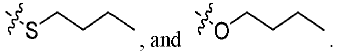

16. The antibody-drug conjugate according to claim 7, wherein the moiety D in general formula I is selected from the following structures:

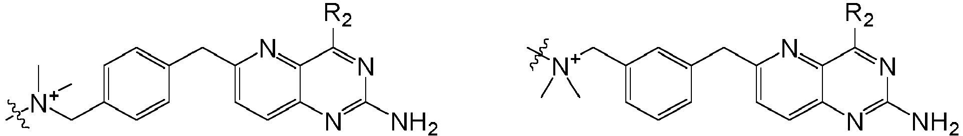

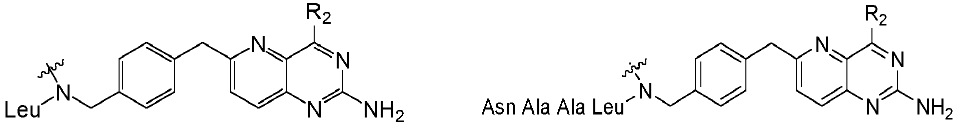

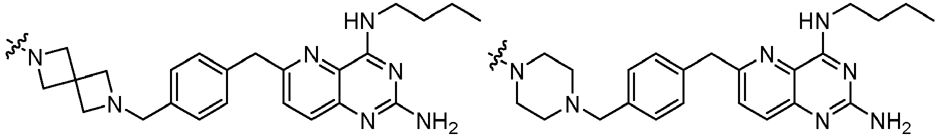

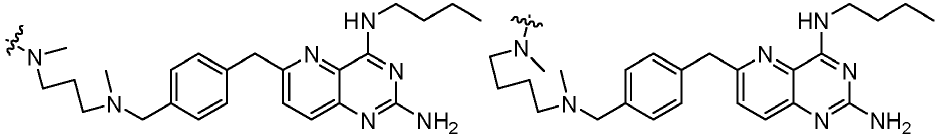

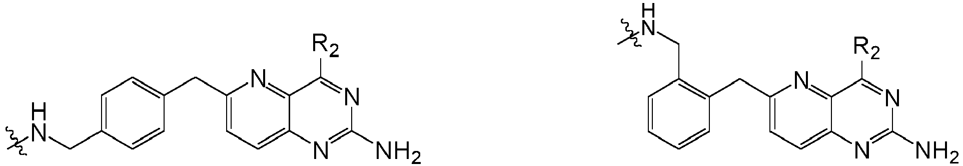

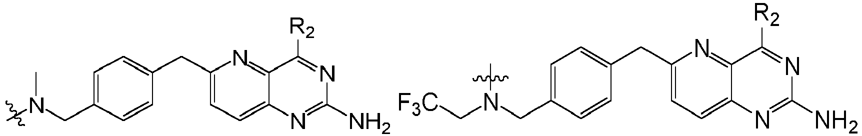

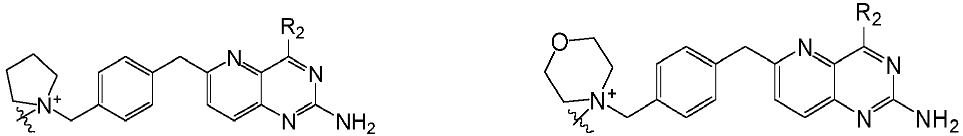

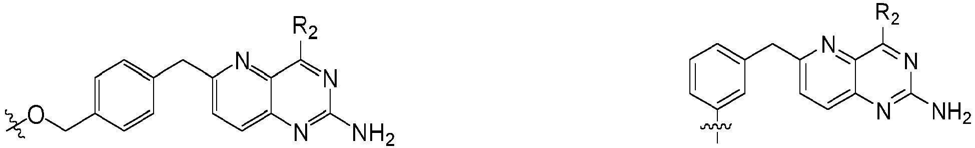

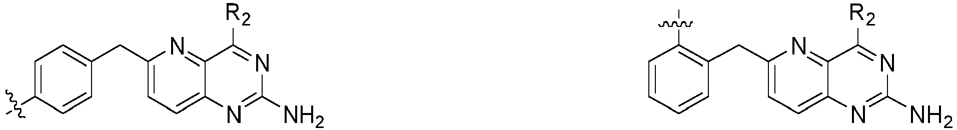

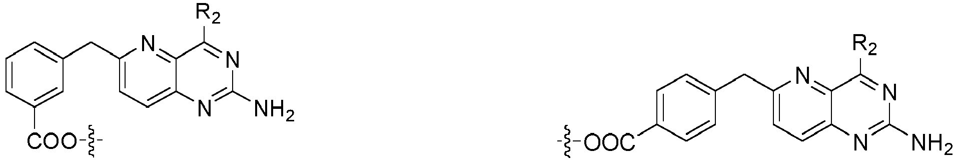

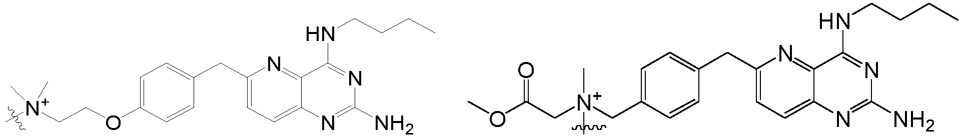

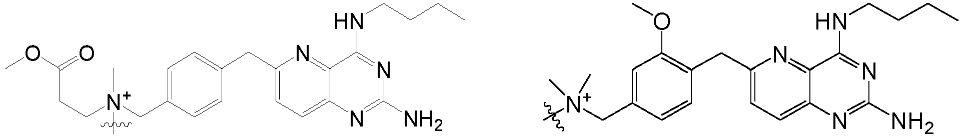

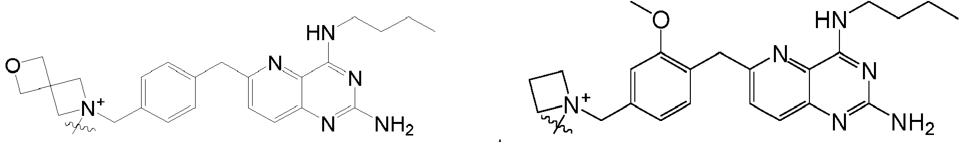

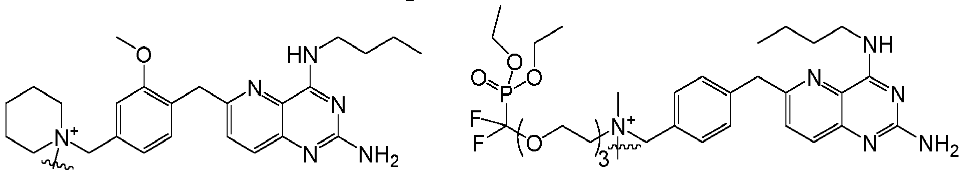

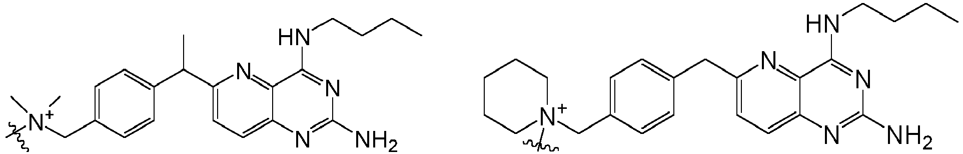

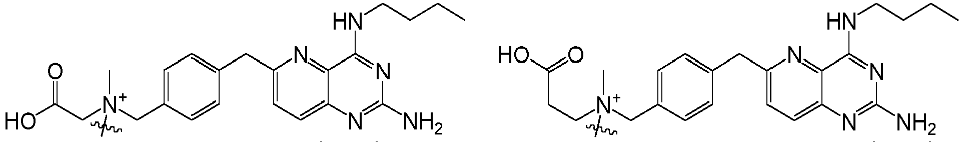

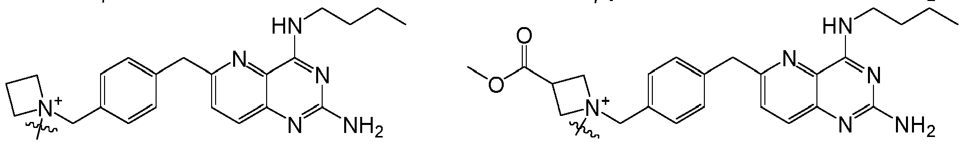

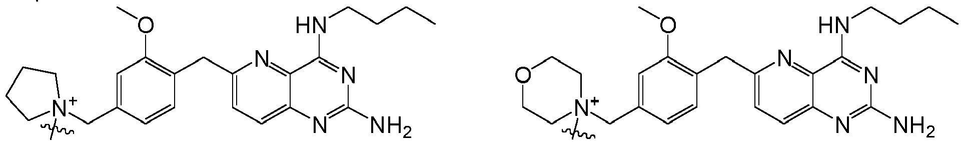

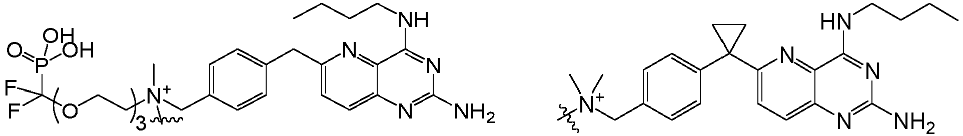

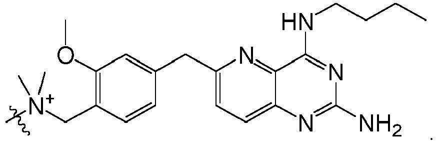

17. The antibody-drug conjugate according to claim 7, wherein the moiety D in general formula I has the following structure:

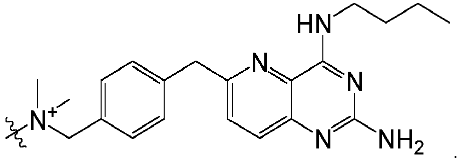

18. The antibody-drug conjugate according to any one of claims 1 to 17, wherein L comprises a functional group Y for bonding with an active group of Ab, such as a single bond, 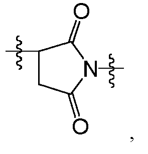 -S-, -CO-, -NH-, -CONH-, 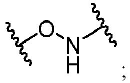
preferably, L further comprises a group Q, Q being a divalent saturated or unsaturated, linear or branched C1-50 hydrocarbon chain, with 0-6 methylene units independently substituted with: -Cy-, -O-, -NR₁₀-, -S-, -OC(O)-, -C(O)O-, -C(O)-, -S(O)-, -S(O)₂-, -NR₁₀S(O)₂-, -S(O)₂-NR₁₀-, -NR₁₀-C(O)-, -C(O)NR₁₀-, -OC(O)NR₁₀-, -NR₁₀-C(O)O-, 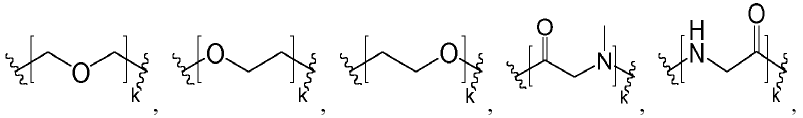 amino acid residue, and
oligopeptide residue, wherein k is selected from integers of 1 to 10, and each -Cy- is independently an optionally-substituted divalent ring selected from: arylene, cycloalkylene, and heterocyclylene; R₁₀ is selected from: H, -OH, C₁-C₆ alkyl, C₃-C₆ cycloalkyl, and heterocycloalkyl;
preferably, each -Cy- can be independently selected from: 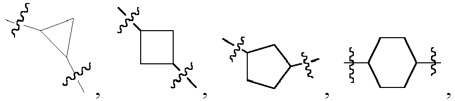 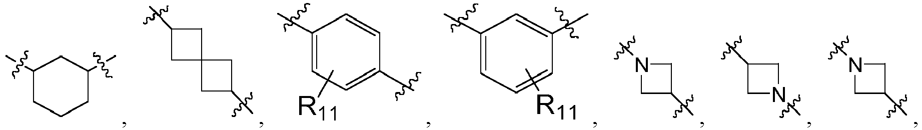 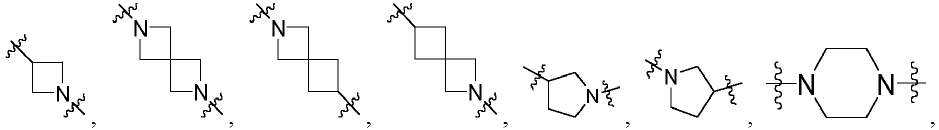 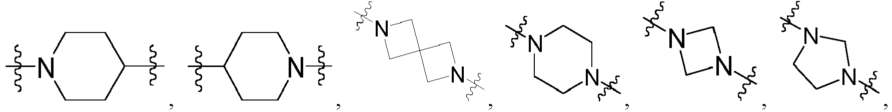 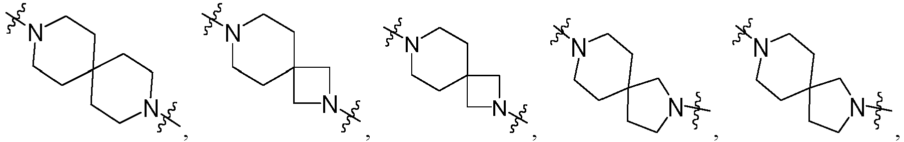 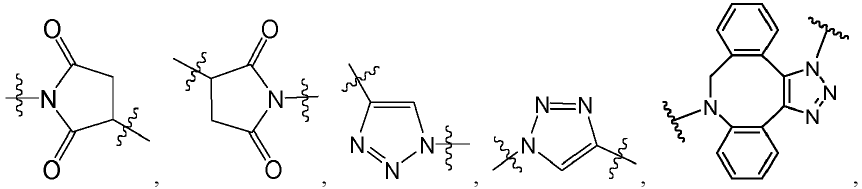 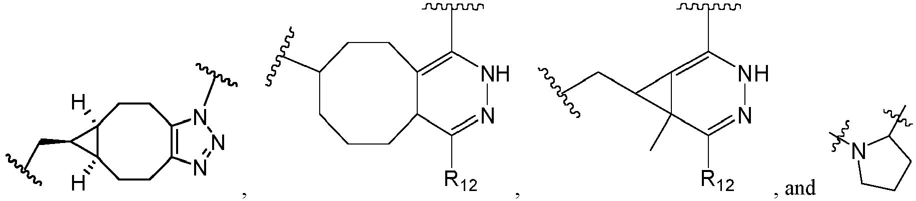 , wherein
R₁₁ is one or more independent substituents on the ring and is selected from: H, halogen, -CN, -NO₂, -CF₃, -OCF₃, - NH₂, -OH, C₁-C₆ alkyl, -O(C₁-C₆ alkyl), -NH(C₁-C₆ alkyl), -N(C₁-C₆ alkyl)(C₁-C₆ alkyl), and a residue of a monosaccharide or a derivative thereof, and R₁₂ is selected from: H, substituted or unsubstituted alkyl, substituted or unsubstituted aryl, substituted or unsubstituted aralkyl, substituted or unsubstituted cycloalkyl, substituted or unsubstituted cycloalkylalkyl, substituted or unsubstituted heterocyclyl, and substituted or unsubstituted heterocyclylalkyl;
more preferably, each -Cy- is independently selected from:

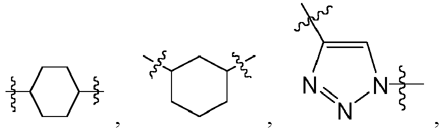

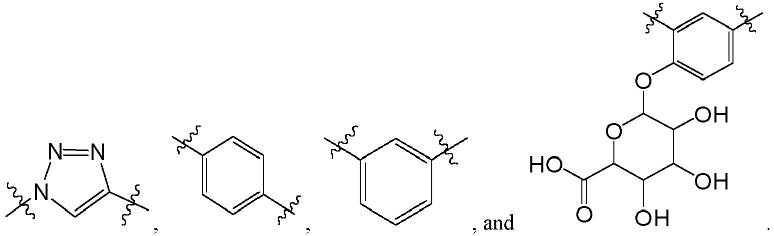

19. The antibody-drug conjugate according to any one of claims 1 to 17, wherein the moiety L in general formula I has the following structure: 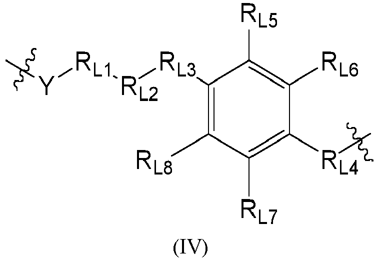 wherein,
Y is a functional group for bonding with an active group of Ab;
R_{L1}, R_{L3}, and R_{L4} are independently selected from one or a combination of two or more of: 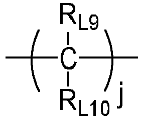 , -(CH₂)ⱼO-, -(CH₂)ⱼN(R_{L9})-, -(CH₂)ⱼCO-, -(CH₂)ⱼOCOO-, -(CH₂)ⱼOCON(R_{L9})-, -(CH₂)ⱼN(R_{L9})CON(R_{L10})-, -(CH₂)ⱼN(R_{L9})CO-, - O(CH₂)ⱼCOO-, -(CH₂)ⱼCOO-, -(CH₂)ⱼCON(R_{L9})-, -(CH₂)ⱼS-S-, -(CH₂)ⱼN(R_{L9})-NH=, 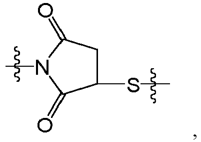 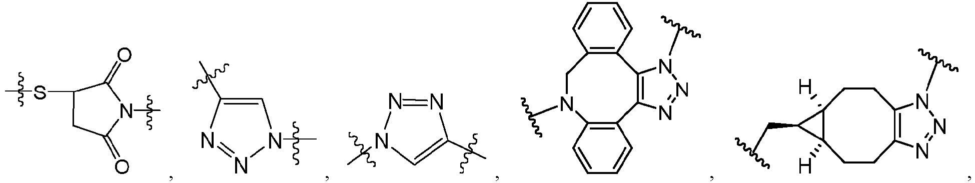 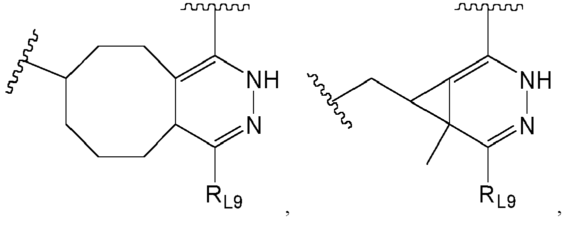 , cycloalkyl, and aryl, wherein j is an integer of 0 to 10;
R_{L2} is selected from: a single bond, -(CH₂)ᵢQCO-, -(CH₂)ᵢQCOO-, -(CH₂)ᵢNH-COO-, amino acid residue, and oligopeptide residue, wherein i is an integer of 0 to 10;
R_{L5}, R_{L6}, R_{L7}, and R_{L8} are independently selected from: H, substituted or unsubstituted alkyl, halogen, nitro, cyano, - OR_{L9}, -NR_{L9}R_{L10}, -S(O)ₜR_{L9}, -C(O)OR_{L9}, -C(O)R_{L9}, and -C(O)NR_{L9}R_{L10}, wherein t is 0, 1, or 2; and
each of R_{L9} and R_{L10} is independently selected from: H, substituted or unsubstituted alkyl, substituted or unsubstituted aryl, substituted or unsubstituted aralkyl, substituted or unsubstituted cycloalkyl, substituted or unsubstituted cycloalkylalkyl, substituted or unsubstituted heterocyclyl, and substituted or unsubstituted heterocyclylalkyl.

20. The antibody-drug conjugate according to claim 19, wherein the moiety L has the following structure:

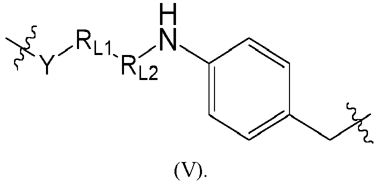

21. The antibody-drug conjugate according to claim 19 or 20, wherein Y is selected from: a single bond, 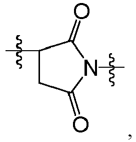 -S-, -CO-, -NH-, -CONH-, and

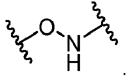

22. The antibody-drug conjugate according to claim 19 or 20, wherein R_{L1} is 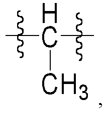 or R_{L1} is -(CH₂)ⱼCO-, wherein j is an integer of 0 to 6.

23. The antibody-drug conjugate according to claim 19 or 20, wherein R_{L2} is -(CH₂)ᵢOCO-, wherein i is an integer of 0 to 6, or R_{L2} is an oligopeptide residue, and the oligopeptide residue is selected from: avaline-citrulline residue, an alanine-alanine-asparagine residue, an aspartic acid-valine residue, and a glutamic acid-valine residue.

24. The antibody-drug conjugate according to claim 23, wherein the moiety L has the following structure:

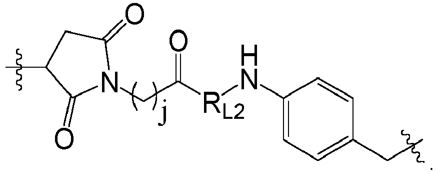

25. The antibody-dmg conjugate according to any one of claims 1 to 17, wherein the moiety L has the following structure:

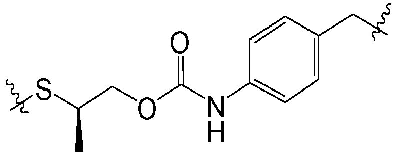

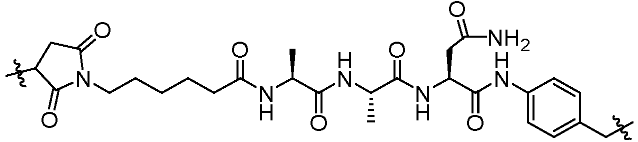

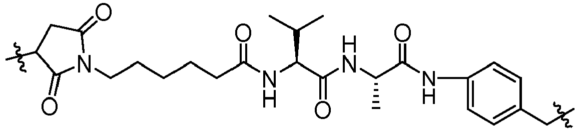

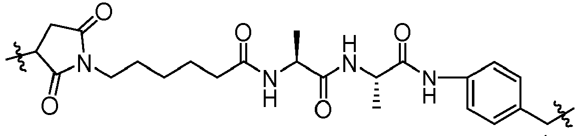

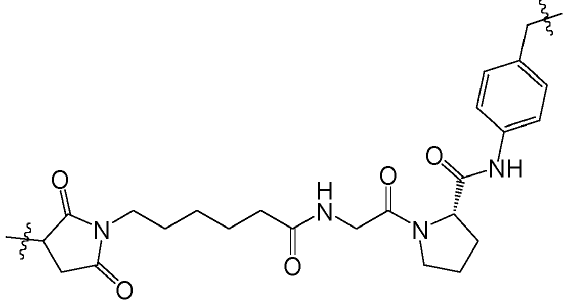

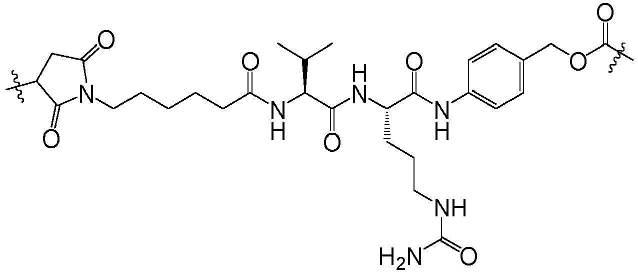

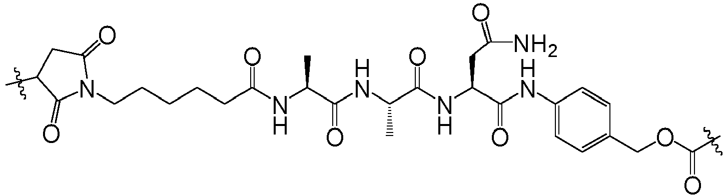

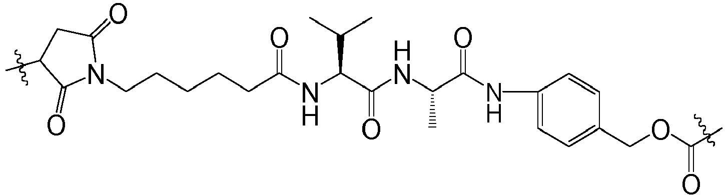

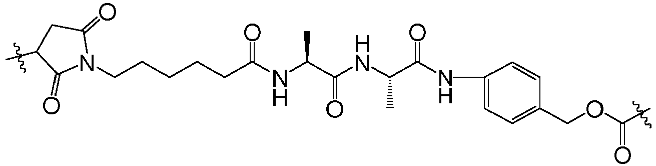

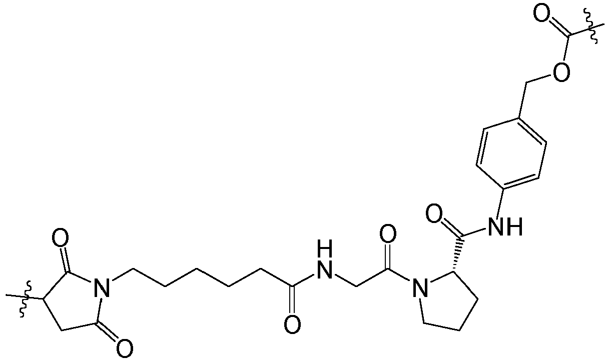

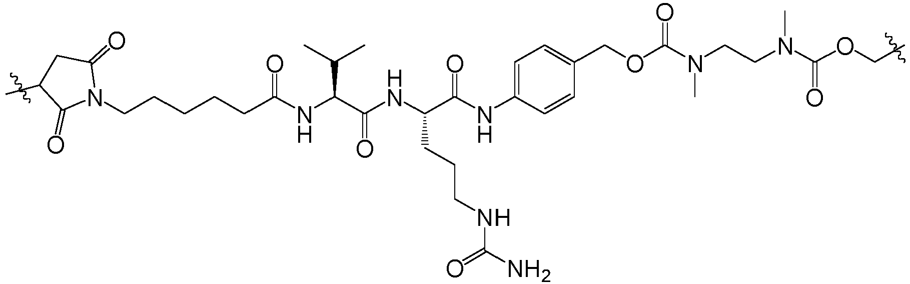

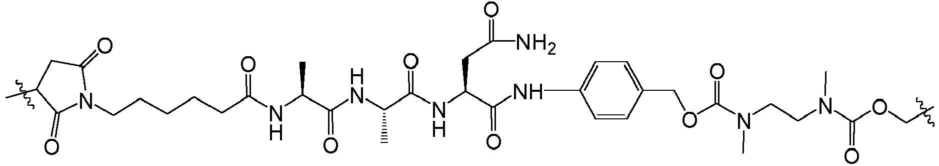

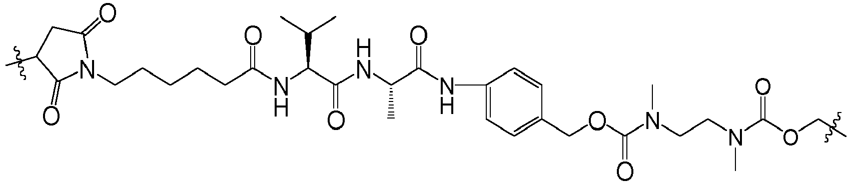

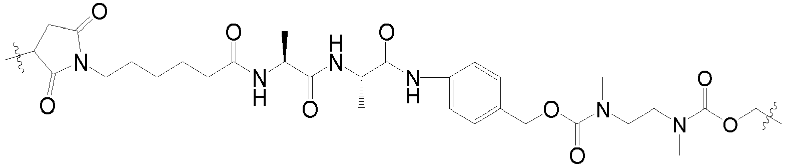

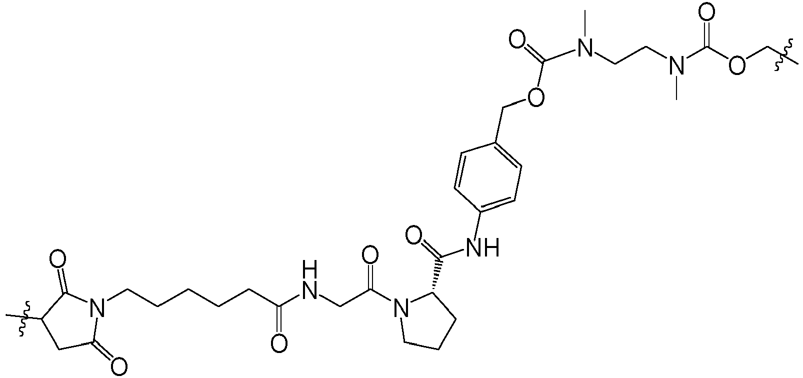

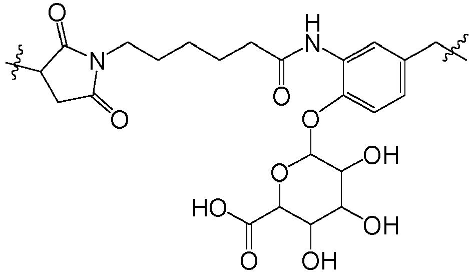

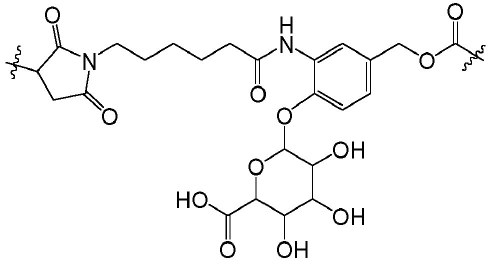

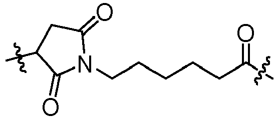

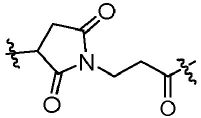

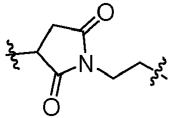

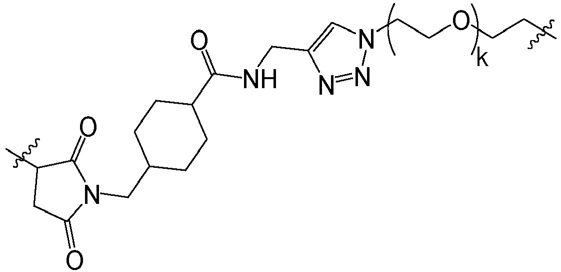

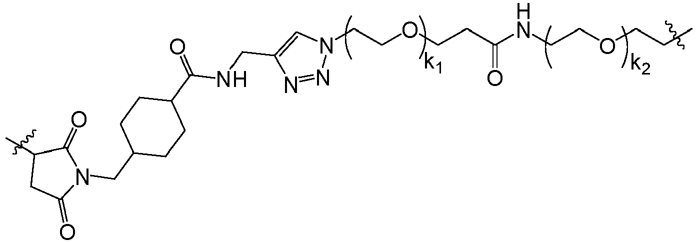

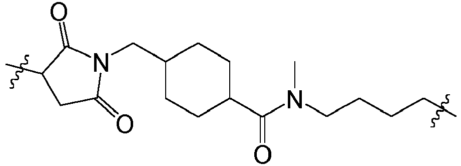

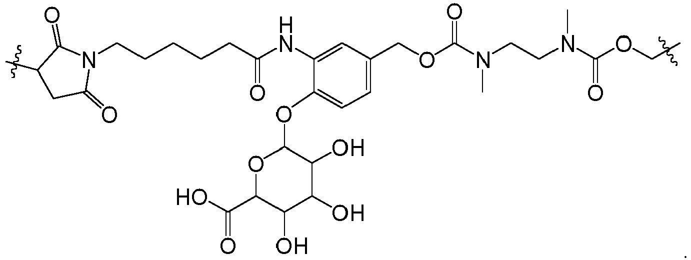

26. The antibody-drug conjugate according to any one of claims 1 to 6, wherein the antibody-drug conjugate has the following structure: 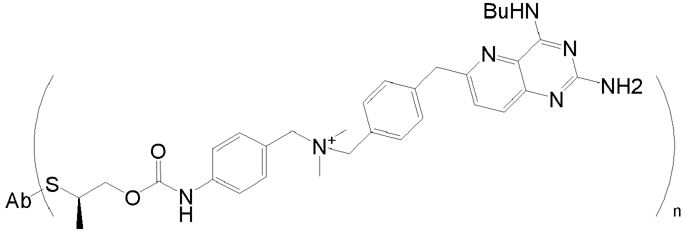 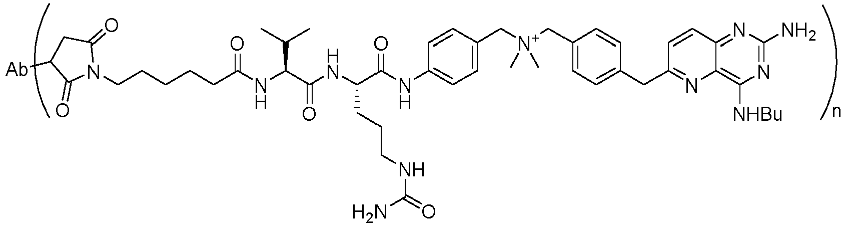 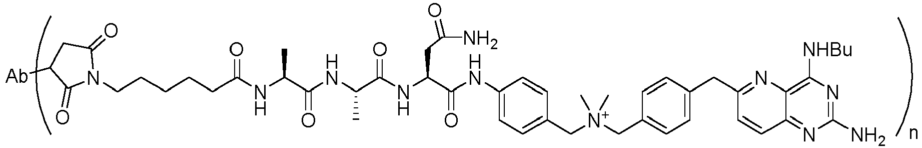 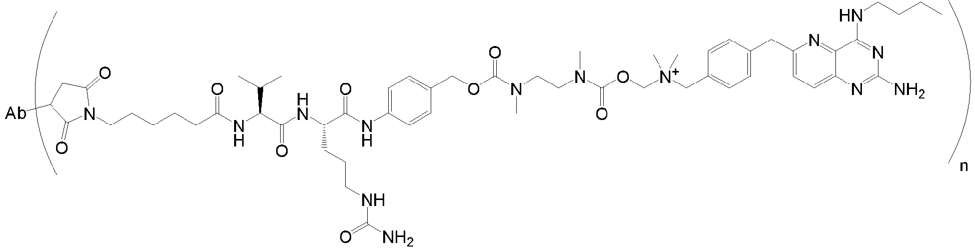 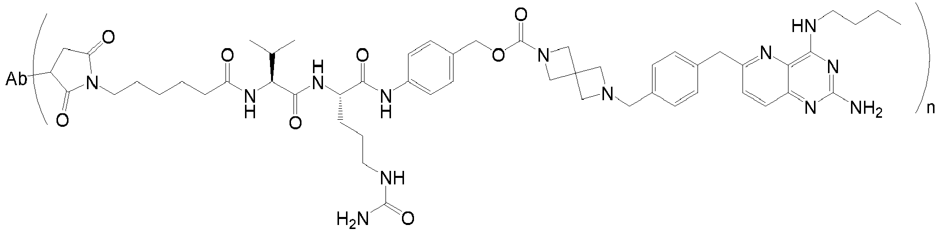 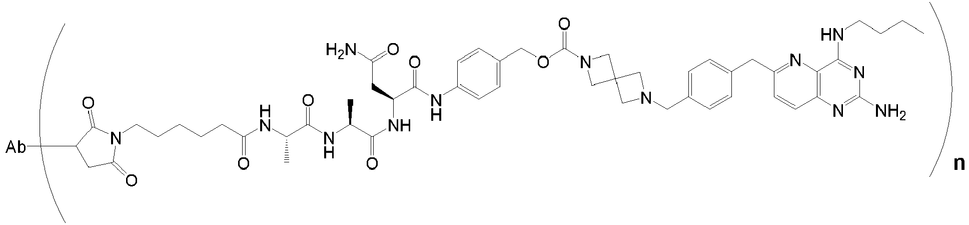 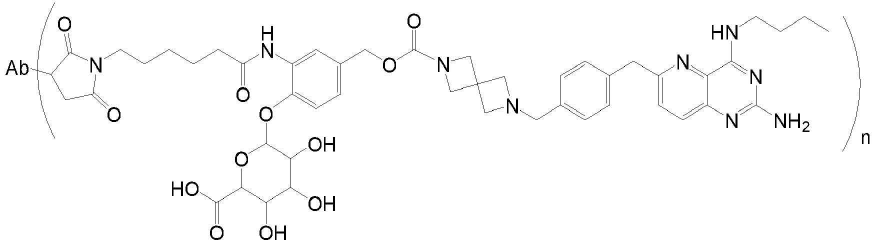 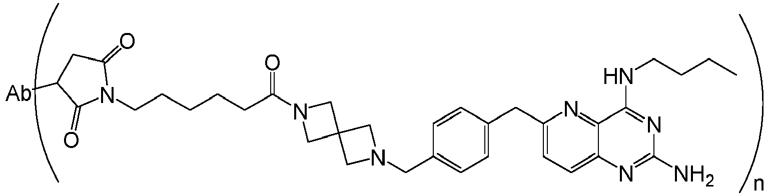 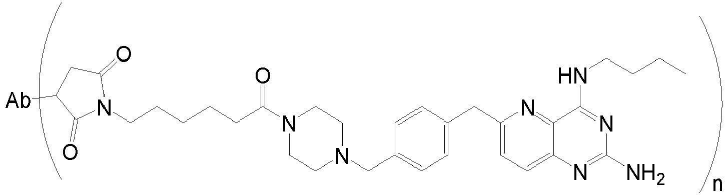 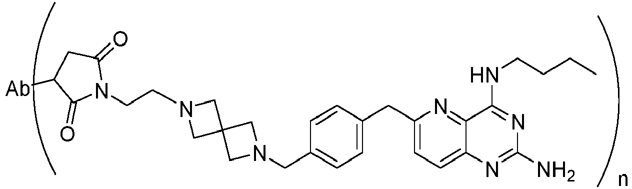 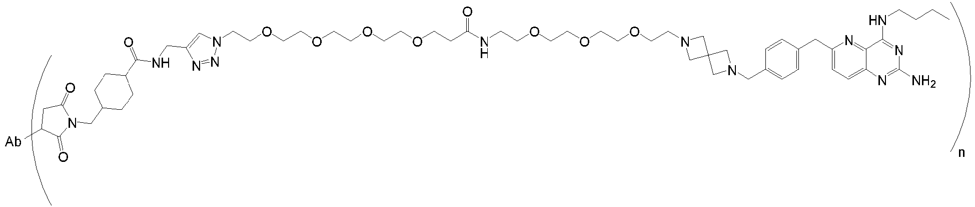 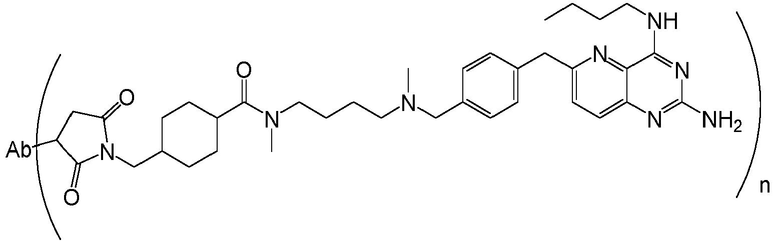 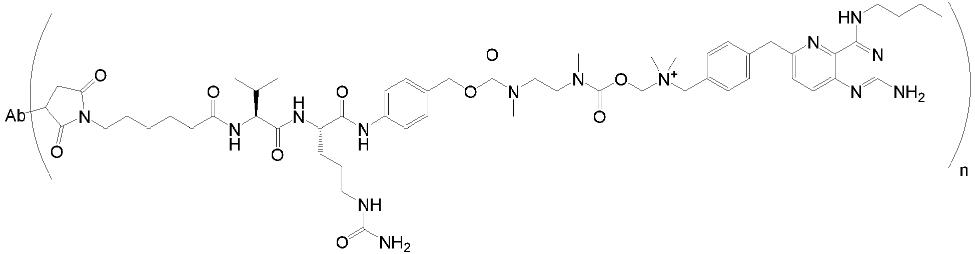 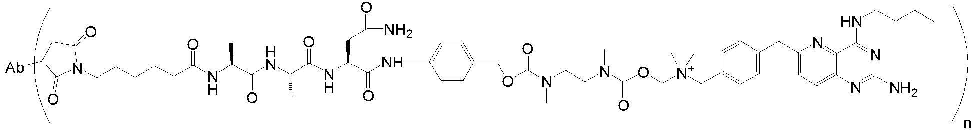 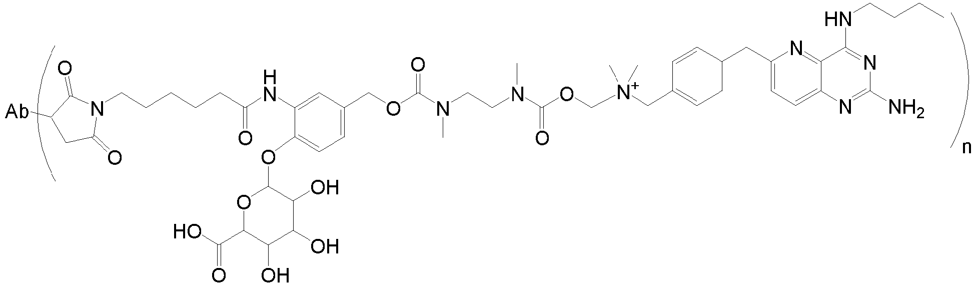 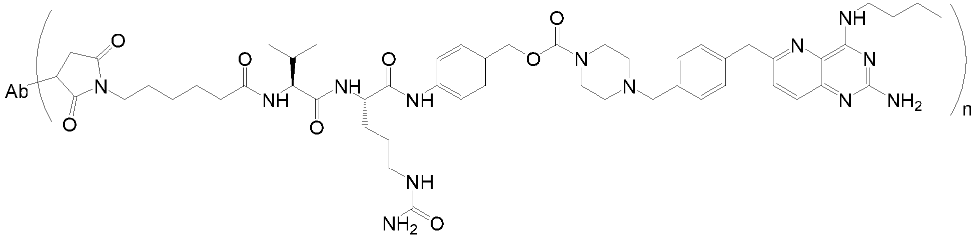 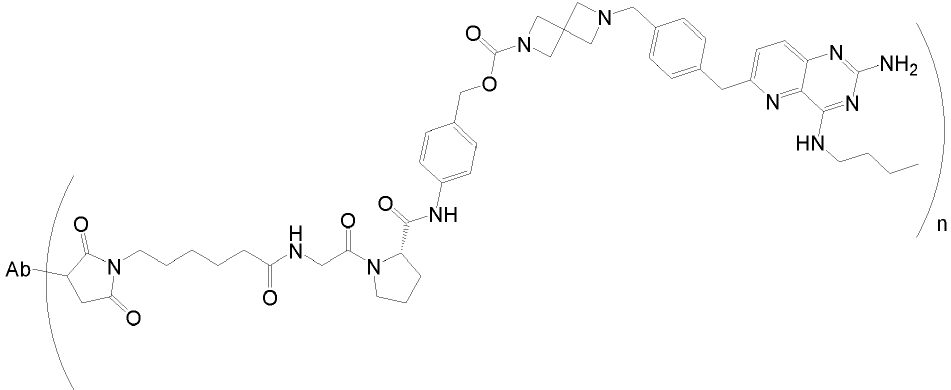 and

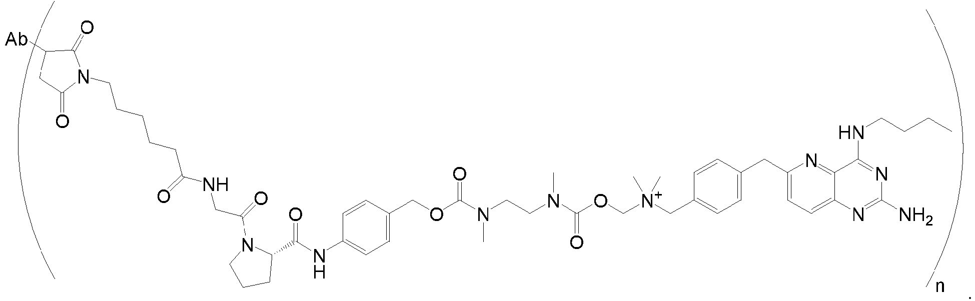

27. A preparation method for the antibody-drug conjugate according to any one of claims 1 to 26, comprising a step of coupling a conjugate of a small molecule drug and a linking unit with an antibody.

28. A modified anti-PD-L1 antibody, comprising heavy chains and light chains,
wherein the light chain has an amino acid sequence set forth in SEQ ID NO: 9, or an amino acid sequence having at least 80%, 85%, 90%, 91%, 92%, 93%, 94%, 95%, 96%, 97%, 98%, or 99% identity to SEQ ID NO: 9;
preferably, the antibody has a light chain sequence set forth in SEQ ID NO: 9;
more preferably, the antibody has a heavy chain sequence set forth in SEQ ID NO: 4 and a light chain sequence set forth in SEQ ID NO: 9.

29. A compound, or a pharmaceutically acceptable salt, a solvate, a prodrug, or a stereoisomer thereof, having the following structure: 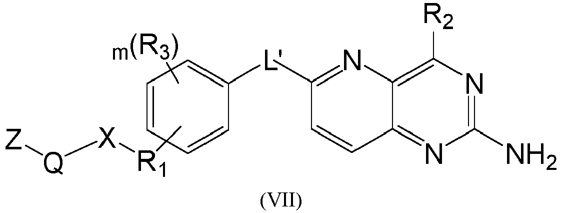 wherein,
Z is an active group;
L' is a linking group and is selected from: a single bond, C₁-C₆ alkylene, C₁-C₆ alkenylene, and C₃-C₆ cycloalkylene, wherein the groups can be optionally substituted with C₁-C₄ alkyl;
R₁ is selected from: a single bond, C₁-C₆ alkylene, C₁-C₆ alkenylene, and C₁-C₆ alkyleneoxy, wherein the groups can be optionally substituted with C₁-C₄ alkyl;
X is selected from: 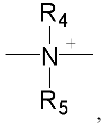 -NR₄-, -O-, -S-, a single bond, -OCO-, and -COO-, wherein R₄ and R₅ are independently selected from: H, Ci-Ce alkyl, C₁-C₆ alkoxy, amino acid residues, oligopeptide residues, haloalkyl, carboxyl-substituted
alkyl, ester-substituted alkyl, and 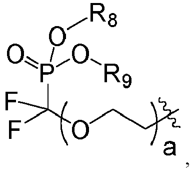 , wherein a is an integer of 1 to 10, R₈ and R₉ are independently selected from: H, and C₁-C₆ alkyl, or R₈ and R₉, together with atoms to which they are attached, form 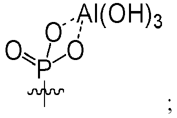 or, R₄ and R₅, together with the nitrogen atom to which they are attached, form heterocyclyl;
R₂ is selected from: -NR₆R₇, -OR₆, and -SR₆, wherein R₆ and R₇ are independently selected from: H, C₁-C₆ alkyl, C₁-C₆ alkoxy, and C₁-C₆ alkoxyalkyl, or R₆ and R₇, together with the nitrogen atom to which they are attached, form heterocyclyl, or R₆, together with the oxygen atom to which it is attached, forms heterocyclyl, or R₆, together with the sulfur atom to which it is attached, forms heterocyclyl;
R₃ is one or more independent substituents on the phenyl ring and is selected from: H, Ci-Ce alkyl, C₁-C₆ alkoxy, and C₁-C₆ alkoxyalkyl;
m is an integer of 0 to 4;
Q is a divalent saturated or unsaturated, linear or branched C1-50 hydrocarbon chain with 0-6 methylene units independently substituted with: -Cy-, -O-, -NR₁₀-, -S-, -OC(O)-, -C(O)O-, -C(O)-, -S(O)-, -S(O)₂-, -NR₁₀S(O)₂-, -S(O)₂-NR₁₀-, -NR₁₀-C(O)-, -C(O)NR₁₀-, -OC(O)NR₁₀-, -NR₁₀-C(O)O-, 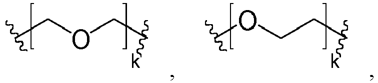 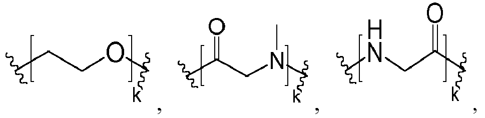 amino acid residue, and oligopeptide residue, wherein k is selected from integers of 1 to 10, and each -Cy- is independently an optionally-substituted divalent ring selected from: arylene, cycloalkylene, heterocyclylene; R₁₀ is selected from: H, -OH, C₁-C₆ alkyl, C₃-C₆ cycloalkyl, and heterocycloalkyl;
preferably, each -Cy- can be independently selected from: 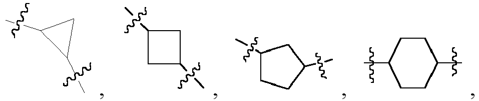 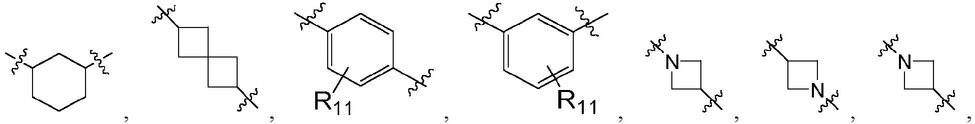 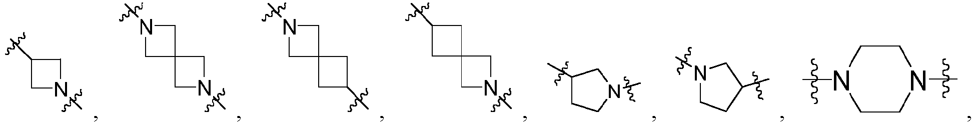 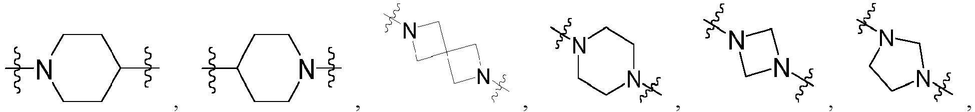 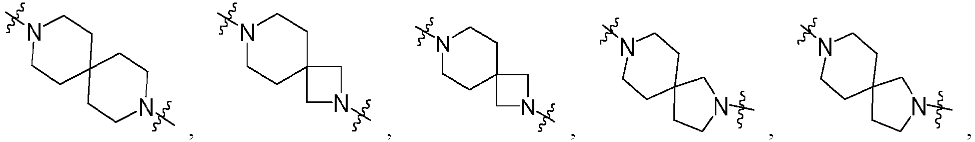 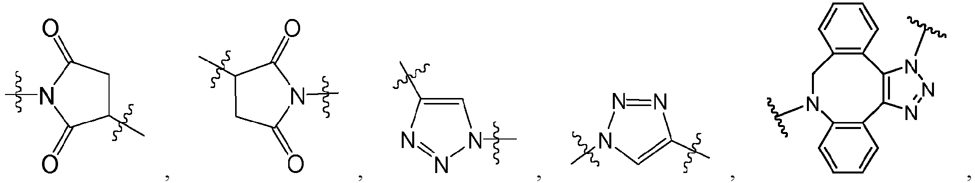 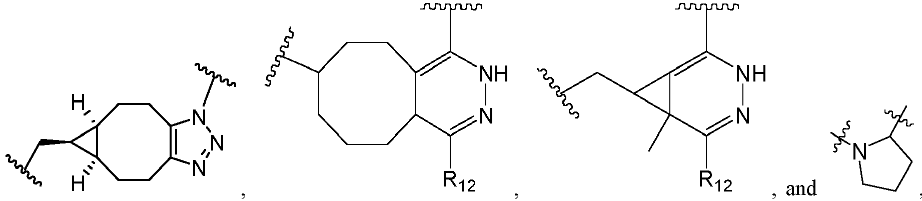 , wherein
R₁₁ is one or more independent substituents on the ring and is selected from: H, halogen, -CN, -NOz, -CF₃, -OCF₃, -NH₂, -OH, C₁-C₆ alkyl, -O(C₁-C₆ alkyl), -NH(C₁-C₆alkyl), -N(C₁-C₆ alkyl)(C₁-C₆ alkyl), and a residue of a monosaccharide or a derivative thereof, and R₁₂ is selected from: H, substituted or unsubstituted alkyl, substituted or unsubstituted aryl, substituted or unsubstituted aralkyl, substituted or unsubstituted cycloalkyl, substituted or unsubstituted cycloalkylalkyl, substituted or unsubstituted heterocyclyl, and substituted or unsubstituted heterocyclylalkyl;
more preferably, each -Cy- is independently selected from:

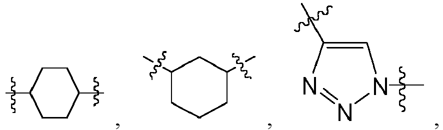

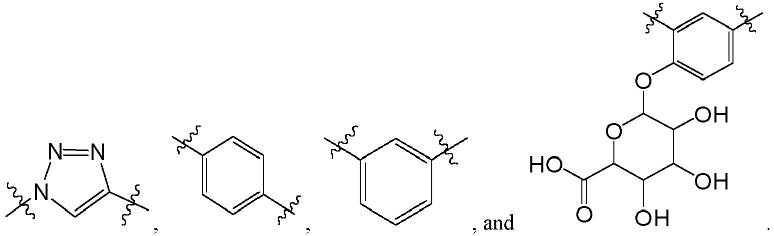

30. The compound according to claim 29, wherein the compound has the following structure: 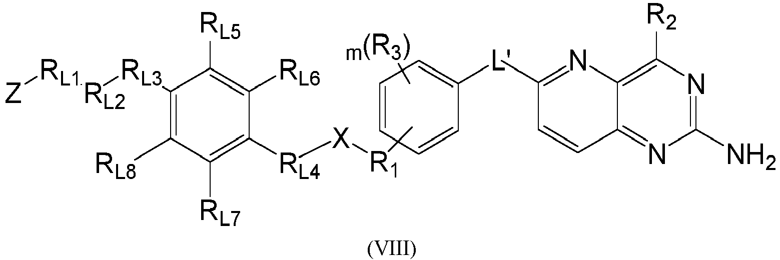 wherein,
R_{L1}, R_{L3}, and R_{L4} are independently selected from one or a combination of two or more of: 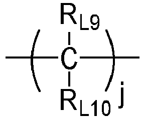 , -(CH₂)ⱼO-, -(CH₂)ⱼN(R_{L9})-, -(CH₂)ⱼCO-, -(CH₂)ⱼOCOO-, -(CH₂)ⱼOCON(R_{L9})-, -(CH₂)ⱼN(R_{L9})CON(R_{L10})-, -(CH₂)ⱼN(R_{L9})CO-, - O(CH₂)ⱼCOO-, -(CH₂)ⱼCOO-, -(CH₂)ⱼCON(R_{L9})-, -(CH₂)ⱼS-S-, -(CH₂)ⱼN(R_{L9})-NH=, 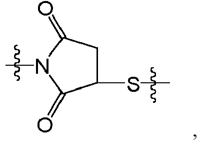 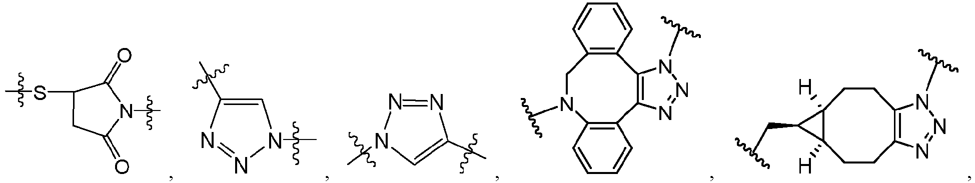 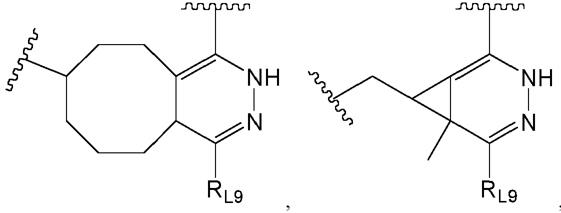 cycloalkyl, and aryl, wherein j is an integer of 0 to 10;
R_{L2} is selected from: a single bond, -(CH₂)ᵢOCO-, -(CH₂)ᵢOCOO-, (CH₂)ᵢNH-COO-, amino acid residues, and oligopeptide residues, wherein i is an integer of 0 to 10;
R_{L5}, R_{L6}, R_{L7}, and R_{L8} are independently selected from: H, substituted or unsubstituted alkyl, halogen, nitro, cyano, - OR_{L9}, -NR_{L9}R_{L10}, -S(O)ₜR_{L9}, -C(O)OR_{L9}, -C(O)R_{L9}, and -C(O)NR_{L9}R_{L10}, wherein t is 0, 1, or 2; and
each of R_{L9} and R_{L10} is independently selected from: H, substituted or unsubstituted alkyl, substituted or unsubstituted aryl, substituted or unsubstituted aralkyl, substituted or unsubstituted cycloalkyl, substituted or unsubstituted cycloalkylalkyl, substituted or unsubstituted heterocyclyl, and substituted or unsubstituted heterocyclylalkyl.

31. The compound according to claim 30, wherein the compound has the following structure: 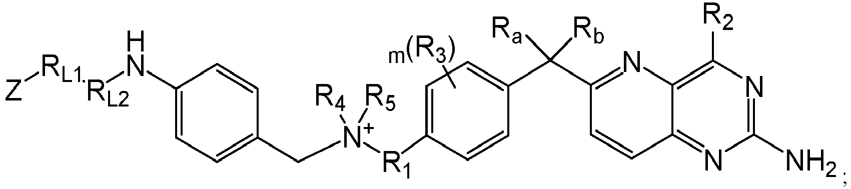 wherein Rₐ and R_{b} are independently selected from: H, C₁-C₃ alkyl, or Rₐ and R_{b}, together with the carbon atom to which they are attached, form C₃-C₆ cycloalkylene.

32. The compound according to claim 29, wherein L' is selected from: -CH₂-, 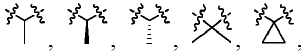 and

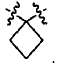

33. The compound according to claim 29 or 30, wherein R₁ is C₁-C₆ alkylene, such as C₁-C₃ alkylene, e.g., -CH₂-; or,
R₁ is a single bond; or,
R₁ is Ci-Ce alkyleneoxy, such as, C₁-C₃ alkyleneoxy, e.g., -CH₂O-, -CH₂CH₂O-, or -CH₂CH₂CH₂O-.

34. The compound according to claim 29 or 30, wherein R₃ is one or more independent substituents on the phenyl ring and is selected from: H, methyl, and methoxy.

35. The compound according to claim 29 or claim 30, wherein m is 0 or 1.

36. The compound according to claim 29 or 30, wherein R₈ and R₉ are independently selected from: H, methyl, ethyl, n-propyl, and isopropyl.

37. The compound according to claim 29 or 30, wherein R₄ and R₅ are independently selected from: H, methyl, ethyl, n-propyl, isopropyl, n-butyl, tert-butyl, amino acid residues, oligopeptide residues, -CF₃, -CH₂CF₃, 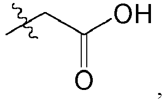 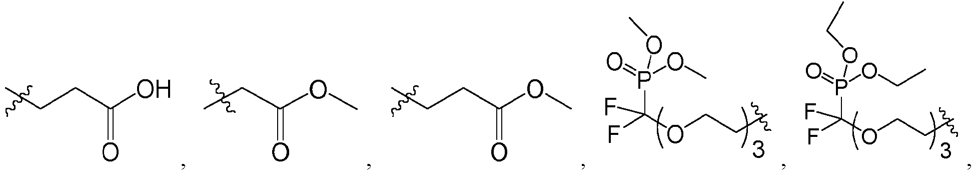 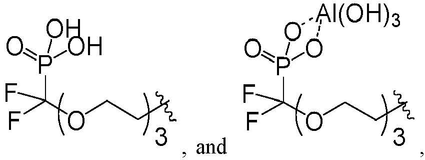 or, R₄ and R₅, together with the nitrogen atom to which they are attached, form substituted or unsubstituted heterocyclyl;
preferably, the substituted or unsubstituted heterocyclyl is selected from: 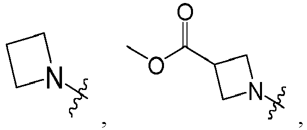 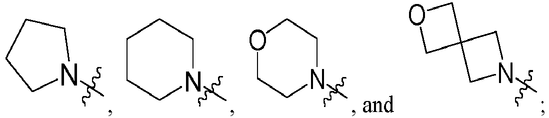
preferably, the amino acid residue is selected from: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine residues;
preferably, amino acid residues in the oligopeptide residues are independently selected from one or more of: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine residues.

38. The compound according to claim 29 or 30, wherein R₂ is selected from: -NHR₆, -OR₆, and -SR₆, wherein R₆ is C₁-C₆ alkyl or C₁-C₆ alkoxyalkyl; or,
R₂ is -NR₆R₇, wherein R₆ and R₇, together with the nitrogen atom to which they are attached, form heterocyclyl;
preferably, the heterocyclyl is selected from:

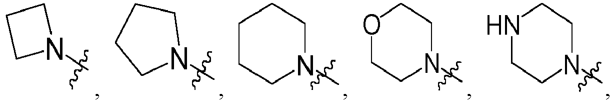

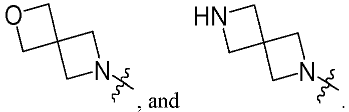

39. The compound according to claim 29 or 30, wherein R₂ is selected from:

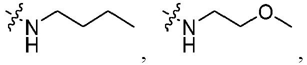

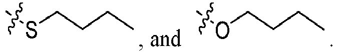

40. The compound according to claim 30, wherein R_{L1} is 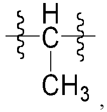 or R_{L1} is -(CH₂)ⱼCO-, wherein j is an integer of 0 to 6.

41. The compound according to claim 30, wherein R_{L2} is -(CH₂)ᵢOCO-, wherein i is an integer of 0 to 6, or R_{L2} is an oligopeptide residue, and the oligopeptide residue is selected from: a valine-citrulline residue, an alanine-alanine-asparagine residue, an aspartic acid-valine residue, and a glutamic acid-valine residue.

42. The compound according to claim 30, wherein R_{L3} is -(CH₂)ⱼNH-, wherein j is an integer of 0 to 6.

43. The compound according to claim 30, wherein R_{L4} is -(CH₂)ⱼ-, wherein j is an integer of 0 to 5.

44. The compound according to any one of claims 29 to 43, wherein Z is selected from: maleimido, carboxyl, amido, halogen, disulfide, ester group, acyl halide group, acid anhydride, epoxy, and hydroxyl; preferably Z is

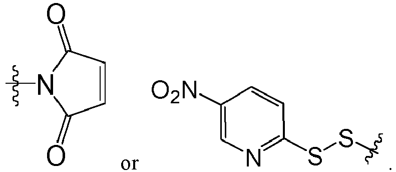

45. The compound according to claim 29, wherein the compound has the following structure: 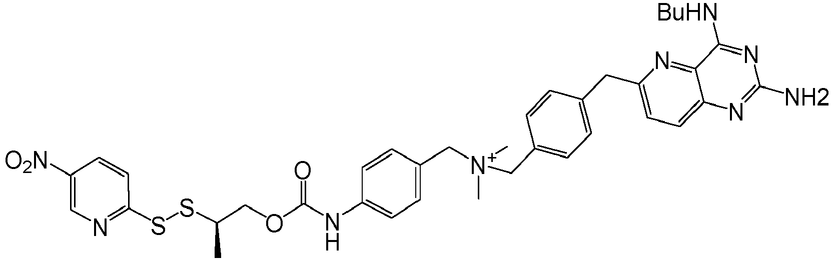 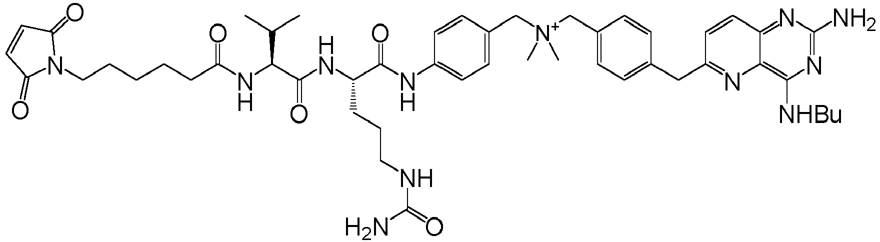 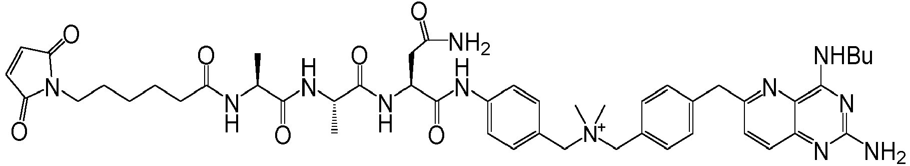 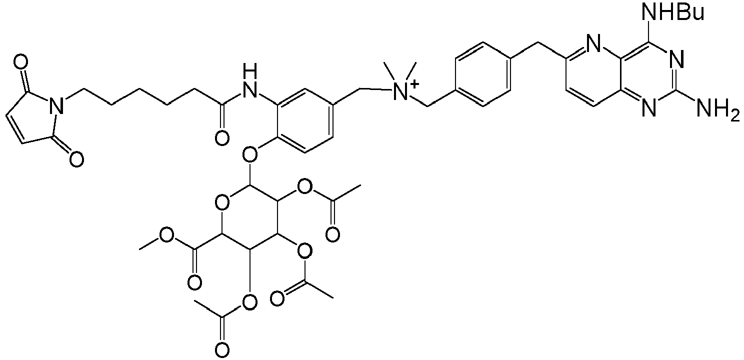 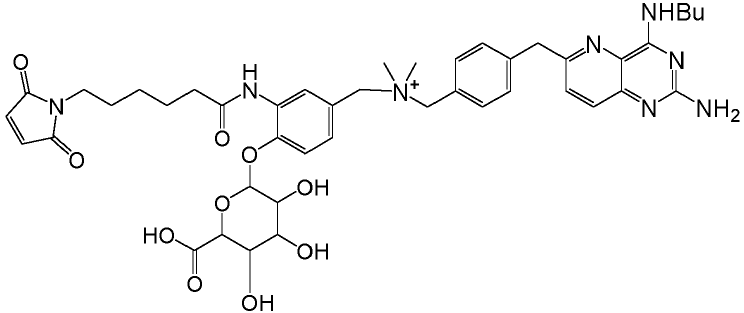 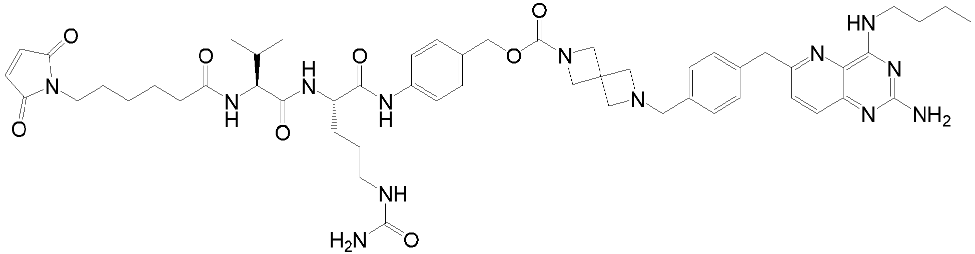 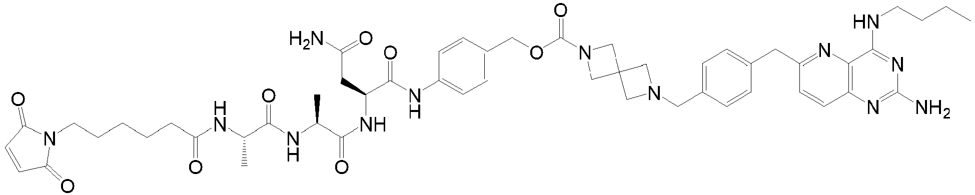 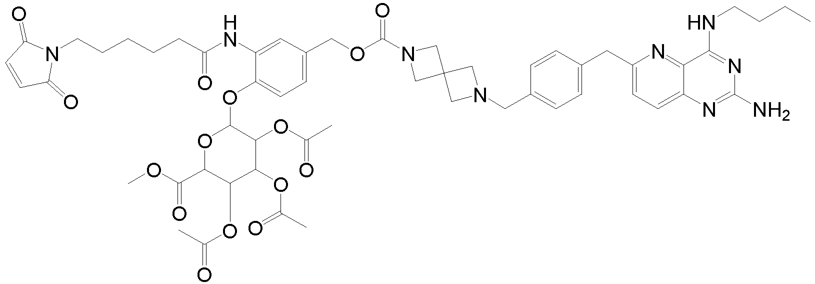 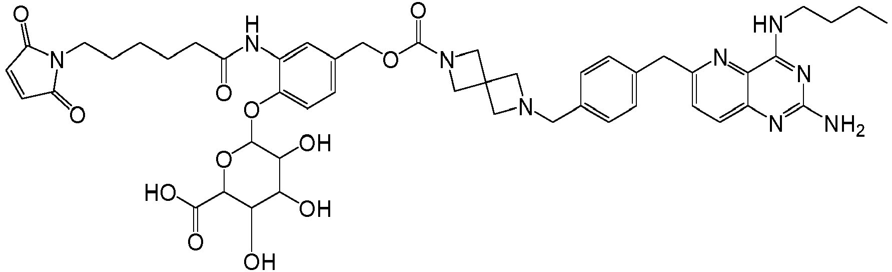 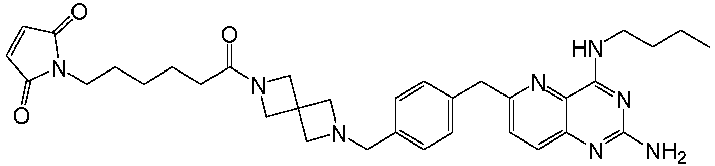 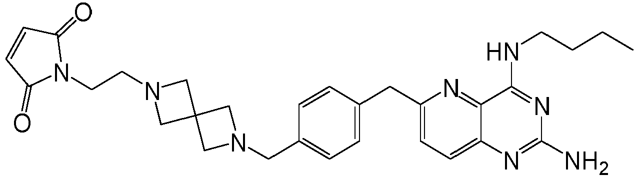 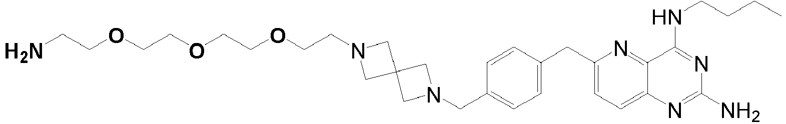 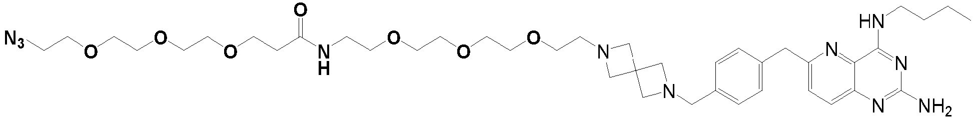 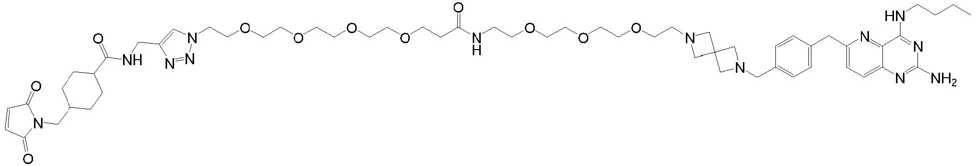 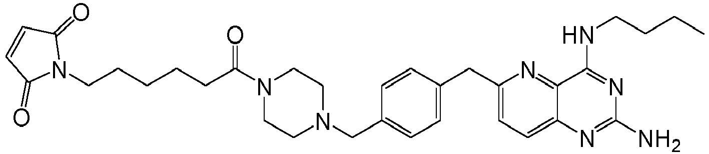 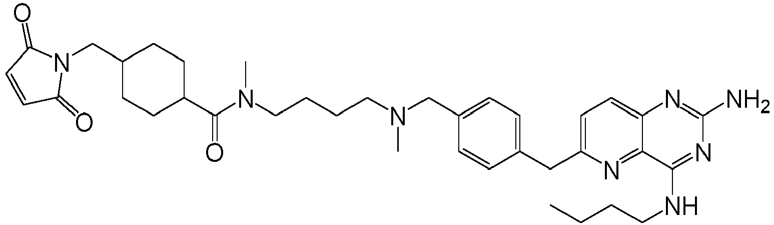 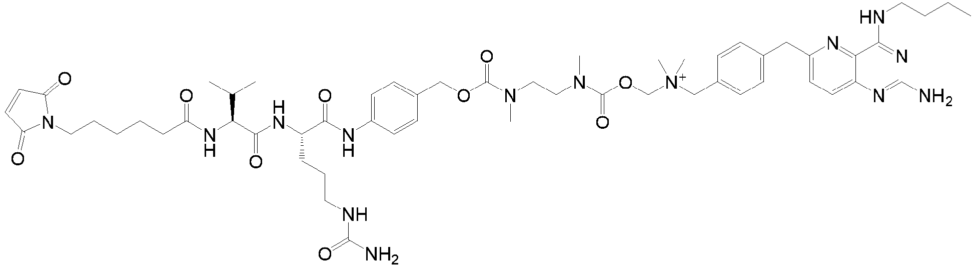 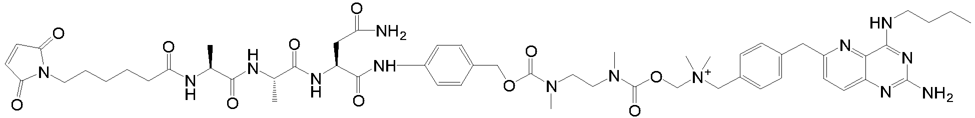 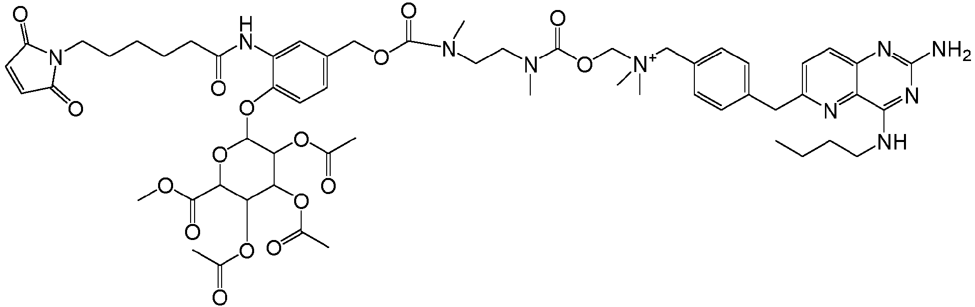 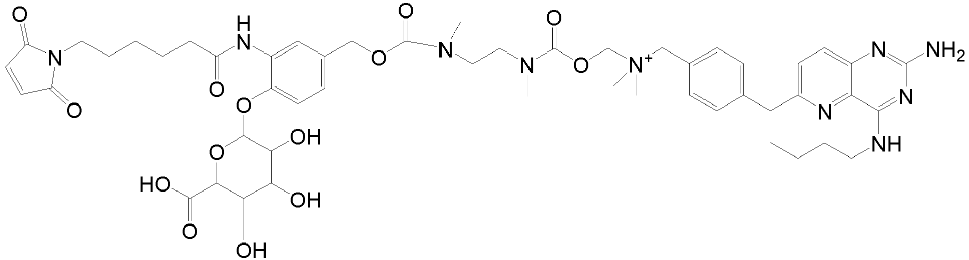 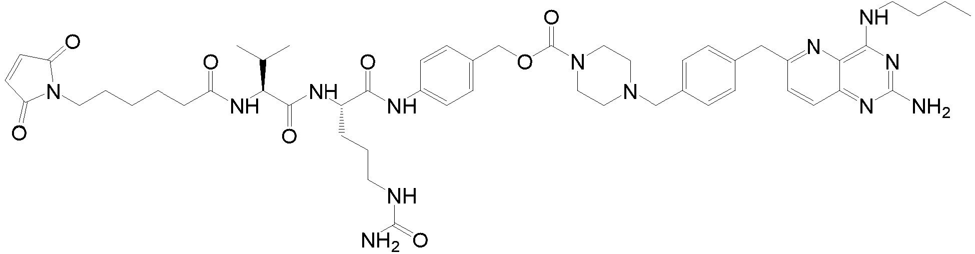 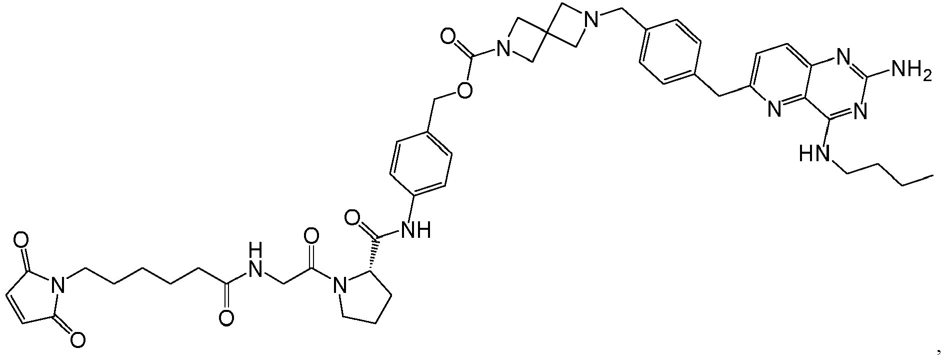 or

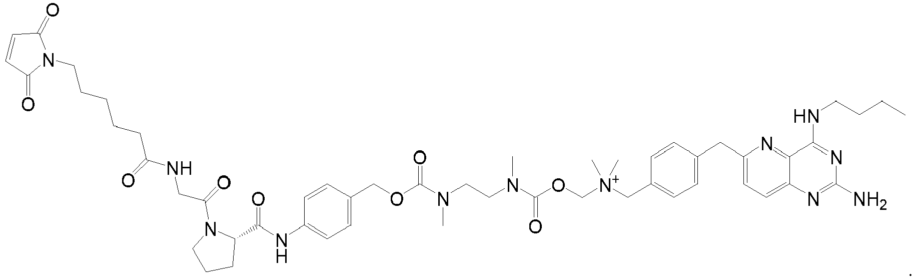

46. A pharmaceutical composition, comprising the antibody-drug conjugate according to any one of claims 1 to 26 and a pharmaceutically acceptable auxiliary material.

47. A composition, comprising an anti-PD-L1 antibody and a small molecule drug as active ingredients, wherein the small molecule drug is a Toll-like receptor agonist.

48. The composition according to claim 47, wherein the small molecule drug has the following structure: 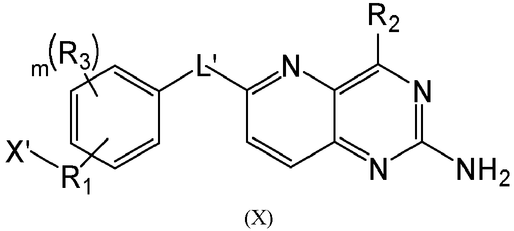
wherein L' is a linking group and is selected from: a single bond, C₁-C₆ alkylene, C₁-C₆ alkenylene, and C₃-C₆ cycloalkylene, wherein the groups can be optionally substituted with C₁-C₄ alkyl;
R₁ is selected from: a single bond, C₁-C₆ alkylene, C₁-C₆ alkenylene, and C₁-C₆ alkyleneoxy, wherein the groups can be optionally substituted with C₁-C₄ alkyl;
X is selected from: 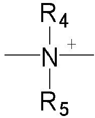 , -NR₄-, -O-, -S-, a single bond, -OCO-, and -COO-, wherein R₄ and R₅ are independently selected from: H, Ci-Ce alkyl, C₁-C₆ alkoxy, amino acid residues, oligopeptide residues, haloalkyl, carboxyl-substituted alkyl, ester-substituted alkyl, and 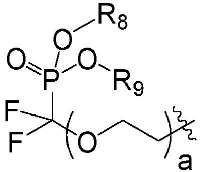 , wherein a is an integer of 1 to 10, R₈ and R₉ are independently selected from: H, and C₁-C₆ alkyl, or R₈ and R₉, together with atoms to which they are attached, form 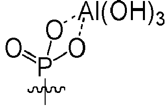 ; or, R₄ and R₅, together with the nitrogen atom to which they are attached, form heterocyclyl;
R₂ is selected from: -NR₆R₇, -OR₆, and -SR₆, wherein R₆ and R₇ are independently selected from: H, C₁-C₆ alkyl, C₁-C₆ alkoxy, and C₁-C₆ alkoxyalkyl, or R₆ and R₇, together with the nitrogen atom to which they are attached, form heterocyclyl, or R₆, together with the oxygen atom to which it is attached, forms heterocyclyl, or R₆, together with the sulfur atom to which it is attached, forms heterocyclyl;
R₃ is one or more independent substituents on the phenyl ring and is selected from: H, C₁-C₆ alkyl, C₁-C₆ alkoxy, and C₁-C₆ alkoxyalkyl;
m is an integer of 0 to 4; and
X' is selected from: H, halogen, -NR₄R₅, -NR₄R₅, -OR₄, -SR₄, azido, phosphonic acid group, phosphonate diethyl, haloalkyl, carboxyl, ester group, and -CN, wherein R₄ and R₅ are independently selected from: H, C₁-C₆ alkyl, C₁-C₆ alkoxy, amino acid residues, oligopeptide residues, and haloalkyl, or R₄ and R₅, together with the nitrogen atom to which they are attached, form heterocyclyl.

49. The composition according to claim 47, wherein the small molecule drug is selected from the following structures:

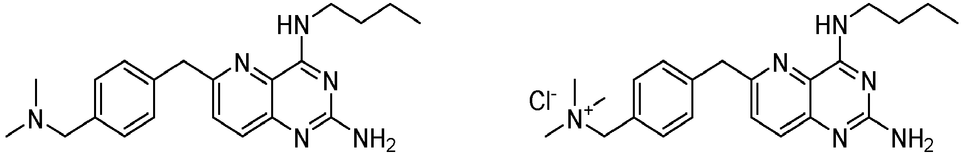

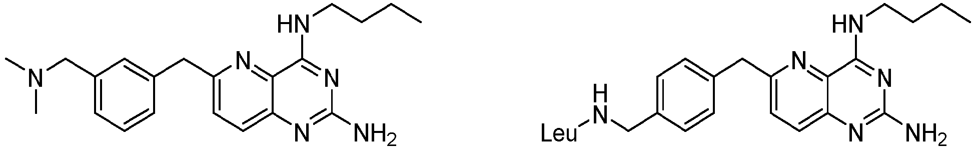

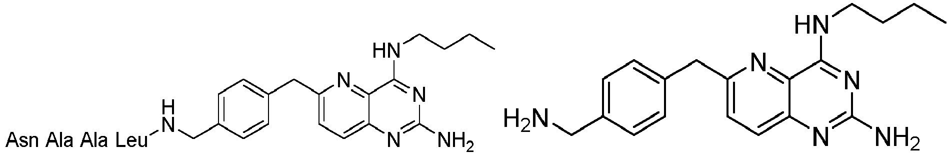

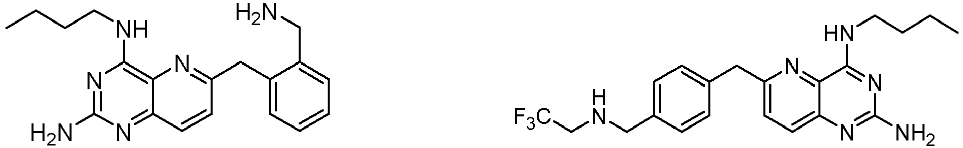

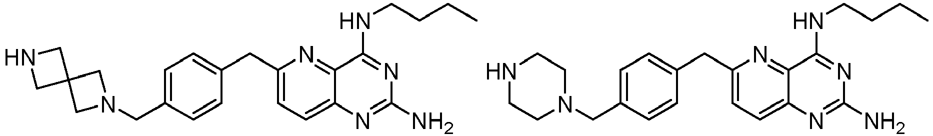

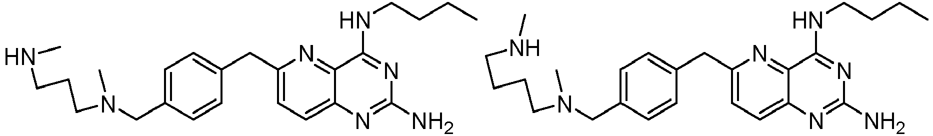

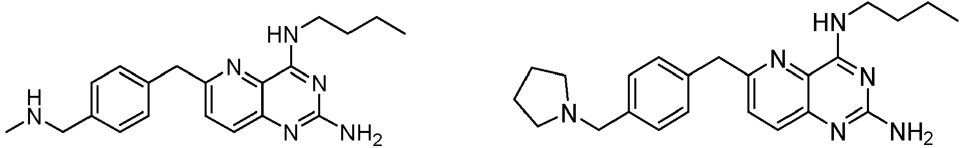

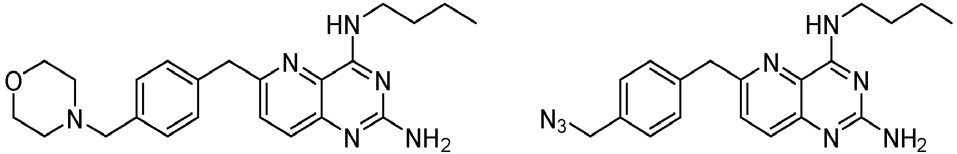

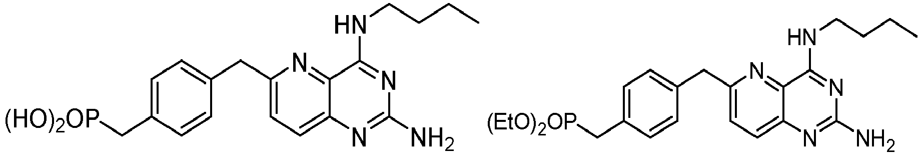

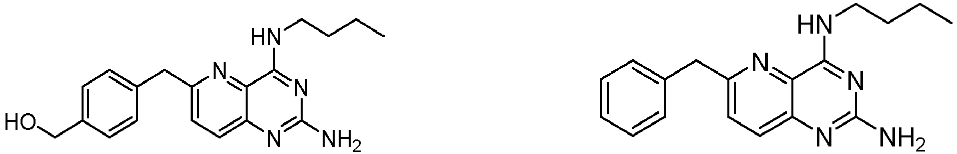

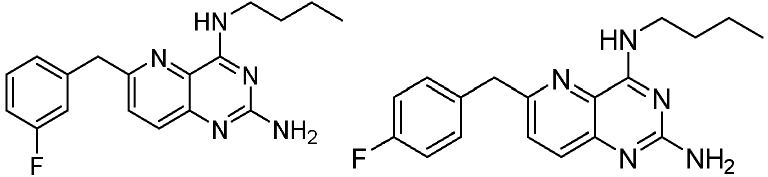

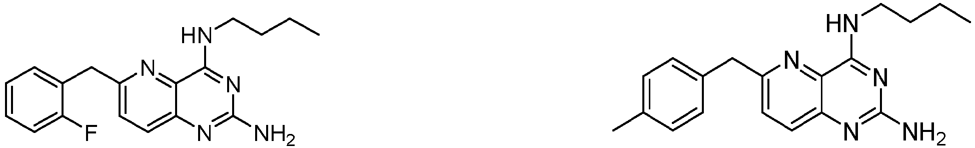

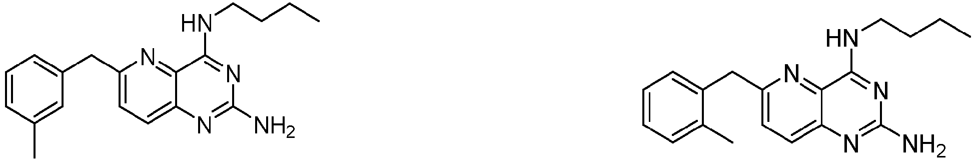

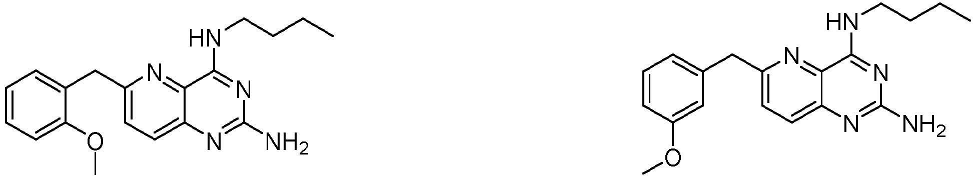

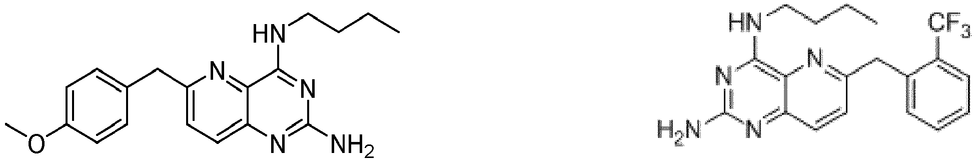

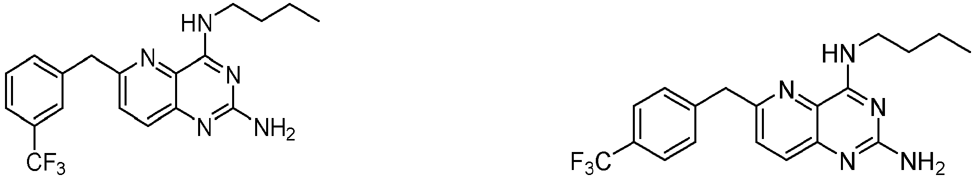

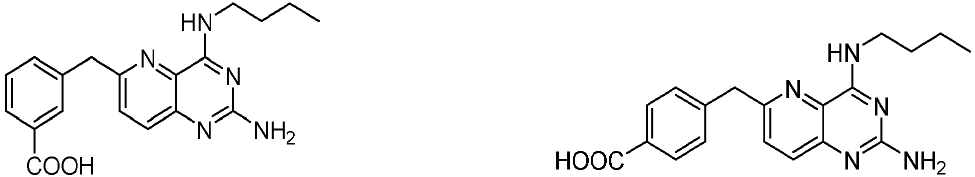

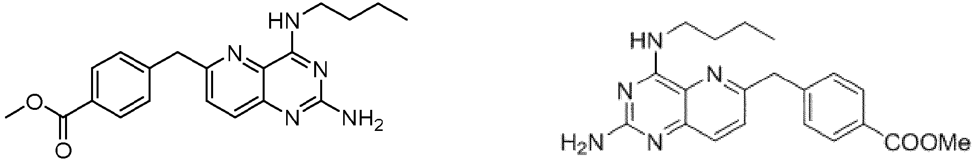

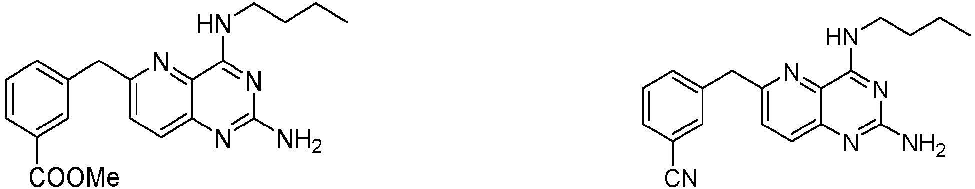

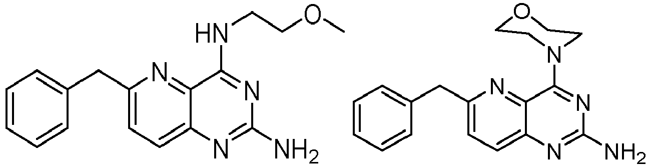

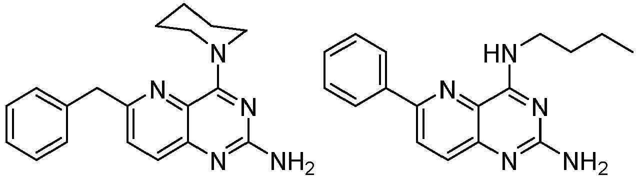

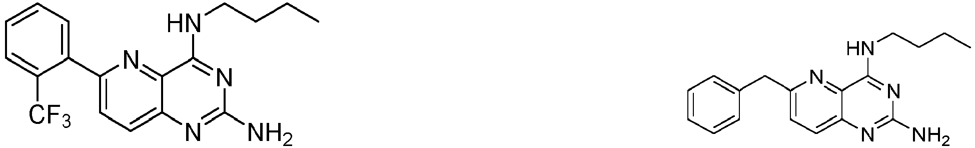

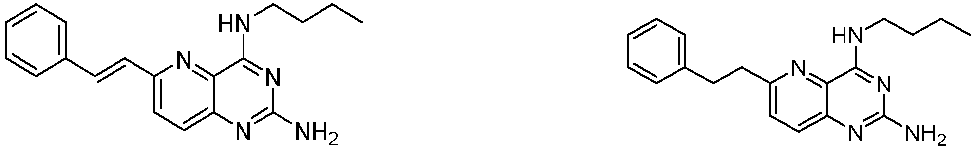

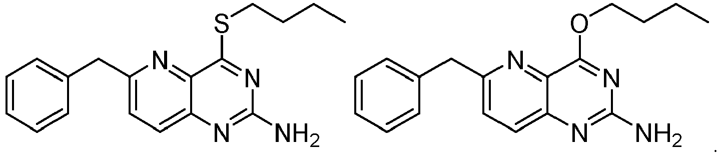

50. The composition according to any one of claims 47 to 49, wherein the anti-PD-L1 antibody is selected from:
atezolizumab, durvalumab, avelumab, cemiplimab, KN035, CS1001, BGB-A333, KL-A167, SHR-1316, and STI-A1014;
the antibody has a light chain sequence set forth in SEQ ID NO: 9; preferably, the antibody has a heavy chain sequence set forth in SEQ ID NO: 4 and a light chain sequence set forth in SEQ ID NO: 9.

51. Use of the antibody-drug conjugate according to any one of claims 1 to 26, the antibody according to claim 28, the compound according to any one of claims 29 to 45, and the composition according to any one of claims 47 to 50 in the preparation of a medicament for preventing and/or treating a disease.

52. The use according to claim 51, wherein the disease is selected from: respiratory disease, immune disease, viral disease, and tumor;
preferably, the respiratory disease is selected from: asthma, chronic obstructive pulmonary disease, and adult respiratory distress syndrome;
preferably, the immune disease is autoimmune diseases and is selected from: systemic lupus erythematosus, rheumatoid arthritis, inflammatory bowel disease, Sjogren's syndrome, polymyositis, vasculitis, Wegener granulomatosis, sarcoidosis, ankylosing spondylitis, Reiter's syndrome, psoriatic arthritis, and Behcet's syndrome;
preferably, the viral disease is selected from: influenza, SARS, COVID-19, hepatitis A, hepatitis B, hepatitis C, hepatitis D, AIDS, rabies, Dengue virus, and Ebola virus disease;
preferably, the tumor is selected from: lymphoma, blastoma, medulloblastoma, retinoblastoma, liposarcoma, synovial cell sarcoma, neuroendocrine tumor, carcinoid tumor, gastrinoma, islet cell carcinoma, mesothelioma, schwannoma, acoustic neuroma, meningioma, adenocarcinoma, melanoma, leukemia and lymphoid malignancy, squamous cell carcinoma, epithelial squamous cell carcinoma, lung carcinoma (small cell lung carcinoma, non-small cell lung carcinoma, adenocarcinoma lung carcinoma, squamous lung carcinoma), peritoneal carcinoma, hepatocellular carcinoma, gastric carcinoma, intestinal carcinoma, pancreatic carcinoma, glioblastoma, cervical carcinoma, ovarian carcinoma, liver carcinoma, bladder carcinoma, breast carcinoma, metastatic breast carcinoma, colon carcinoma, rectal carcinoma, colorectal carcinoma, uterine carcinoma, salivary gland carcinoma, kidney carcinoma, prostate carcinoma, vulval carcinoma, thyroid carcinoma, anal carcinoma, penile carcinoma, Merkel cell carcinoma, esophageal carcinoma, biliary tract carcinoma, head and neck carcinoma, and hematological malignancies.
